# EUROPEAN PATENT SPECIFICATION

(11) **EP 1 325 138 B1**
(45) Date of publication and mention of the grant of the patent: **17.07.2013**
(21) Application number: 01984006.5
(22) Date of filing: 11.10.2001
(51) Int. Cl.: C12N 15/67, C07H 21/00, C07K 14/745, C12N 15/63

(54) **OPTIMIZED MESSENGER RNA**
OPTIMIERTE BOTEN-RNA (MRNA)
ARN MESSAGER OPTIMISE

(30) Priority: 11.10.2000 US 686497
(43) Date of publication of application: 09.07.2003
(60) Divisional application: 10180592.7
(73) Proprietor: Shire Human Genetic Therapies, Inc., Lexington MA 02421 (US)
(72) Inventor: SELDON, Richard F., Wellesley, MA 02481 (US); MILLER, Allan M., Boxford, MA 01921 (US); TRECO, Douglas S., Arlington, MA 02476 (US)
(74) Representative: Williams, Gareth Owen
(86) International application number: PCT/US2001/042655
(87) International publication number: WO 2002/064799

(56) References cited:
- WO-A-96/09378
- WO-A-96/09378
- WO-A-98/11206
- WO-A-98/12207
- WO-A-98/12207
- SCHIFFMANN R ET AL: "Infusion of alpha-galactosidase A reduces tissue globotriaosylceramide storage in patients with Fabry disease" PROCEEDINGS OF THE NATIONAL ACADEMY OF SCIENCES OF USA, NATIONAL ACADEMY OF SCIENCE. WASHINGTON, US, vol. 97, no. 1, 4 January 2000 (2000-01-04), pages 365-370, XP002209996 ISSN: 0027-8424
- LYNCH C M ET AL: "SEQUENCES IN THE CODING REGION OF CLOTTING FACTOR VIII ACT AS DOMINANT INHIBITORS OF RNA ACCUMULATION AND PROTEIN PRODUCTION" HUMAN GENE THERAPY, vol. 4, no. 3, 1 June 1993 (1993-06-01), pages 259-272, XP002051708 ISSN: 1043-0342
- LIND P. ET AL.: "Novel forms of B-domain-deleted recombinant factor VIII molecules. Construction and biochemical characterization" EUROPEAN JOURNAL OF BIOCHEMISTRY, vol. 232, no. 1, 15 August 1995 (1995-08-15), pages 19-27, XP002111900
- WASLEY L.C. ET AL.: "PACE/furin can process the vitamin K-dependent pro-factor IX precursor within the secretory pathway" JOURNAL OF BIOLOGICAL CHEMISTRY, vol. 268, no. 12, 25 April 1993 (1993-04-25), pages 8458-8465, XP002209927

## Description

### Field of the Invention

The invention is directed to methods for optimizing the properties of mRNA molecules, optimized mRNA molecules, methods of using optimized mRNA molecules, and compositions which include optimized mRNA molecules.

### Background of the Invention

In eukaroytes, gene expression is affected, in part, by the stability and structure of the messenger RNA (mRNA) molecule. mRNA stability influences gene expression by affecting the steady-state level of the mRNA. It can affect the rates at which the mRNA disappears following transcriptional repression and accumulates following transcriptional induction. The structure and nucleotide sequence of the mRNA molecule can also influence the efficiency with which these individual mRNA molecules are translated.

The intrinsic stability of a given mRNA molecule is influenced by a number of specific internal sequence elements which can exert a destabilizing effect on the mRNA. These elements may be located in any region of the transcript, and e.g., can be found in the 5' untranslated region (5'UTR), in the coding region and in the 3' untranslated region (3'UTR). It is well established that shortening of the poly(A) tail initiates mRNA decay (Ross, Trends in Genetics, 12:171-175, 1996). The poly(A) tract influences cytoplasmic mRNA stability by protecting mRNA from rapid degradation. Adenosine and uridine rich elements (AUREs) in the 3'UTR are also associated with unstable mammalian mRNA's. It has been demonstrated that proteins that bind to AURE, AURE-binding proteins (AUBPs) can affect mRNA stability. The coding region can also alter the half-life of many RNAs. For example, the coding region can interact with proteins that protect it from endonucleolytic attack. Furthermore, the efficiency with which individual mRNA molecules are translated has a strong influence on the stability of the mRNA molecule (Herrick et al., Mol Cell Biol. 10, 2269-2284, 1990, and Hoekema et al., Mol Cell Biol. 7, 2914-2924, 1987).

The single-stranded nature of mRNA allows it to adopt secondary and tertiary structure in a sequence-dependent manner through complementary base pairing. Examples of such structures include RNA hairpins, stem loops and more complex structures such as bifurcations, pseudoknots and triple-helices. These structures influence both mRNA stability, e.g., the stem loop elements in the 3' UTR can serve as an endonuclease cleavage site, and affect translational efficiency.

In addition to the structure of the mRNA, the nucleotide content of the mRNA can also play a role in the efficiency with which the mRNA is translated. For example, mRNA with a high GC content at the 5'untranslated region (UTR) may be translated with low efficiency and a reduced translational effect can reduce message stability. Thus, altering the sequence of a mRNA molecule can ultimately influence mRNA transcript stability, by influencing the translational stability of the message.

Factor VIII and Factor IX are important plasma proteins that participate in the intrinsic pathway of blood coagulation. Their dysfunction or absence in individuals can result in blood coagulation disorders, e.g., a deficiency of Factor VIII or Factor IX results in Hemophilia A or B, respectively. Isolating Factor VIII or Factor IX from blood is difficult, e.g., the isolation of Factor VIII is characterized by low yields, and also has the associated danger of being contaminated with infectious agents such as Hepatitis B virus, Hepatitis C virus or HIV. Recombinant DNA technology provides an alternative method for producing biologically active Factor VIII or Factor IX. While these methods have had some success, improving the yield of Factor VIII or Factor IX is still a challenge.

An approach to increasing protein yield using recombinant DNA technology is to modify the coding sequence of a protein of interest, e.g., Factor VIII or Factor IX, without altering the amino acid sequence of the gene product. This approach involves altering, for example, the native Factor VIII or Factor IX gene sequence such that codons which are not so frequently used in mammalian cells are replaced with codons which are overrepresented in highly expressed mammalian genes. Seed et al., (WO 98/12207) used this approach with a measure of success. They found that substituting the rare mammalian codons with those frequently used in mammalian cells results in a four fold increase in Factor VIII production from mammalian cells.

D1 (WO98/11206) teaches that an individual suspected of having an α-gal A deficiency such as Fabry disease can be treated either with (1) human cells that have been genetically modified to overexpress and secrete human α-gal A, or (2) purified human α-gal A obtained from cultured, genetically modified human cells.

### Summary of the Invention

The invention provides a synthetic nucleic acid in accordance with claim 1. Further features of the invention are described in the dependent claims. Also provided is a vector in accordance with claim 5. Further provided is a cell containing the vector in accordance with claim 6. The invention also provides a method of producing alpha-galactosidase in accordance with claim 7. Also provided is the use of a synthetic nucleic acid that directs synthesis of an optimized message for alpha-galactosidase in the preparation of a medicament in accordance with claim 10. Further provided is a synthetic nucleic acid that directs synthesis of an optimized message for alpha-galactosidase in the preparation of a medicament in accordance with claim 11. Further features of claim 10 and 11 are defined in dependent claims 12 to 16.

The synthetic nucleic acid can direct the synthesis of an optimized messenger mRNA. In a preferred embodiment, the continuous stretch of common codons can include: the sequence of a pre-pro-protein; the sequence of a pro-protein; the sequence of a mature protein; the "pre" sequence of a pre-pro-protein; the "pre-pro" sequence of a pre-pro-protein; the "pro" sequence of a pre-pro or a pro-protein; or a portion of any of the aforementioned sequences.

In a preferred embodiment, the synthetic nucleic acid sequence includes a continuous stretch of at least 90, 95, 100, 125, 150, 200, 250, 300 or more codons all of which are common codons.

In another preferred embodiment, the nucleic acid sequence encoding a protein has at least 30, 50, 60, 75, 100, 200 or more non-common or less-common codons replaced with a common codon.

In a preferred embodiment, the number of non-common or less-common codons replaced is less than 15,14, 13, 12, 11, 10, 9, 8, 7, 6, 5, 4, 3, 2 or 1.

In a preferred embodiment, the number of non-common or less-common codons remaining is less than 15, 14,13, 12, 11,10, 9, 8, 7, 6, 5, 4, 3, 2 or 1.

In preferred embodiments, the non-common and less-common codons replaced, taken together, are equal or less then 6%, 5%, 4%, 3%, 2%, 1% of the codons in the synthetic nucleic acid sequence.

In preferred embodiments, the non-common and less-common codons remaining, taken together, are equal or less then 6%, 5%, 4%, 3%, 2%, 1% of the codons in the synthetic nucleic acid sequence.

In a preferred embodiment, all of the non-common or less-common codons of the synthetic nucleic acid sequence encoding a protein have been replaced with common codons.

In a preferred embodiment, the synthetic nucleic acid sequence encodes a protein of at least about 90, 95, 100, 105, 110, 120, 130, 150, 200, 500, 700, 1000 or more amino acids in length.

In various preferred embodiments, at least 94%, 95%, 96%, 97%, 98%, 99%, or all, of the codons in the synthetic nucleic acid sequence are common codons. Preferably, all of the codons in the synthetic nucleic acid sequence are common codons.

In preferred embodiments, the protein is expressed in a eukaryotic cell, e.g., a mammalian cell, e.g., a human cell, and the protein is a mammalian protein, e.g., a human protein.

Further described is a synthetic nucleic acid sequence which can direct the synthesis of an optimized message which encodes α-galactosidase.

In a preferred embodiment, the synthetic nucleic acid sequence which encodes α-galactosidase is inserted, e.g., via transfection, into a non-transformed cell, e.g., a primary or secondary cell, e.g., a primary human fibroblast.

In another aspect, the invention features, a plasmid or a DNA construct, e.g., an expression plasmid or a DNA construct, which includes a synthetic nucleic acid sequence described herein.

In yet another aspect, the invention features, a synthetic nucleic acid sequence described herein introduced into the genome of an animal cell. In a preferred embodiment, the animal cell is a primate cell, e.g., a mammal cell, e.g., a human cell.

In still another aspect, the invention features, a cell harboring a synthetic nucleic acid sequence described herein, e.g., a cell from a primary or secondary cell strain, or a cell from a continuous cell line, e.g., a Bowes Melanoma cell (ATCC Accession No. CRL 9607), a Daudi cell (ATCC Accession No. CCL 213), a HeLa cell and a derivative of a HeLa cell (ATCC Accession Nos. CCL 2, CCL2.1, and CCL 2.2), a HL-60 cell (ATCC Accession No. CCL 240), a HT-1080 cell (ATCC Accession No. CCL 121), a Jurkat cell (ATCC Accession No. TIB 152), a KB carcinoma cell (ATCC Accession No. CCL 17), a K-562 leukemia cell (ATCC Accession No. CCL 243), a MCF-7 breast cancer cell (ATCC Accession No. BTH 22), a MOLT-4 cell (ATCC Accession No. 1582), a Namalwa cell (ATCC Accession No. CRL 1432), a Raji cell (ATCC Accession No. CCL 86), a RPMI 8226 cell (ATCC Accession No. CCL 155), a U-937 cell (ATCC Accession No. CRL 1593), a WI-38VA13 sub line 2R4 cell (ATCC Accession No. CLL 75.1), a CCRF-CEM cell (ATCC Accession No. CCL 119) and a 2780AD ovarian carcinoma cell (Van Der Blick et al., Cancer Res. 48: 5927-5932, 1988), as well as heterohybridoma cells produced by fusion of human cells and cells of another species. In another embodiment, the immortalized cell line can be a cell line other than a human cell line, e.g., a CHO cell line or a COS cell line. In a preferred embodiment, the cell is a non-transformed cell. In a preferred embodiment, the cell is from a clonal cell strain. In various preferred embodiments, the cell is a mammalian cell, e.g., a primary or secondary mammalian cell, e.g., a fibroblast, a hematopoietic stem cell, a myoblast, a keratinocyte, an epithelial cell, an endothelial cell, a glial cell, a neural cell, a cell comprising a formed element of the blood, a muscle cell and precursors of these somatic cells. In a most preferred embodiment, the cell is a secondary human fibroblast.

In another aspect, the invention features a method for preparing a synthetic nucleic acid encoding alpha-galactosidase which is at least 90 codons in length. The method includes identifying a non-common codon and a less-common codon in a non-optimized gene sequence which encodes an alpha-galactosidase protein; and replacing at least 94% of the non-common and less-common codons with a common codon encoding the same amino acid as the replaced codon for increased protein expression; wherein by a common codon is meant Ala (gcc); Arg (cgc); Asn (aac); Asp (gac); Cys (tgc); Gln (cag); Gly (ggc); His (cac); Ile (atc); Leu (ctg); Lys (aag); Pro (ccc); Phe (ttc); Ser (agc); Thr (acc); Tyr (tac); Glu (gag) and Val (gtg); wherein less-common codons are Gly (ggg); Ile (att); Leu (ctc); Ser (tcc); Val (gtc) and Arg (agg). Preferably, all codons other than common codons and less-common codons are non-common codons.

Also described herein, the method includes identifying non-common and less-common codons in the non-optimized gene encoding the protein and replacing at least 94%, 95%, 96%, 97%, 98%, 99% or more of the non-common and less-common codons with a common codon encoding the same amino acid as the replaced codon.

In a preferred embodiment, the synthetic nucleic acid sequence encodes a protein of at least about 90, 95, 100, 105, 110, 120, 130, 150, 200, 500, 700, 1000 or more codons in length.

In preferred embodiments, the protein is expressed in a eukaryotic cell, e.g., a mammalian cell, e.g., a human cell, and the protein is a mammalian protein, e.g., a human protein.

Also described herein, is a method for making a nucleic acid sequence which directs the synthesis of a optimized message of a protein of at least 90, 100, or 120 amino acids in length, e.g., a synthetic nucleic acid sequence described herein. The method includes: synthesizing at least two fragments of the nucleic acid sequence, wherein the two fragments encode adjoining portions of the protein and wherein both fragments are mRNA optimized, e.g., as described herein; and joining the two fragments such that a non-common codon is not created at a junction point, thereby making the mRNA optimized nucleic acid sequence.

In a preferred embodiment, the two fragments are joined together such that a unique restriction endonuclease site used to create the two fragments is not recreated at the junction point. In another preferred embodiment, the two fragments are joined together such that a unique restriction site is created.

In a preferred embodiment, the synthetic nucleic acid sequence encodes a protein of at least about 90, 95, 100, 105, 110, 120, 130, 150, 200, 500, 700, 1000 or more codons in length.

In a preferred embodiment, at least 3, 4, 5, 6, 7, 8, 9, 10 or more fragments of the nucleic acid sequence are synthesized.

In a preferred embodiment, the fragments are joined together by a fusion, e.g., a blunt end fusion.

In various preferred embodiments, at least 94%, 95%, 96%, 97%, 98%, 99%, or all of the codons in the synthetic nucleic acid sequence are common codons. Preferably, all of the codons in the synthetic nucleic acid sequence are common codons.

In preferred embodiments, the number of codons which are not common codons is equal to or less than 15, 10, 9, 8, 7, 6, 5, 4, 3, 2, or 1.

In preferred embodiments, each fragment is at least 30, 40, 50, 75, 100, 120, 150 or more codons in length.

Described herein is a method of providing a subject, e.g., a human, with a protein. The methods includes: providing a synthetic nucleic acid sequence that can direct the synthesis of an optimized message for a protein, e.g., a synthetic nucleic acid sequence described herein; introducing the synthetic nucleic acid sequence that directs the synthesis of an optimized message for a protein into the subject; and allowing the subject to express the protein, thereby providing the subject with the protein.

In preferred embodiments, the method further includes inserting the nucleic acid sequence that can direct the synthesis of an optimized message into a cell. The cell can be an autologous, allogeneic, or xenogeneic cell, but is preferably autologous. A preferred cell is a fibroblast, a hematopoietic stem cell, a myoblast, a keratinocyte, an epithelial cell, an endothelial cell, a glial cell, a neural cell, a cell comprising a formed element of the blood, a muscle cell and precursors of these somatic cells. The mRNA optimized synthetic nucleic acid sequence can be inserted into the cell ex *vivo* or *in vivo.* If inserted ex *vivo,* the cell can be introduced into the subject.

In preferred embodiments, at least 94%, 95%, 96%, 97%, 98%, 99%, or all of the codons in the synthetic nucleic acid sequence are common codons. Preferably, all of the codons in the synthetic nucleic acid sequence are common codons.

In preferred embodiments, the number of codons which are not common codons is equal to or less than 15, 10, 9, 8, 7, 6, 5, 4, 3, 2, or 1.

Further provided are synthetic nucleic acid fragments which encode a portion of a protein. Such synthetic nucleic acid fragments are similar to the synthetic nucleic acid sequences of the invention except that they encode only a portion of a protein. Such nucleic acid fragments preferably encode at least 50, 60, 70, 80, 100, 110, 120, 130, 150, 200, 300, 400, 500, or more contiguous amino acids of the protein.

Also described herein are transfected or infected primary and secondary somatic cells of vertebrate origin, particularly of mammalian origin, e.g., of human, mouse, or rabbit origins, e.g., primary human cells, secondary human cells, or primary or secondary rabbit cells. The cells are transfected or infected with exogenous synthetic nucleic acid, e.g., DNA, described herein. The synthetic nucleic acid can encode a protein, e.g., a therapeutic protein, e.g., an enzyme, e.g., α-galactosidase, a cytokine, a hormone, an antigen, an antibody, a clotting factor, e.g., Factor VIII, Factor IX, or a regulatory protein. Also provided are methods by which primary and secondary cells are transfected or infected to include exogenous synthetic DNA, methods of producing clonal cell strains or heterogenous cell strains, and methods of gene therapy in which the transfected or infected primary or secondary cells are used. The synthetic nucleic acid directs the synthesis of an optimized message, e.g., an optimized message as described herein.

Further provided herein are primary and secondary somatic cells, which have been transfected or infected with an exogenous synthetic nucleic acid described herein, which is stably integrated into their genomes or is expressed in the cells episomally. In preferred embodiments the cells are fibroblasts, keratinocytes, epithelial cells, endothelial cells, glial cells, neural cells, cells comprising a formed element of the blood, muscle cells, other somatic cells which can be cultured, or somatic cell precursors. The resulting cells are referred to, respectively, as transfected or infected primary cells and transfected or infected secondary cells. The exogenous synthetic DNA encodes a protein, or a portion thereof, e.g., a therapeutic protein (e.g., Factor VIII or Factor IX). In the embodiment in which the exogenous synthetic DNA encodes a protein, or a portion thereof, to be expressed by the recipient cells, the resulting protein can be retained within the cell, incorporated into the cell membrane or secreted from the cell. In this embodiment, the exogenous synthetic DNA encoding the protein is introduced into cells along with additional DNA sequences sufficient for expression of the exogenous synthetic DNA in the cells. The additional DNA sequences may be of viral or non-viral origin. Primary cells modified to express exogenous synthetic DNA are referred to herein as transfected or infected primary cells, which include cells removed from tissue and placed on culture medium for the first time. Secondary cells modified to express or render available exogenous DNA are referred to herein as transfected or infected secondary cells.

Primary and secondary cells transfected or infected by the subject method, e.g., cloned cell strains, can be seen to fall into three types or categories: 1) cells which do not, as obtained, make or contain the therapeutic protein, 2) cells which make or contain the therapeutic protein but in lower quantities than normal (in quantities less than the physiologically normal lower level) or in defective form, and 3) cells which make the therapeutic protein at physiologically normal levels, but are to be augmented or enhanced in their content or production. Examples of proteins that can be made by the present method include cytokines or clotting factors.

Exogenous synthetic DNA is introduced into primary or secondary cell by a variety of techniques. For example, a DNA construct which includes exogenous synthetic DNA encoding a therapeutic protein and additional DNA sequences necessary for expression in recipient cells can be introduced into primary or secondary cells by electroporation, microinjection, or other means (e.g., calcium phosphate precipitation, modified calcium phosphate precipitation, polybrene precipitation, liposome fusion, receptor-mediated DNA delivery). Alternatively, a vector, such as a retroviral or other vector which includes exogenous synthetic DNA can be used and cells can be genetically modified as a result of infection with the vector.

In addition to the exogenous synthetic DNA, transfected or infected primary and secondary cells may optionally contain DNA encoding a selectable marker, which is expressed and confers upon recipients a selectable phenotype, such as antibiotic resistance, resistance to a cytotoxic agent, nutritional prototrophy or expression of a surface protein. Its presence makes it possible to identify and select cells containing the exogenous DNA. A variety of selectable marker genes can be used, such as neo, gpt, dhfr, ada, pac, hyg, mdr and hisD.

Transfected or infected cells described herein are useful, as populations of transfected or infected primary cells or secondary cells, transfected or infected clonal cell strains, transfected or infected heterogenous cell strains, and as cell mixtures in which at least one representative cell of one of the three preceding categories of transfected or infected cells is present, (e.g., the mixture of cells contains essentially transfected or infected primary or secondary cells and may include untransfected or uninfected primary or secondary cells) as a delivery system for treating an individual with an abnormal or undesirable condition which responds to delivery of a therapeutic protein, which is either: 1) a therapeutic protein (e.g., a protein which is absent, underproduced relative to the individual's physiologic needs, defective, or inefficiently or inappropriately utilized in the individual, e.g., Factor VIII or Factor IX; or 2) a therapeutic protein with novel functions such as enzymatic or transport functions such as α-galactosidase. In the method described herein for providing a therapeutic protein, transfected or infected primary cells or secondary cells, clonal cell strains or heterogenous cell strains, are administered to an individual in whom the abnormal or undesirable condition is to be treated or prevented, in sufficient quantity and by an appropriate route, to express the exogenous synthetic DNA at physiologically relevant levels. A physiologically relevant level is one which either approximates the level at which the product is produced in the body or results in improvement of the abnormal or undesirable condition.

Clonal cell strains of transfected or infected secondary cells (referred to as transfected or infected clonal cell strains) expressing exogenous synthetic DNA (and, optionally, including a selectable marker gene) can be produced by the method described herein. The method includes the steps of: 1) providing a population of primary cells, obtained from the individual to whom the transfected or infected primary cells will be administered or from another source; 2) introducing into the primary cells or into secondary cells derived from primary cells a DNA construct which includes exogenous DNA as described above and the necessary additional DNA sequences described above, producing transfected or infected primary or secondary cells; 3) maintaining transfected or infected primary or secondary cells under conditions appropriate for their propagation; 4) identifying a transfected or infected primary or secondary cell; and 5) producing a colony from the transfected or infected primary or secondary cell identified in (4) by maintaining it under appropriate culture conditions until a desired number of cells is obtained. The desired number of clonal cells is a number sufficient to provide a therapeutically effective amount of product when administered to an individual, e.g., an individual with hemophilia A is provided with a population of cells that produce a therapeutically effective amount of Factor VIII, such that that the condition is treated. The individual can also be, for example, an individual with hemophilia B or an individual with a deficiency of α-galactosidase such as an individual with Fabry disease. The number of cells required for a given therapeutic dose depends on several factors including the expression level of the protein, the condition of the host animal and the limitations associated with the implantation procedure. In general, the number of cells required for implantation is in the range of 1x10⁶ to 5x10⁹, and preferably 1x10⁸ to 5x10⁸. In one embodiment of the method, the cell identified in (4) undergoes approximately 27 doublings (i.e., undergoes 27 cycles of cell growth and cell division) to produce 100 million clonal transfected or infected cells. In another embodiment of the method, exogenous synthetic DNA is introduced into genomic DNA by homologous recombination between DNA sequences present in the DNA construct and genomic DNA. In another embodiment, the exogenous synthetic DNA is present episomally in a transfected cell, e.g., primary or secondary cell.

In one embodiment of producing a clonal population of transfected secondary cells, a cell suspension containing primary or secondary cells is combined with exogenous synthetic DNA encoding a therapeutic protein and DNA encoding a selectable marker, such as the neo gene. The two DNA sequences are present on the same DNA construct or on two separate DNA constructs. The resulting combination is subjected to electroporation, generally at 250-300 volts with a capacitance of 960 µFarads and an appropriate time constant (e.g., 14 to 20 m sec) for cells to take up the DNA construct. In an alternative embodiment, microinjection is used to introduce the DNA construct into primary or secondary cells. In either embodiment, introduction of the exogenous DNA results in production of transfected primary or secondary cells. The exogenous synthetic DNA introduced into the cell can be stably integrated into genomic DNA or is present episomally in the cell.

In the method of producing heterogenous cell strains described herein, the same steps are carried out as described for production of a clonal cell strain, except that a single transfected primary or secondary cell is not isolated and used as the founder cell. Instead, two or more transfected primary or secondary cells are cultured to produce a heterogenous cell strain. A heterogenous cell strain can also contain in addition to two or more transfected primary or secondary cells, untransfected primary or secondary cells.

The methods described herein have wide applicability in treating abnormal or undesired conditions and can be used to provide a variety of proteins in an effective amount to an individual. For example, they can be used to provide secreted proteins (with either predominantly systemic or predominantly local effects, e.g., Factor VIII and Factor IX), membrane proteins (e.g., for imparting new or enhanced cellular responsiveness, facilitating removal of a toxic product or for marking or targeting to a cell) or intracellular proteins (e.g., for affecting gene expression or producing autocrine effects).

A method described herein is particularly advantageous in treating abnormal or undesired conditions in that it: 1) is curative (one gene therapy treatment has the potential to last a patient's lifetime); 2) allows precise dosing (the patient's cells continuously determine and deliver the optimal dose of the required protein based on physiologic demands, and the stably transfected or infected cell strains can be characterized extensively in vitro prior to implantation, leading to accurate predictions of long term function in vivo); 3) is simple to apply in treating patients; 4) eliminates issues concerning patient compliance (following a one-time gene therapy treatment, daily protein injections are no longer necessary); and 5) reduces treatment costs (since the therapeutic protein is synthesized by the patient's own cells, investment in costly protein production and purification is unnecessary).

As used herein, the term "optimized messenger RNA" refers to a synthetic nucleic acid sequence encoding a protein wherein at least one non-common codon or less-common codon in the sequence encoding the protein has been replaced with a common codon.

By "common codon" is meant the most common codon representing a particular amino acid in a human sequence. The codon frequency in highly expressed human genes is outlined below in Table 1. Common codons include: Ala (gcc); Arg (cgc); Asn (aac); Asp (gac); Cys (tgc); Gln (cag); Gly (ggc); His (cac); Ile (atc); Leu (ctg); Lys (aag); Pro (ccc); Phe (ttc); Ser (agc); Thr (acc); Tyr (tac); Glu (gag); and Val (gtg) (see Table 1). "Less-common codons" are codons that occurs frequently in humans but are not the common codon: Gly (ggg); Ile (att); Leu (etc); Ser (tcc); Val (gtc); and Arg (agg). All codons other than common codons and less-common codons are "non-common codons".

**TABLE 1: Codon Frequency in Highly Expressed Human Genes**

| | % occurrence | | | % occurrence | |
|---|---|---|---|---|---|
| Ala | | | Cys | | |
| GC | C | 53 | TG | C | 68 |
| | T | 17 | | T | 32 |
| | A | 13 | | | |
| | G | 17 | Gln | | |
| | | | CA | A | 12 |
| Arg | | | | G | 88 |
| CG | C | 37 | | | |
| | T | 7 | Glu | | |
| | A | 6 | GA | A | 25 |
| | G | 21 | | G | 75 |
| AG | A | 10 | | | |
| | G | 18 | Gly | | |
| | | | GG | C | 50 |
| Asn | | | | T | 12 |
| AA | C | 78 | | A | 14 |
| | T | 25 | | G | 24 |
| | | | | | |
| Leu | | | His | | |
| CT | C | 26 | CA | C | 79 |
| | T | 5 | | T | 21 |
| | A | 3 | | | |
| | G | 58 | Ilc | | |
| TT | A | 2 | AT | C | 77 |
| | G | 6 | | T | 18 |
| | | | | A | 5 |
| Lys | | | | | |
| AA | A | 18 | Ser | | |
| | G | 82 | TC | C | 28 |
| | | | | T | 13 |
| Pro | | | | A | 5 |
| CC | C | 48 | | G | 9 |
| | T | 19 | AG | C | 34 |
| | A | 16 | | T | 10 |
| | G | 17 | | | |
| | | | Thr | | |
| Phe | | | AC | C | 57 |
| TT | C | 80 | | T | 14 |
| | T | 20 | | A | 14 |
| | | | | G | 15 |
| | | | | | |
| | | | Tyr | | |
| | | | TA | C | 74 |
| | | | | T | 26 |
| | | | | | |
| | | | Val | | |
| | | | GT | C | 25 |
| | | | | T | 7 |
| | | | | A | 5 |
| | | | | G | 64 |

Codon frequency in Table 1 was calculated using the GCG program established by the University of Wisconsin Genetics Computer Group. Numbers represent the percentage of cases in which the particular codon is used.

The term "primary cell" includes cells present in a suspension of cells isolated from a vertebrate tissue source (prior to their being plated i.e., attached to a tissue culture substrate such as a dish or flask), cells present in an explant derived from tissue, both of the previous types of cells plated for the first time, and cell suspensions derived from these plated cells. The term secondary cell or cell strain refers to cells at all subsequent steps in culturing. That is, the first time a plated primary cell is removed from the culture substrate and replated (passaged), it is referred to herein as a secondary cell, as are all cells in subsequent passages. Secondary cells are cell strains which consist of secondary cells which have been passaged one or more times. A cell strain consists of secondary cells that: 1) have been passaged one or more times; 2) exhibit a finite number of mean population doublings in culture; 3) exhibit the properties of contact-inhibited, anchorage dependent growth (anchorage-dependence does not apply to cells that are propagated in suspension culture); and 4) are not immortalized. A "clonal cell strain" is defined as a cell strain that is derived from a single founder cell. A "heterogenous cell strain" is defined as a cell strain that is derived from two or more founder cells.

The term "transfected cell" refers to a cell into which an exogenous synthetic nucleic acid sequence, e.g., a sequence which encodes a protein, is introduced. Once in the cell, the synthetic nucleic acid sequence can integrate into the recipients cells chromosomal DNA or can exist episomally. Standard transfection methods can be used to introduce the synthetic nucleic acid sequence into a cell, e.g., transfection mediated by liposome, polybrene, DEAE dextran-mediated transfection, electroporation, calcium phosphate precipitation or microinjection. The term "transfection" does not include delivery of DNA or RNA into a cell by a virus The term "infected cell" refers to a cell into which an exogenous synthetic nucleic acid sequence, e.g., a sequence which encodes a protein, is introduced by a virus. Viruses known to be useful for gene transfer include an adenovirus, an adeno-associated virus, a herpes virus, a mumps virus, a poliovirus, a retrovirus, a Sindbis virus, a lentivirus and a vaccinia virus such as a canary pox virus. Other features and advantages of the invention will be apparent from the following detailed description and the claims.

### Detailed Description of the Invention

The drawings are first briefly described.
*Figure 1* is a schematic representation of domain structures of full-length and B-domain deleted human Factor VIII (hFVIII).
*Figure 2* is a schematic representation of full-length hFVIII.
*Figure 3* is a schematic representation of 5R BDD hFVIII expression plasmid pXF8.186.
*Figure 4* is a schematic representation of LE BDD hFVIII expression plasmid pXF8.61.
*Figure 5* is a schematic representation of the fourteen fragments (Fragments A-Fragment N) assembled to construct pXF8.61.
*Figure 6* is a schematic representation of the assembly of pXF8.61.
*Figure 7* depicts the nucleotide sequence and the corresponding amino acid sequence of the LE B-domain-deleted-Factor VIII (FVIII) insert contained in pAM1-1 (SEQ ID NO:1).
*Figure 8* is a schematic representation of the fragments assembled to construct pXF8.186.
*Figure 9* depicts the nucleotide sequence and the corresponding amino acid sequence of the 5Arg B-domain-deleted-FVIII insert (SEQ ID NO:2).

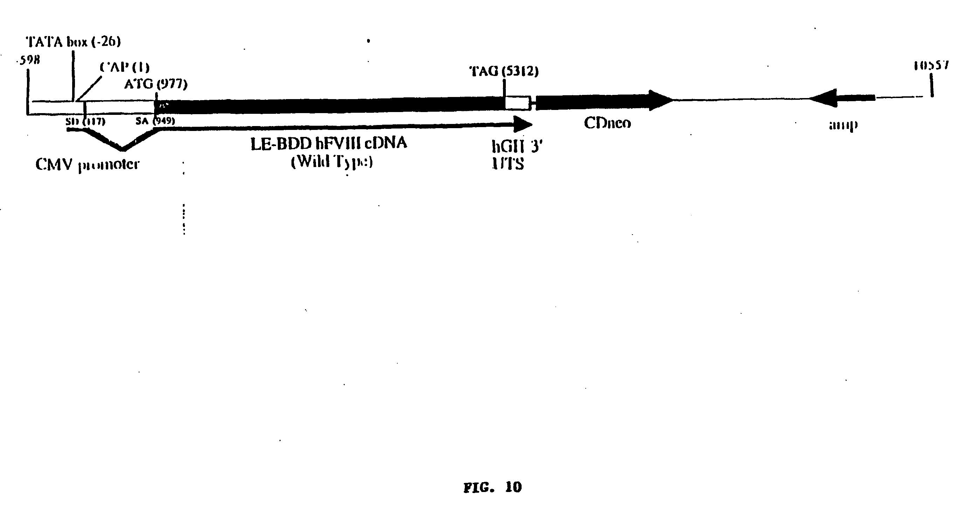

*Figure 10* is a schematic representation of the Factor VIII expression plasmid, pXF8.36. The cytomegalovirus immediate early I (CMV) promoter is depicted as a lightly shaded box. Positions of splice donor (SD) and splice acceptor (SA) sites are indicated below the shaded box. The Factor VIII cDNA sequence is depicted as a solid dark box. The hGH 3'UTS region is depicted as an open box. The new expression cassette is depicted as a shaded box with an arrowhead which corresponds to the direction of transcription. The thin dark line represents the plasmid backbone sequences. The position and direction of transcription of the β-lactamase gene (*amp*) is indicated by the solid boxed arrow.

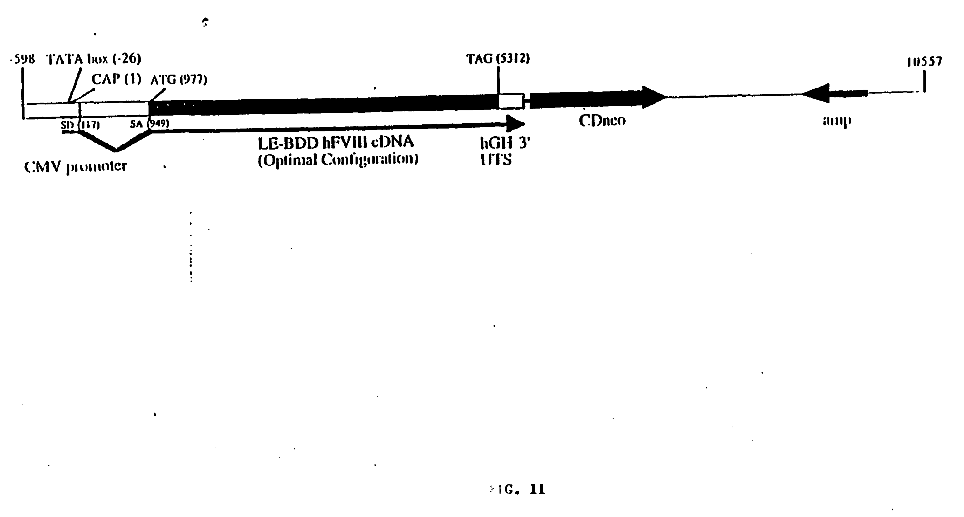

*Figure 11* is a schematic representation of the Factor VIII expression plasmid, pXF8.38. The cytomegalovirus immediate early I (CMV) promoter is depicted as a lightly shaded box. Positions of splice donor (SD) and splice acceptor (SA) sites are indicated below the shaded box. The Factor VIII cDNA sequence is depicted as a solid dark box. The hGH 3'UTS region is depicted as an open box. The *neo* expression cassette is depicted as a shaded box with an arrowhead which corresponds to the direction of transcription. The thin dark line represents the plasmid backbone sequences. The position and direction of transcription of the β-lactamase gene (*amp*) is indicated by the solid boxed arrow.

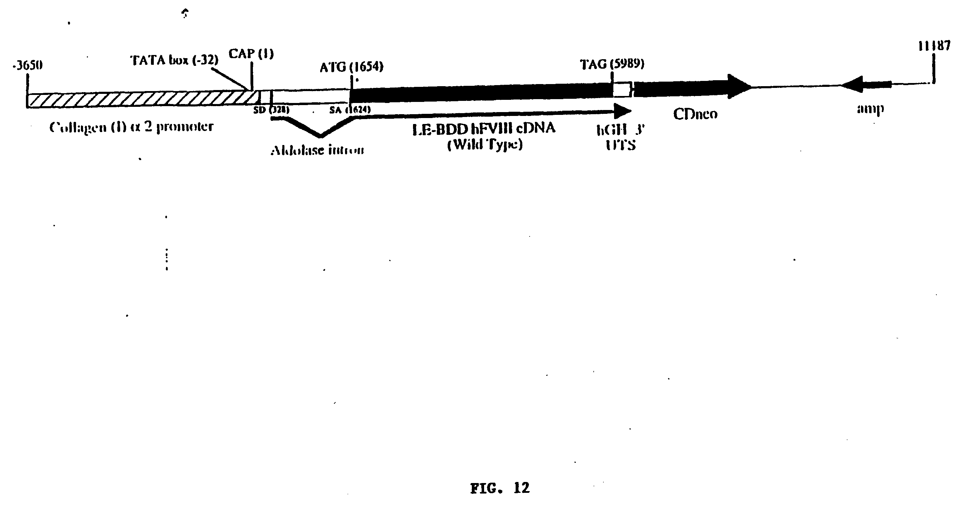

*Figure 12* is a schematic representation of the Factor VIII expression plasmid, pXF8.269. The collagen (I) α 2 promoter is depicted as a striped box. The region representing aldolase-derived 5' untranslated sequences is depicted as a lightly shaded box. Positions of splice donor (SD) and splice acceptor (SA) sites are indicated below the shaded box. The Factor VIII cDNA sequence is depicted as a solid dark box. The hGH 3'UTS region is depicted as an open box. The *neo* expression cassette is depicted as a shaded box with an arrowhead which corresponds to the direction of transcription. The thin dark line represents the plasmid backbone sequences. The position and direction of transcription of the β-lactamase gene (*amp*) is indicated by the solid boxed arrow.

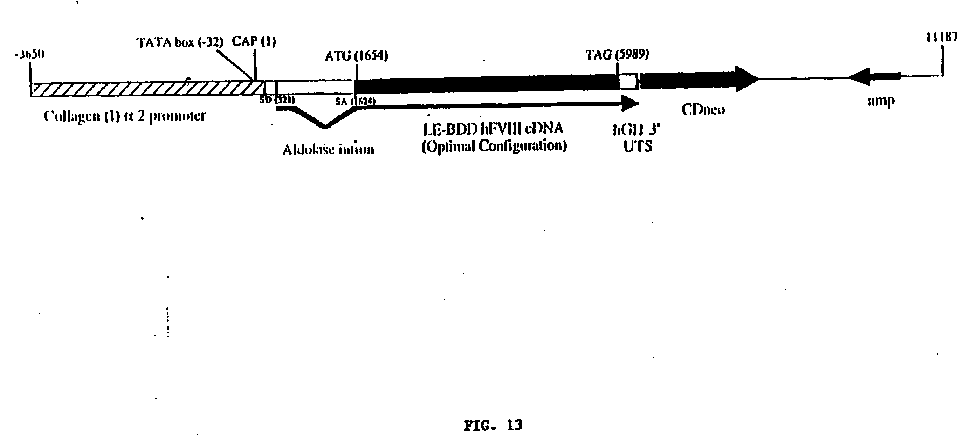

*Figure 13* is a schematic representation of the Factor VIII expression plasmid, pXF8.224. The collagen (I) α 2 promoter is depicted as a striped box. The region representing aldolase-derived 5' untranslated sequences is depicted as a lightly shaded box. Positions of splice donor (SD) and splice acceptor (SA) sites are indicated below the shaded box. The Factor VIII cDNA sequence is depicted as a solid dark box. The hGH 3'UTS region is depicted as an open box. The neo expression cassette is depicted as a shaded box with an arrowhead which corresponds to the direction of transcription. The thin dark line represents the plasmid backbone sequences. The position and direction of transcription of the β-lactamase gene (*amp*) is indicated by the solid boxed arrow.
*Figure 14* is a schematic representation of the fragments assembled to construct pFIXABCD. The restriction sites that are cut are in bold and the junctions from the last step are underlines. The direction of transcription of the FIXABCD sequence is indicated by the solid black arrow.
*Figure 15* depicts the nucleotide sequence of the FIXABCD insert (SEQ ID NO:105).
*Figure 16* is a schematic representation of the Factor IX expression plasmids pXIX76 and pXIX170. The arrows inside the circle denote open reading frames. Arrows on the circle denote promoter sequences; a double headed arrow denotes an enhancer. Thin lines denote bacterial vector sequences or introns and thick boxes delineate the translated sequence. Double lines denote untranscribed genomic sequences, while lines of intermediate thickness denote untranslated portions of the mRNA. Plasmid pXIX170 has a Factor IX cDNA sequence that is optimized, while pXIX76 does not.
*Figure 17* depicts the nucleotide sequence of the α-galactosidase insert SEQ ID NO:106).
*Figure 18* is a schematic representation of the α-galactosidase expression plasmids pXAG94 and pXAG95. The arrows inside the circle denote open reading frames. Arrows on the circle denote promoter sequences; a double headed arrow denotes an enhancer. Thin lines denote bacterial vector sequences or introns and thick boxes delineate the translated sequence. Double lines denote untranscribed genomic sequences, while lines of intermediate thickness denote untranslated portions of the mRNA. Plasmid pXAG95 has an α-galactosidase cDNA sequence that is optimized, while pXAG94 does not.
*Figure 19* is a schematic representation of the α-galactosidase expression plasmids pXAG73 and pXAG74. The arrows inside the circle denote open reading frames. Arrows on the circle denote promoter sequences; a double headed arrow denotes an enhancer. Thin lines denote bacterial vector sequences or introns and thick boxes delineate the translated sequence. Double lines denote untranscribed genomic sequences, while lines of intermediate thickness denote untranslated portions of the mRNA. Plasmid pXAG74 has an α-galactosidase cDNA sequence that is optimized, while pXAG73 does not.

### Message Optimization

Methods described herein are directed to optimized messages and synthetic nucleic acid sequences which direct the production of optimized mRNAs. An optimized mRNA can direct the synthesis of a protein of interest, e.g., a human protein, e.g. a human Factor VIII, human Facto IX or human α-galactosidase. A message for a protein of interest, e.g., human Factor VIII, human Factor IX or human α-galactosidase, can be optimized as described herein, e.g., by replacing at least 94%, 95%, 96%, 97%, 98%, 99%, and preferably all of the non-common codons or less-common codons with a common codon encoding the same amino acid as outlined in Table 1.

The coding region of a synthetic nucleic acid sequence can include the sequence "cg" without any discrimination, if the sequence is found in the common codon for that amino acid. Alternatively, the sequence "cg" can be limited in various regions, e.g., the first 20% of the coding sequence can be designed to have a low incidence of the sequence "cg".

Optimizing a message (and its synthetic DNA sequence) can negatively or positively affect gene expression or protein production. For example, replacing a less-common codon with a more common codon may affect the half-life of the mRNA or alter its structure by introducing a secondary structure that interferes with translation of the message. It may therefore be necessary, in certain instances, to alter the optimized message.

All or a portion of a message (or its gene) can be optimized. In some cases the desired modulation of expression is achieved by optimizing essentially the entire message. In other cases, the desired modulation will be achieved by optimizing part but not all of the message or gene.

The codon usage of any coding sequence can be adjusted to achieve a desired property, for example high levels of expression in a specific cell type. The starting point for such an optimization may be a coding sequence with 100% common codons, or a coding sequence which contains a mixture of common and non-common codons.

Two or more candidate sequences that differ in their codon usage are generated and tested to determine if they possess the desired property. Candidate sequences may be evaluated initially by using a computer to search for the presence of regulatory elements, such as silencers or enhancers, and to search for the presence of regions of coding sequence which could be converted into such regulatory elements by an alteration in codon usage. Additional criteria may include enrichment for particular nucleotides, e.g., A, C, G or U, codon bias for a particular amino acid, or the presence or absence of particular mRNA secondary or tertiary structure. Adjustment to the candidate sequence can be made based on a number of such criteria.

Promising candidate sequences are constructed and then evaluated experimentally. Multiple candidates may be evaluated independently of each other, or the process can be iterative, either by using the most promising candidate as a new starting point, or by combining regions of two or more candidates to produce a novel hybrid. Further rounds of modification and evaluation can be included.

Modifying the codon usage of a candidate sequence can result in the creation or destruction of either a positive or negative element. In general, a positive element refers to any element whose alteration or removal from the candidate sequence could result in a decrease in expression of the therapeutic protein, or whose creation could result in an increase in expression of a therapeutic protein. For example, a positive element can include an enhancer, a promoter, a downstream promoter element, a DNA binding site for a positive regulator (e.g., a transcriptional activator), or a sequence responsible for imparting or removing mRNA secondary or tertiary structure. A negative element refers to any element whose alteration or removal from the candidate sequence could result in an increase in expression of the therapeutic protein, or whose creation would result in a decrease in expression of the therapeutic protein. A negative element includes a silencer, a DNA binding site for a negative regulator (e.g., a transcriptional repressor), a transcriptional pause site, or a sequence that is responsible for imparting or removing mRNA secondary or tertiary structure. In general, a negative element arises more frequently than a positive element. Thus, any change in codon usage that results in an increase in protein expression is more likely to have arisen from the destruction of a negative element rather than the creation of a positive element. In addition, alteration of the candidate sequence is more likely to destroy a positive element than create a positive element. In one embodiment, a candidate sequence is chosen and modified so as to increase the production of a therapeutic protein. The candidate sequence can be modified, e.g., by sequentially altering the codons or by randomly altering the codons in the candidate sequence. A modified candidate sequence is then evaluated by determining the level of expression of the resulting therapeutic protein or by evaluating another parameter, e.g., a parameter correlated to the level of expression. A candidate sequence which produces an increased level of a therapeutic protein as compared to an unaltered candidate sequence is chosen.

In another approach, one or a group of codons can be modified, e.g., without reference to protein or message structure and tested. Alternatively, one or more codons can be chosen on a message-level property, e.g., location in a region of predetermined, e.g., high or low, GC or AU content, location in a region having a structure such as an enhancer or silencer, location in a region that can be modified to introduce a structure such as an enhancer or silencer, location in a region having, or predicted to have, secondary or tertiary structure, e.g., intra-chain pairing, inter-chain pairing, location in a region lacking, or predicted to lack, secondary or tertiary structure, e.g., intra-chain or inter-chain pairing. A particular modified region is chosen if it produces the desired result.

Methods which systematically generate candidate sequences are useful. For example, one or a group, e.g., a contiguous block of codons, at various positions of a synthetic nucleic acid sequence can be replaced with common codons (or with non common codons, if for example, the starting sequence has been optimized) and the resulting sequence evaluated. Candidates can be generated by optimizing (or de-optimizing) a given "window" of codons in the sequence to generate a first candidate, and then moving the window to a new position in the sequence, and optimizing (or de-optimizing) the codons in the new position under the window to provide a second candidate. Candidates can be evaluated by determining the level of expression they provide, or by evaluating another parameter, e.g., a parameter correlated to the level of expression. Some parameters can be evaluated by inspection or computationally, e.g., the possession or lack thereof of high or low GC or AU content; a sequence element such as an enhancer or silencer; secondary or tertiary structure, e.g., intra-chain or inter-chain paring

Thus, hybrid messages, i.e., messages having a region which is optimized and a region which is not optimized, can be evaluated to determine if they have a desired property. The evaluation can be effected by, e.g., synthesizing the candidate message or messages, and determining a property such as its level of expression. Such a determination can be made in a cell-free system or in a cell-based system. The generation and testing of one or more candidates can also be performed, by computational methods, e.g., on a computer. For example, a computer program can be used to generate a number of candidate messages and those messages analyzed by a computer program which predicts the existence of primary structure elements or secondary or tertiary structure.

A candidate message can be generated by dividing a region into subregions and optimizing each subregion. An optimized subregion is then combined with a non-optimized subregion to produce a candidate. For example, a region is divided into three subregions, a, b and c, each of which is then optimized to provide optimized subregions a', b' and c'. The optimized subregions, a', b', and c' can then be combined with one or more of the non-optimized subregions, e.g., a, b and c. For example, ab'c could be formed and tested. Different combinations of optimized and non-optimized subregions can be generated. By evaluating a series of such hybrid candidate sequences, it is possible to analyze the effect of modification of different subregions and, e.g., to define the particular version of each subregion that contributes most to the desired property. A preferred candidate can include the versions of each subregion that performed best in a series of such experiments.
An algorithm for creating an optimized candidate sequence is as follows:
1. Provide a message sequence (an entire message or a portion thereof). Go to step 2.
2. Generate a novel candidate sequence by modifying the codon usage of a candidate sequence by using, the most promising candidate sequence previously identified, or by combining regions of two or more candidates previously identified to produce a novel hybrid. Go to step 3.
3. Evaluate the candidate sequence and determine if it has a predetermined property. If the candidate has the predetermined property, then proceed to step 4, otherwise proceed to step 2.
4. Use the candidate sequence as an optimized message.

Methods can include first optimizing a mammalian synthetic nucleic acid sequence which encodes a protein of interest or a portion thereof, e.g., human Factor VIII, human Factor IX, human α-galactosidase, etc. The synthetic nucleic acid sequence can be optimized such that 94%, 95%, 96%, 97%, 98%, 99%, or all, of the codons of the synthetic DNA are replaced with common codons. The next step involves determining the amount of protein produced as a result of message optimization compared to the amount of protein produced using the wild type sequence. In instances where the amount of protein produced is not of the desired or expected level, it may be desirable to replace one or more of the common codons of the protein-coding region with a less-common codon or non-common codon. A mammalian optimized message which is re-engineered such that common codons are replaced with less-common or non-common mammalian codons, or common codons of other eukaryotic species can result in at least 1%, 5%, 10%, 20% or more of the common codons being replaced. Re-engineering the optimized message can be done, for example, systematically by replacing a single common codon with a less-common or non-common codon. Alternatively, a block of 2, 4, 6, 10, 20, 40 or more codons may be replaced with a less-common or non-common codons. The level of protein produced by these "re-engineered optimized" messages determines which re-engineered optimized message is chosen.

Another approach of optimizing a message for increased protein expression includes altering the specific nucleotide content of an optimized synthetic nucleic acid sequence. The synthetic nucleic acid sequence can be altered by increasing or decreasing specific nucleotide(s) content, e.g., G, C, A, T, GC or AT content of the sequence. Increasing or decreasing the specific nucleotide content of a synthetic nucleotide sequence can be done by substituting the nucleotide of interest with another nucleotide. For example, a sequence that has a large number of codons that have a high GC content, e.g., glycine (GGC), can be substituted with codons that have a less GC rich content, e.g., glycine (GGT) or an AT rich codon. Similarly, a sequence that has a large number of codons that have a high AT content, can be substituted with codons that have a less AT rich content, e.g., a GC rich codon. Any region, or all, of a synthetic nucleic acid sequence can be altered in this manner, e.g., the 5'UTR (e.g., the promoter-proximal coding region), the coding region, the intron sequence, or the 3'UTR. Preferably, nucleotide substitutions in the coding region do not result in an alteration of the amino acid sequence of the expressed product. Preferably, the nucleotide content, e.g., GC or AT content, of a sequence is increased or reduced by 10%, 20%, 30%, 40% or more.

The synthetic nucleic acid sequence can encode a mammalian, e.g., a human protein. The protein can be, e.g., one which is endogenously a human, or an engineered protein. Engineered proteins include proteins which differ from the native protein by one or more amino acid residues. Examples of such proteins include fragments, e.g., internal fragments or truncations, deletions, fusion proteins, and proteins having one or more amino acid replacements.

A sequence which encodes the protein can have one or more introns. The synthetic nucleic acid sequence can include introns, as they are found in the non-optimized sequence or can include introns from a non-related gene. In other embodiments the intronic sequences can be modified. For example, all or part of one or more introns present in the gene can be removed or introns not found in the sequence can be added. In preferred embodiments, one or more entire introns present in the gene are not present in the synthetic nucleic acid. In another embodiment, all or part of an intron present in a gene is replaced by another sequence, e.g., an intronic sequence from another protein.

The synthetic nucleic acid sequence can encode: any protein including a blood factor, e.g., blood clotting factor V, blood clotting factor VII, blood clotting factor VIII, blood clotting factor IX, blood clotting factor X, or blood clotting factor XIII; an interleukin, e.g., interleukin 1, interleukin 2, interleukin 3, interleukin 6, interleukin 11, or interleukin 12; erythropoietin; calcitonin; growth hormone; insulin; insulinotropin; insulin-like growth factors; parathyroid hormone; β-interferon; γ-interferon; nerve growth factors; Fishβ; tumor necrosis factor; glucagon; bone growth factor-2; bone growth factor-7 TSH-β; CSF-granulocyte; CSF-macrophage; CSF-granulocyte/macrophage; immunoglobulins; catalytic antibodies; protein kinase C; glucocerebrosidase; superoxide dismutase; tissue plasminogen activator; urokinase; antithrombin III; DNAse; α-galactosidase; tyrosine hydroxylase; apolipoprotein E; apolipoprotein A-I; globins; low density lipoprotein receptor; IL-2 receptor; IL-2 antagonists; alpha-1 antitrypsin; immune response modifiers; soluble CD4; a protein expressed under disease conditions; and proteins encoded by viruses, e.g., proteins which are encoded by a virus (including a retrovirus) which are expressed in mammalian cells post-infection.

In preferred embodiments, the synthetic nucleic acid sequence can express its protein, e.g., a eukaryotic e.g., mammalian, protein, at a level which is at least 110%, 150%, 200%, 500%, 1,000%, 5,000% or even 10,000% of that expressed by nucleic acid sequence that has not been optimized. This comparison can be made, e.g., in an *in vitro* mammalian cell culture system wherein the non-optimized and optimized sequences are expressed under the same conditions (e.g., the same cell type, same culture conditions, same expression vector).

Suitable cell culture systems for measuring expression of the synthetic nucleic acid sequence and corresponding non-optimized nucleic acid sequence are known in the art (e.g., the pBS phagemic vectors, Stratagene, La Jolla, CA) and are described in, for example, the standard molecular biology reference books. Vectors suitable for expressing the synthetic and non-optimized nucleic acid sequences encoding the protein of interest are described below and in the standard reference books described below. Expression can be measured using an antibody specific for the protein of interest (e.g., ELISA). Such antibodies and measurement techniques are known to those skilled in the art.

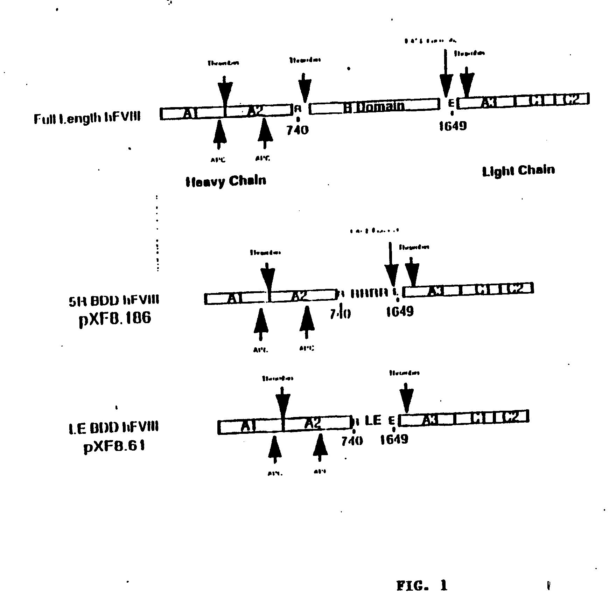

In a preferred embodiment the protein is a human protein. In more preferred embodiments, the protein is human Factor VIII and the protein is a B domain deleted human Factor VIII. In another preferred embodiment the protein is B domain deleted human Factor VIII with a sequence which includes a recognition site for an intracellular protease of the PACE/furin class, such as X-ARG-X-X-ARG site, a short-peptide linker, e.g., a two peptide linker, e.g., a leucine-glutamic acid peptide linker (LE), or a three, or four peptide linker, inserted at the heavy-light chain junction (see Fig. 1).

A large fraction of the codons in the human messages encoding Factor VIII and Factor IX are non-common codons or less common codons. Replacement of at least 98% of these codons with common codons will yield nucleic acid sequences capable of higher level expression in a cell culture. Preferably, all of the codons are replaced with common codons and such replacement results in at least a 2 to 5 fold, more preferably a 10 fold and most preferably a 20 fold increase in expression when compared to an expression of the corresponding native sequence in the same expression system.

The synthetic nucleic acid sequences of the invention can be introduced into the cells of a living organism. The sequences can be introduced directly, e.g., via homologous recombination, or via a vector. For example, DNA constructs or vectors can be used to introduce a synthetic nucleic acid sequence into cells of a living organism for gene therapy. See, e.g., U.S. Patent No. 5,460,959; and co-pending U.S. applications USSN 08/334,797; USSN 08/231,439; USSN 08/334,455; and USSN 08/928,881.

### Transfected or Infected Cells

Primary and secondary cells to be transfected or infected can be obtained from a variety of tissues and include cell types which can be maintained and propagated in culture. For example, primary and secondary cells which can be transfected or infected include fibroblasts, keratinocytes, epithelial cells (e.g., mammary epithelial cells, intestinal epithelial cells), endothelial cells, glial cells, neural cells, a cell comprising a formed element of the blood (e.g., lymphocytes, bone marrow cells), muscle cells and precursors of these somatic cell types. Primary cells are preferably obtained from the individual to whom the transfected or infected primary or secondary cells are administered. However, primary cells may be obtained from a donor (other than the recipient) of the same species or another species (e.g., mouse, rat, rabbit, cat, dog, pig, cow, bird, sheep, goat, horse).

Primary or secondary cells of vertebrate, particularly mammalian, origin can be transfected or infected with exogenous synthetic DNA encoding a therapeutic protein and produce an encoded therapeutic protein stably and reproducibly, both in vitro and in vivo, over extended periods of time. In addition, the transfected or infected primary and secondary cells can express the encoded product in vivo at physiologically relevant levels, cells can be recovered after implantation and, upon reculturing, to grow and display their preimplantation properties.

The transfected or infected primary or secondary cells may also include DNA encoding a selectable marker which confers a selectable phenotype upon them, facilitating their identification and isolation. Methods for producing transfected primary, secondary cells which stably express exogenous synthetic DNA, clonal cell strains and heterogenous cell strains of such transfected cells, methods of producing the clonal and heterogenous cell strains, and methods of treating or preventing an abnormal or undesirable condition through the use of populations of transfected primary or secondary cells are described herein. Primary and secondary cells which can be transfected or infected include fibroblasts, keratinocytes, epithelial cells (e.g., mammary epithelial cells, intestinal epithelial cells), endothelial cells, glial cells, neural cells, a cell comprising a formed element of the blood (e.g., a lymphocyte, a bone marrow cell), muscle cells and precursors of these somatic cell types. Primary cells are preferably obtained from the individual to whom the transfected or infected primary or secondary cells are administered. However, primary cells may be obtained from a donor (other than the recipient) of the same species or another species (e.g., mouse, rat, rabbit, cat, dog, pig, cow, bird, sheep, goat, horse). Transformed or immortalized cells can also be used e.g., a Bowes Melanoma cell (ATCC Accession No. CRL 9607), a Daudi cell (ATCC Accession No. CCL 213), a HeLa cell and a derivative of a HeLa cell (ATCC Accession Nos. CCL 2, CCL2.1, and CCL 2.2), a HL-60 cell (ATCC Accession No. CCL 240), a HT-1080 cell (ATCC Accession No. CCL 121), a Jurkat cell (ATCC Accession No. TIB 152), a KB carcinoma cell (ATCC Accession No. CCL 17), a K-562 leukemia cell (ATCC Accession No. CCL 243), a MCF-7 breast cancer cell (ATCC Accession No. BTH 22), a MOLT-4 cell (ATCC Accession No. 1582), a Namalwa cell (ATCC Accession No. CRL 1432), a Raji cell (ATCC Accession No. CCL 86), a RPMI 8226 cell (ATCC Accession No. CCL 155), a U-937 cell (ATCC Accession No. CRL 1593), WI-38VA13 sub line 2R4 cells (ATCC Accession No. CLL 75.1), a CCRF-CEM cell (ATCC Accession No. CCL 119) and a 2780AD ovarian carcinoma cell (Van Der Blick et al., Cancer Res. 48: 5927-5932, 1988), as well as heterohybridoma cells produced by fusion of human cells and cells of another species. In another embodiment, the immortalized cell line can be a cell line other than a human cell line, e.g., a CHO cell line or a COS cell line. In a preferred embodiment, the cell is a non-transformed cell. In various preferred embodiments, the cell is a mammalian cell, e.g., a primary or secondary mammalian cell, e.g., a fibroblast, a hematopoietic stem cell, a myoblast, a keratinocyte, an epithelial cell, an endothelial cell, a glial cell, a neural cell, a cell comprising a formed element of the blood, a muscle cell and precursors of these somatic cells. In a most preferred embodiment, the cell is a secondary human fibroblast.

Alternatively, DNA can be delivered into any of the cell types discussed above by a viral vector infection. Viruses known to be useful for gene transfer include adenoviruses, adeno-associated virus, herpes virus, mumps virus, poliovirus, retroviruses, Sindbis virus, and vaccinia virus such as canary pox virus. Use of viral vectors is well known in the art: see e.g., Robbins and Ghizzani, Mol. Med. Today 1:410-417,1995. A cell which has an exogenous DNA introduced into it by a viral vector is referred to as an "infected cell"

The disclosure also includes the genetic manipulation of a cell which normally produces a therapeutic protein. In this instance, the cell is manipulated such that the endogenous sequence which encodes the therapeutic protein is replaced with an optimized coding sequence, e.g., by homologous recombination.

### Exogenous Synthetic DNA

Exogenous synthetic DNA incorporated into primary or secondary cells by the present method can be a synthetic DNA which encodes a protein, or a portion thereof, useful to treat an existing condition or prevent it from occurring.

Synthetic DNA incorporated into primary or secondary cells can be an entire gene encoding an entire desired protein or a gene portion which encodes, for example, the active or functional portion(s) of the protein. The protein can be, for example, a hormone, a cytokine, an antigen, an antibody, an enzyme, a clotting factor, e.g., Factor VIII or Factor XI, a transport protein, a receptor, a regulatory protein, a structural protein, or a protein which does not occur in nature. The DNA can be produced, using genetic engineering techniques or synthetic processes. The DNA introduced into primary or secondary cells can encode one or more therapeutic proteins. After introduction into primary or secondary cells, the exogenous synthetic DNA is stably incorporated into the recipient cell's genome (along with the additional sequences present in the DNA construct used), from which it is expressed or otherwise functions. Alternatively, the exogenous synthetic DNA may exist episomally within the primary or secondary cells.

### Selectable Markers

A variety of selectable markers can be incorporated into primary or secondary cells. For example, a selectable marker which confers a selectable phenotype such as drug resistance, nutritional auxotrophy, resistance to a cytotoxic agent or expression of a surface protein, can be used. Selectable marker genes which can be used include neo, gpt, dhfr, ada, pac (puromycin), hyg and hisD. The selectable phenotype conferred makes it possible to identify and isolate recipient primary or secondary cells.

### DNA Constructs

DNA constructs, which include exogenous synthetic DNA and, optionally, DNA encoding a selectable marker, along with additional sequences necessary for expression of the exogenous synthetic DNA in recipient primary or secondary cells, are used to transfect primary or secondary cells in which the encoded protein is to be produced. Alternatively, infectious vectors, such as retroviral, herpes, lentivirus, adenovirus, adenovirus-associated, mumps and poliovirus vectors, can be used for this purpose.

A DNA construct which includes the exogenous synthetic DNA and additional sequences, such as sequences necessary for expression of the exogenous synthetic DNA, can be used. A DNA construct which includes DNA encoding a selectable marker, along with additional sequences, such as a promoter, polyadenylation site and splice junctions, can be used to confer a selectable phenotype upon introduction into primary or secondary cells. The two DNA constructs are introduced into primary or secondary cells, using methods described herein. Alternatively, one DNA construct which includes exogenous synthetic DNA, a selectable marker gene and additional sequences (e.g., those necessary for expression of the exogenous synthetic DNA and for expression of the selectable marker gene) can be used.

### Transfection of Primary or Secondary Cells and Production of Clonal or Heterogenous Cell Strains

Vertebrate tissue can be obtained by standard methods such as punch biopsy or other surgical methods of obtaining a tissue source of the primary cell type of interest. For example, punch biopsy is used to obtain skin as a source of fibroblasts or keratinocytes. A mixture of primary cells is obtained from the tissue, using known methods, such as enzymatic digestion. If enzymatic digestion is used, enzymes such as collagenase, hyaluronidase, dispase, pronase, trypsin, elastase and chymotrypsin can be used.

The resulting primary cell mixture can be transfected directly or it can be cultured first, removed from the culture plate and resuspended before transfection is carried out. Primary cells or secondary cells are combined with exogenous synthetic DNA to be stably integrated into their genomes and, optionally, DNA encoding a selectable marker, and treated in order to accomplish transfection. The exogenous synthetic DNA and selectable marker-encoding DNA are each on a separate construct or on a single construct and an appropriate quantity of DNA to ensure that at least one stably transfected cell containing and appropriately expressing exogenous DNA is produced. In general, 0.1 to 500 ug DNA is used.

Primary or secondary cells can be transfected by electroporation. Electroporation is carried out at appropriate voltage and capacitance (and time constant) to result in entry of the DNA construct(s) into the primary or secondary cells. Electroporation can be carried out over a wide range of voltages (e.g., 50 to 2000 volts) and capacitance values (e.g., 60-300 µFarads). Total DNA of approximately 0.1 to 500 µg is generally used.

Primary or secondary cells can be transfected using microinjection. Alternatively, known methods such as calcium phosphate precipitation, modified calcium phosphate precipitation and polybrene precipitation, liposome fusion and receptor-mediated gene delivery can be used to transfect cells. A stably, transfected cell is isolated and cultured and subcultivated, under culturing conditions and for sufficient time, to propagate the stably transfected secondary cells and produce a clonal cell strain of transfected secondary cells. Alternatively, more than one transfected cell is cultured and subcultured, resulting in production of a heterogenous cell strain.

Transfected primary or secondary cells undergo a sufficient number of doublings to produce either a clonal cell strain or a heterogenous cell strain of sufficient size to provide the therapeutic protein to an individual in effective amounts. In general, for example, 0.1 cm² of skin is biopsied and assumed to contain 100,000 cells; one cell is used to produce a clonal cell strain and undergoes approximately 27 doublings to produce 100 million transfected secondary cells. If a heterogenous cell strain is to be produced from an original transfected population of approximately 100,000 cells, only 10 doublings are needed to produce 100 million transfected cells.

The number of required cells in a transfected clonal or heterogenous cell strain is variable and depends on a variety of factors, including but not limited to, the use of the transfected cells, the functional level of the exogenous DNA in the transfected cells, the site of implantation of the transfected cells (for example, the number of cells that can be used is limited by the anatomical site of implantation), and the age, surface area, and clinical condition of the patient. To put these factors in perspective, to deliver therapeutic levels of human growth hormone in an otherwise healthy 10 kg patient with isolated growth hormone deficiency, approximately one to five hundred million transfected fibroblasts would be necessary (the volume of these cells is about that of the very tip of the patient's thumb).

### Episomal Expression of Exogenous Synthetic DNA

DNA sequences that are present within the cell yet do not integrate into the genome are referred to as episomes. Recombinant episomes may be useful in at least three settings: 1) if a given cell type is incapable of stably integrating the exogenous synthetic DNA; 2) if a given cell type is adversely affected by the integration of synthetic DNA; and 3) if a given cell type is capable of improved therapeutic function with an episomal rather than integrated synthetic DNA.

Using transfection and culturing as described herein, exogenous synthetic DNA in the form of episomes can be introduced into vertebrate primary and secondary cells. Plasmids can be converted into such an episome by the addition DNA sequences for the Epstein-Barr virus origin of replication and nuclear antigen (Yates, J.L. Nature 319:780-7883 (1985)). Alternatively, vertebrate autonomously replicating sequences can be introduced into the construct (Weidle, U.H. Gene 73(2):427-437 (1988). These and other episomally derived sequences can also be included in DNA constructs without selectable markers, such as pXGH5 (Selden et al., Mol Cell Biol. 6:3173-3179, 1986). The episomal synthetic exogenous DNA is then introduced into primary or secondary vertebrate cells as described in this application (if a selective marker is included in the episome a selective agent is used to treat the transfected cells).

### Implantation of Clonal Cell Strain or Heterogenous Cell Strain of Transfected Secondary Cells

The transfected or infected cells produced as described above can be introduced into an individual to whom the therapeutic protein is to be delivered, using known methods. The clonal cell strain or heterogenous cell strain is then introduced into an individual, using known methods, using various routes of administration and at various sites (e.g., renal subcapsular, subcutaneous, central nervous system (including intrathecal), intravascular, intrahepatic, intrasplanchnic, intraperitoneal (including intraomental, or intramuscular implantation). In a preferred embodiment, the clonal cell strain or heterogeneous cell strain is introduced into the omentum. The omentum is a membranous structure containing a sheet of fat. Usually, the omentum is a fold of peritoneum extending from the stomach to adjacent abdominal organs. The greater omentim is attached to the inferior edge of the stomach and hangs down in front of the intestines. The other edge is attached to the transverse colon. The lesser omentum is attached to the superior edge of the stomach and extends to the undersurface of the liver. The cells may be introduced into any part of the omentum by surgical implantation, laparoscopy or direct injection, e.g., via CT-guided needle or ultrasound. Once implanted in the individual, the cells produce the therapeutic product encoded by the exogenous synthetic DNA or are affected by the exogenous synthetic DNA itself. For example, an individual who has been diagnosed with Hemophilia A, a bleeding disorder that is caused by a deficiency in Factor VIII, a protein normally found in the blood, is a candidate for a gene therapy treatment. In another example, an individual who has been diagnosed with Hemophilia B, a bleeding disorder that is caused by a deficiency in Factor IX, a protein normally found in the blood, is a candidate for a gene therapy treatment. The patient has a small skin biopsy performed. This is a simple procedure which can be performed on an out-patient basis. The piece of skin, approximately the size of a match head, is taken, for example, from under the arm and requires about one minute to remove. The sample is processed, resulting in isolation of the patient's cells and genetically engineered to produce the missing Factor IX or Factor VIII. Based on the age, weight, and clinical condition of the patient, the required number of cells are grown in large-scale culture. The entire process requires 4-6 weeks and, at the end of that time, the appropriate number, e.g., approximately 100-500 million genetically engineered cells are introduced into the individual, once again as an outpatient (e.g., by injecting them back under the patient's skin). The patient is now capable of producing his or her own Factor IX or Factor VIII and is no longer a hemophiliac.

A similar approach can be used to treat other conditions or diseases. For example, short stature can be treated by administering human growth hormone to an individual by implanting primary or secondary cells which express human growth hormone; anemia can be treated by administering erythropoietin (EPO) to an individual by implanting primary or secondary cells which express EPO; or diabetes can be treated by administering glucogen-like peptide-1 (GLP-1) to an individual by implanting primary or secondary cells which express GLP-1. A lysosomal storage disease (LSD) can be treated by this approach. LSD's represent a group of at least 41 distinct genetic diseases, each one representing a deficiency of a particular protein that is involved in lysosomal biogenesis. A particular LSD can be treated by administering a lysosomal enzyme to an individual by implanting primary or secondary cells which express the lysosomal enzyme, e.g., Fabry Disease can be treated by administering α-galactosidase to an individual by implanting primary or secondary cells which express α-galactosidase; Gaucher disease can be treated by administering β-glucoceramidase to an individual by implanting primary or secondary cells which express β-glucoceramidase; MPS (mucopolysaccharidosis) type 1 (Hurley-Scheie syndrome) can be treated by administering α-iduronidase to an individual by implanting primary or secondary cells which express α-iduronidase; MPS type II (Hunter syndrome) can be treated by administering α-L-iduronidase to an individual by implanting primary or secondary cells which express α-L-iduronidase; MPS type III-A (Sanfilipo A syndrome) can be treated by administering glucosamine-N-sulfatase to an individual by implanting primary or secondary cells which express glucosamine-N-sulfatase; MPS type III-B (Sanfilipo B syndrome) can be treated by administering alpha-N-acetylglucosaminidase to an individual by implanting primary or secondary cells which express alpha-N-acetylglucosaminidase; MPS type III-C (Sanfilipo C syndrome) can be treated by administering acetylcoenzyme A:α-glucosmainide-N-acetyltransferase to an individual by implanting primary or secondary cells which express acetylcoenzyme A:α-glucosmainide-N-acetyltransferase; MPS type 111-D (Sanfilippo D syndrome) can be treated by administering N-acetylglucosamine-6-sulfatase to an individual by implanting primary or secondary cells which express N-acetylglucosamine-6-sulfatase; MPS type IV-A (Morquip A syndrome) can be treated by administering N-Acetylglucosamine-6-sulfatase to an individual by implanting primary or secondary cells which express N-acetylglucosamine-6-sulfatase; MPS type IV-B (Morquio B syndrome) can be treated by administering β-galactosidase to an individual by implanting primary or secondary cells which express β-galactosidase; MPS type VI (Maroteaux-Larry syndrome) can be treated by administering N-acetylgalactosamine-6-sulfatase to an individual by implanting primary or secondary cells which express N-acetylgalactosamine-6-sulfatase; MPS type VII (Sly syndrome) can be treated by administering β-glucuronidase to an individual by implanting primary or secondary cells which express β-glucuronidase.

The cells used for implantation will generally be patient-specific genetically engineered cells. It is possible, however, to obtain cells from another individual of the same species or from a different species. Use of such cells might require administration of an immunosuppressant, alteration of histocompatibility antigens, or use of a barrier device to prevent rejection of the implanted cells. For many diseases, this will be a one-time treatment and, for others, multiple gene therapy treatments will be required.

### Uses of Transfected or Infected Primary and Secondary Cells and Cell Strains

Transfected or infected primary or secondary cells or cell strains have wide applicability as a vehicle or delivery system for therapeutic proteins, such as enzymes, hormones, cytokines, antigens, antibodies, clotting factors, anti-sense RNA, regulatory proteins, transcription proteins, receptors, structural proteins, novel (non-optimized) proteins and nucleic acid products, and engineered DNA. For example, transfected primary or secondary cells can be used to supply a therapeutic protein, including, but not limited to, Factor VIII, Factor IX, erythropoietin, alpha-1 antitrypsin, calcitonin, glucocerebrosidase, growth hormone, low density lipoprotein (LDL), receptor IL-2 receptor and its antagonists, insulin, globin, immunoglobulins, catalytic antibodies, the interleukins, insulin-like growth factors, superoxide dismutase, immune responder modifiers, parathyroid hormone and interferon, nerve growth factors, tissue plasminogen activators, and colony stimulating factors. Alternatively, transfected primary and secondary cells can be used to immunize an individual (i.e., as a vaccine).

The wide variety of uses of cell strains of the present invention can perhaps most conveniently be summarized as shown below. The cell strains can be used to deliver the following therapeutic products.
1. a secreted protein with predominantly systemic effects;
2. a secreted protein with predominantly local effects;
3. a membrane protein imparting new or enhanced cellular responsiveness;
4. membrane protein facilitating removal of a toxic product;
5. a membrane protein marking or targeting a cell;
6. an intracellular protein;
7. an intracellular protein directly affecting gene expression; and
8. an intracellular protein with autocrine effects.

Transfected or infected primary or secondary cells can be used to administer therapeutic proteins (e.g., hormones, enzymes, clotting factors) which are presently administered intravenously, intramuscularly or subcutaneously, which requires patient cooperation and, often, medical staff participation. When transfected or infected primary or secondary cells are used, there is no need for extensive purification of the polypeptide before it is administered to an individual, as is generally necessary with an isolated polypeptide. In addition, transfected or infected primary or secondary cells of the present invention produce the therapeutic protein as it would normally be produced.

An advantage to the use of transfected or infected primary or secondary cells is that by controlling the number of cells introduced into an individual, one can control the amount of the protein delivered to the body. In addition, in some cases, it is possible to remove the transfected or infected cells if there is no longer a need for the product. A further advantage of treatment by use of transfected or infected primary or secondary cells of the present invention is that production of the therapeutic product can be regulated, such as through the administration of zinc, steroids or an agent which affects transcription of a protein, product or nucleic acid product or affects the stability of a nucleic acid product.

### Transgenic animals

A number of methods have been used to obtain transgenic, non-human mammals. A transgenic non-human mammal refers to a mammal that has gained an additional gene through the introduction of an exogenous synthetic nucleic acid sequence, i.e., transgene, into its own cells (e.g., both the somatic and germ cells), or into an ancestor's germ line.

There are a number of methods to introduce the exogenous DNA into the germ line (e.g., introduction into the germ or somatic cells) of a mammal. One method is by microinjection of a the gene construct into the pronucleus of an early stage embryo (e.g., before the four-cell stage) (Wagner et al., Proc. Natl. Acad. Sci. USA 78:5016 (1981); Brinster et al., Proc Natl Acad Sci USA 82:4438 (1985)). The detailed procedure to produce such transgenic mice has been described (see e.g., Hogan et al., Manipulating the Mouse Embryo, Cold Spring Harbour Laboratory, Cold Spring Harbour, NY (1986); US Patent No. 5,175,383 (1992)). This procedure has also been adapted for other mammalian species (e.g., Hammer et al., Nature 315:680 (1985); Murray et al., Reprod. Fert. Devl. 1:147 (1989); Pursel et al., Vet. Immunol. Histopath. 17:303 (1987); Rexroad et al., J. Reprod. Fert. 41(suppl):119 (1990); Rexroad et al., Molec. Reprod. Devl. 1:164 (1989); Simons et al., BioTechnology 6:179 (1988); Vize et al., J. Cell. Sci. 90:295 (1988); and Wagner, J. Cell. Biochem. 13B(suppl):164 (1989).

Another method for producing germ-line transgenic mammals is through the use of embryonic stem cells or somatic cells (e.g., embryonic, fetal or adult). The gene construct may be introduced into embryonic stem cells by homologous recombination (Thomas et al., Cell 51:503 (1987); Capecchi, Science 244:1288 (1989); Joyner et al., Nature 338: 153 (1989)). A suitable construct may also be introduced into the embryonic stem cells by DNA-mediated transfection, such as electroporation (Ausubel et al., Current Protocols in Molecular Biology, John Wiley & Sons (1987)). Detailed procedures for culturing embryonic stem cells (e.g. ESD-3, ATCC# CCL-1934, ES-E14TG-2a, ATCC# CCL-1821, American Type Culture Collection, Rockville, MD) and the methods of making transgenic mammals from embryonic stem cells can be found in Teratocarcinomas and Embryonic Stem Cells, A Practical Approach, ed. E.J. Robertson (IRL Press, 1987). Methods of making transgenic animals from somatic cells can be found, for example, in WO 97/07669, WO 97/07668 and U.S. Patent Number 5,945,577.

In the above methods for the generation of a germ-line transgenic mammals, the construct may be introduced as a linear construct, as a circular plasmid, or as a vector which may be incorporated and inherited as a transgene integrated into the host genome. The transgene may also be constructed so as to permit it to be inherited as an extrachromosomal plasmid (Gassmann, M. et al., Proc. Natl. Acad. Sci. USA 92:1292 (1995)).

### Human Factor VIII (COMPARATIVE EXAMPLE)

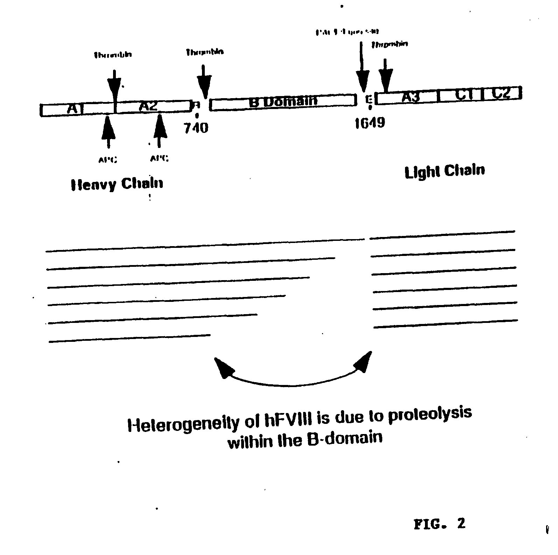

hFVIII is encoded by a 186 kilobase (kb) gene, with the coding region distributed among 26 exons (Gitchier et al., Nature, 312:326-330, (1984)). Transcription of the gene and splicing of the resulting primary transcript results in an mRNA of approximately 9 kb which encodes a primary translation product containing 2351 amino acids (aa), including a 19 aa signal peptide. Excluding the signal peptide, the 2332 aa protein has a domain structure which can be represented as NH2-A1-A2-B-A3-C1-C2-COOH, with a predicted molecular mass of 265 kilodaltons (kD). Glycosylation of this protein results in a product with a molecular mass of approximately 330 kD as determined by SDS-PAGE. In plasma, hFVIII is a heterodimeric protein consisting of a heavy chain that ranges in size from 90 kD to 200 kD in a metal ion complex with an 80 kD light chain. The heterodimeric complex is further stabilized by interactions with vWF. The heavy chain is comprised of domains A1-A2-B and the light chain is comprised of domains A3-C1-C2 (Figure 2). Protease cleavage sites in the B-domain account for the size variation of the heavy chain, with the 90 kD species containing no B-domain sequences and the 200 kD species containing a complete or nearly complete B-domain. The B-domain has no known function and it is fully removed upon hFVIII activation by thrombin.

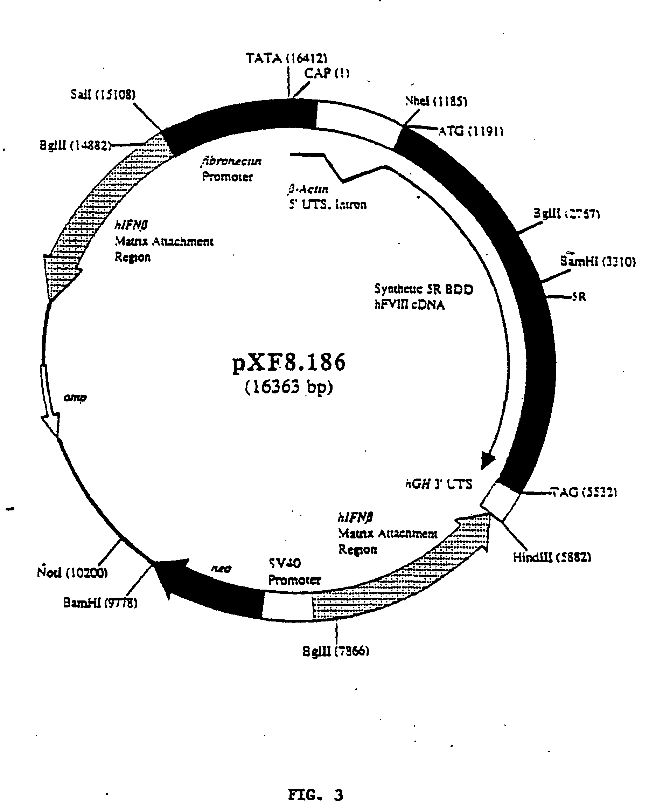

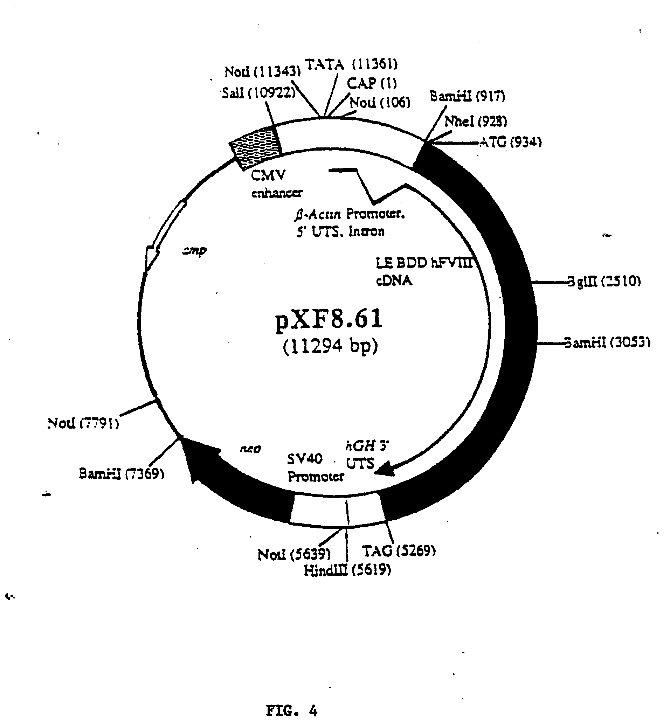

Human Factor VIII expression plasmids, plasmids pXF8.186 (Figure 3), pXF8.61 (Figure 4), pXF8.38 (Fig. 11) and pXF8.224 (Fig. 13) are described below. The hFVIII expression construct plasmid pXF8.186, was developed based on detailed optimization studies which resulted in high level expression of a functional hFVIII. Given the extremely large size of the hFVIII gene and the need to transfer the entire coding region into cells, cDNA expression plasmids were developed for the production of stably transfected clonal cell strains. It has proven difficult to achieve high level expression of hFVIII using the wild-type 9 kb cDNA. Three potential reasons for the poor expression are as follows. First, the wild-type cDNA encodes the 909 aa, heavily glycosylated B-domain which is transiently attached to the heavy chain and has no known function (Figure 1). Removal of the region encoding the B-domain from hFVIII expression constructs leads to greatly improved expression of a functional protein. Analysis of hFVIII derivatives lacking the B-domain has demonstrated that hFVIII function is not adversely affected and that such molecules have biochemical, immunologic, and in vivo functional properties which are very similar to the wild-type protein. Two different BDD hFVIII expression constructs have been developed, which encode proteins with different amino acid sequences flanking the deletion. Plasmid pXF8.186 contains a complete deletion of the B-domain (amino acids 741-1648 of the wild-type mature protein sequence), with the sequence Arg-Arg-Arg-Arg (RRRR) inserted at the heavy chain-light chain junction (Figure 1). This results in a string of five consecutive arginine residues (RRRRR or 5R) at the heavy chain-light chain junction, which comprises a recognition site for an intracellular protease of the PACE/furin class, and was predicted to promote cleavage to produce the correct heavy and light chains. Plasmid pXF8.61 also contains a complete deletion of the B-domain with a synthetic XhoI site at the junction. This linker results in the presence of the dipeptide sequence Leu-Glu (LE) at the heavy chain-light chain junction in the two forms of BDD hFVIII, the expressed proteins are referred to herein as 5R and LE BDD hFVIII.

The second feature which has been reported to adversely affect hFVIII expression in transfected cells relates to the observation that one or more regions of the coding region have been identified which effectively function to block transcription of the cDNA sequence. The inventors have now discovered that the negative influence of the sequence elements can be reduced or eliminated by altering the entire coding sequence. To this end, a completely synthetic B-domain deleted hFVIII cDNA was prepared as described in greater detail below. Silent base changes were made in all codons which did not correspond to the triplet sequence most frequently found for that amino acid in highly expressed human proteins, and such codons were converted to the codon sequence most frequently found in humans for the corresponding amino acid. The resulting coding sequence has a total of 1094 of 4335 base pairs which differ from the wild-type sequence, yet it encodes a protein with the wild-type hFVIII sequence (with the exception of the deletion of the B-domain). 25.2% of the bases were changed, and the GC content of the sequence increased from 44% to 64%. This sequence-altered BDD hFVIII cDNA is expressed at least 5.3-fold more efficiently than a non-altered control construct.

The third feature which was optimized to improve hFVIII expression was the intron-exon structure of the expression construct. The cDNA is, by definition, devoid of introns. While this reduces the size of the expression construct, it has been shown that introns can have strong positive effects on gene expression when added to cDNA expression constructs. The 5' untranslated region of the human beta-actin gene, which contains a complete, functional intron was incorporated into the BDD hFVIII expression constructs pXF8.61 and pXF8.186.

The fourth feature which can adversely affect hFVIII expression is the stability of the Factor VIII mRNA. The stability of the message can affect the steady-state level of the Factor VIII mRNA, and influence gene expression. Specific sequences within Factor VIII can be altered so as to increase the stability of the mRNA, e.g., the removal of AURE from the 3' UTR can result in a more stable Factor VIII mRNA. The data presented below show that coding sequence re-engineering has general utility for the improvement of expression of mammalian and non-mammalian eukaryotic genes in mammalian cells. The results obtained here with human Factor VIII suggest that systemic codon optimization (with disregard to CpG content) provides a fruitful strategy for improving the expression in mammalian cells of a wide variety of eukaryotic genes.

### Methods of Making Synthetic Nucleotide Sequences

A synthetic nucleic acid sequence which directs the synthesis of an optimized message of the invention can be made, e.g., by any of the methods described herein. The methods described below are advantageous for making optimized messages for the following reasons:
1) they allow for production of a highly optimized protein, e.g., a protein having at least 94 to 100% of codons as common codons, especially for proteins larger than 90 amino acids in length. The final product can be 100% optimized, i.e., every single nucleotide is as chosen, without the need to introduce undesirable alterations every 100 - 300 bp. A gene can be synthesized with 100% optimized codons, or it can be synthesized with 100% the codons that are desired. Additional DNA sequence elements can be introduced or avoided without any limitations imposed by the need to introduce restriction enzyme sites. Such sequence elements could include:
   - Transcriptional signals, such as enhancers or silencers.
   - Splicing signals, for example avoiding cryptic splice sites in a cDNA, or optimizing the splice site context in an intron-containing gene. Adding an intron to a cDNA may aid expression and allows the introduction of transcriptional signals within the gene.
   - Instability signals - the creation or avoidance of sequences that direct mRNA breakdown.
   - Secondary structure - the creation or avoidance of secondary structures in the mRNA that may affect mRNA stability, transcriptional termination, or translation.
   - Translational signals - Codon choice. A gene can be synthesized with 100% optimal codons, or the codon bias for any amino acid can be altered without restriction to make gene expression sensitive to the concentration of an amino-acyl-tRNA, whose concentration may vary with growth or metabolic conditions.
   In each case, the goal may be to increase or decrease expression to bring expression under a particular form of regulation.
2) they improve accuracy of the synthetic sequence because they avoid PCR amplification which introduces errors into the amplified sequence; and
3) they reduce the cost of making the synthetic sequence of the invention.

The synthetic nucleic acid sequence which directs the synthesis of the optimized messages of the invention can be prepared, e.g., by using the strategy which is outlined in greater detail below.

### Strategy for building a sequence

The initial step is to devise a cloning protocol.

A sequence file containing 100% the desired DNA sequence is generated. This sequence is analyzed for restriction sites, including fusion sites.

Fusion sites are, in order of preference:
A) Sequences resulting from the ligation of two complementary overhangs normally generated by available restriction enzymes, e.g.,

| | |
|---|---|
| SalI/XhoI = | G^TCGAG |
| | CAGCT^C |
| | |
| or BspDI/BstBI = | AT^CGAA |
| | TAGC^TT |
| | |
| or BstBI/AccI = | TT^CGAC |
| | AAGC^TG. |

B) Sequences resulting from the ligation of two overhangs generated by partially filling-in the overhangs of available restriction enzymes, e.g.,

| | |
|---|---|
| XhoI(+TC)/BamHI(+GA) = | CTC^GATCC. |
| | GAGCT^AGG |

C) Sequences resulting from the blunt ligation of two blunt ends normally generated by available restriction enzymes, e.g.,

| | |
|---|---|
| EheI/SmaI = | GGC^GGG |
| | CCG^CCC. |

D) Sequences resulting from the blunt ligation of two blunt ends, where one or both blunt ends have been generated by filling in an overhang, e.g.,

| | |
|---|---|
| BamHI(+GATC)/SmaI = | GGATC^GGG |
| | CCTAG^CCC |

The filling-in of a 5' overhang generated by a restriction enzyme is performed using a DNA polymerase, for example the Klenow fragment of DNA Polymerase I. If the overhang is to be filled in completely, then all four nucleotides, dATP, dCTP, dGTP, and dTTP, are included in the reaction.. If the overhang is to be only partially filled in, then the requisite nucleotides are omitted from the reaction, In item (B) above, the XhoI-digested DNA would be filled in by Klenow in the presence of dCTP and dTTP and by omitting dATP and dGTP. An order of cloning steps is determined that allows the use of sites about 150-500 bp apart. Note that a fragment must lack the recognition sequence for an enzyme, only if that enzyme is used to clone the fragment. For example, the strategy for the construction of the "desired" Factor VIII coding sequence can use ApaLI in a number of different places, because of the order of assembly of the fragments - ApaLI is not used in any of the later cloning steps.

If there is a region where no useful sites are available, then a sequence-independent strategy can be used: fragments are cloned into a DNA construct that contain recognition sequences for restriction enzymes that cleave outside of their recognition sequence, e.g., BseRI =
GAGGAGNNNNNNNNNN^ (SEQ ID NO:5)
CTCCTCNNNNNNNN^NN (SEQ ID NO:6)
DNA construct cloning site gene fragment

The recognition sequence of the enzyme used to clone the fragment will be removed when the fragment is released by digestion with, e.g. BseRI, leaving a fragment consisting of 100% of the desired sequence, which can then be ligated to a similarly generated adjacent gene fragment.

The next step is to synthesize initial restriction fragments.

The synthesis of the initial restriction fragments can be achieved in a number of ways, including, but not limited to:
1. Chemical synthesis of the entire fragment.
2. Synthesize two oligonucleotides that are complementary at their 3' ends, anneal them, and use DNA polymerase Klenow fragment, or equivalent, to extend, giving a double-stranded fragment.
3. Synthesize a number of smaller oligonucleotides, kinase those oligos that have internal 5' ends, anneal all oligos and ligate, viz.

5`_____ p________ p_________3`
3`_________p _________p _____5`

Techniques 2 and 3 can be used in subsequent steps to join smaller fragments to each other. PCR can be used to increase the quantity of material for cloning, but it may lead to an increase in the number of mutations. If an error-free fragment is not obtained, then site-directed mutagenesis can be used to correct the best isolate. This is followed by concatenation of error-free fragments and sequencing of junctions to confirm their precision.

### Use

The synthetic nucleic acid sequences described herein are useful for expressing a protein normally expressed in a mammalian cell, or in cell culture (e.g. for commercial production of human proteins such as GH, tPA, GLP-1, EPO, α-galactosidase, β-glucoceramidase, α-iduronidase; α-L-iduronidase, glucosamine-N-sulfatase, alpha-N-acetylglucosaminidase, acetylcoenzyme A:α-glucosmainide-N-acetyltransferase, N-acetylglucosamine-6-sulfatase, N-acetylglucosamine-6-sulfatase, β-galactosidase, N-acetylgalactosamine-6-sulfatase, β-glucuronidase. Factor VIII, and Factor IX). The synthetic nucleic acid sequences described herein are also useful for gene therapy. For example, a synthetic nucleic acid sequence encoding a selected protein can be introduced directly, e.g., via non-viral cell transfection or via a vector in to a cell, e.g., a transformed or a non-transformed cell, which can express the protein to create a cell which can be administered to a patient in need of the protein. Such cell-based gene therapy techniques are described in greater detail in co-pending US applications: USSN 08/334,797; USSN 08/231,439; USSN 08/334,455; and USSN 08/928,881.

### Examples

### I. Factor VIII Constructs and Uses thereof (COMPARATIVE EXAMPLE)

### Construction of pXF8.61

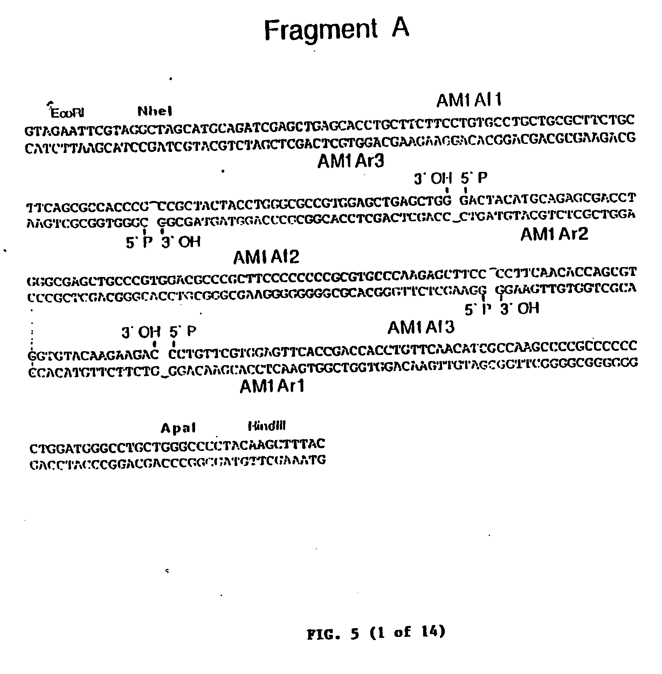

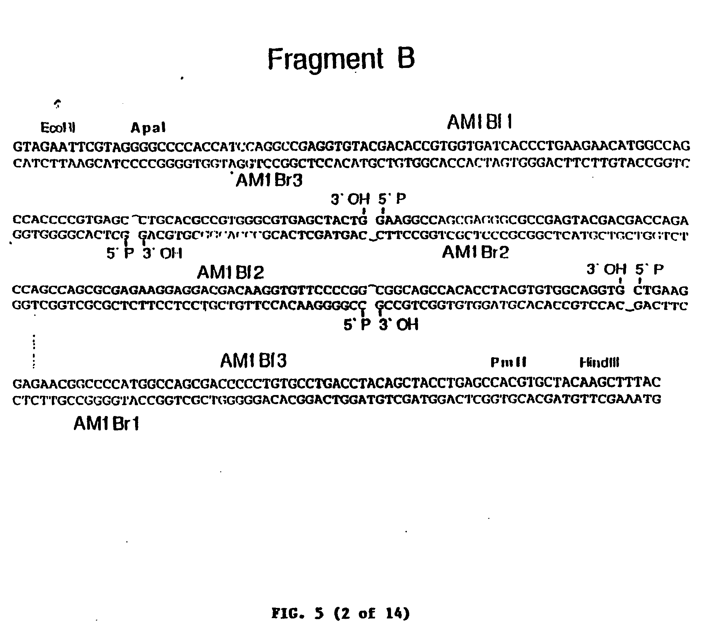

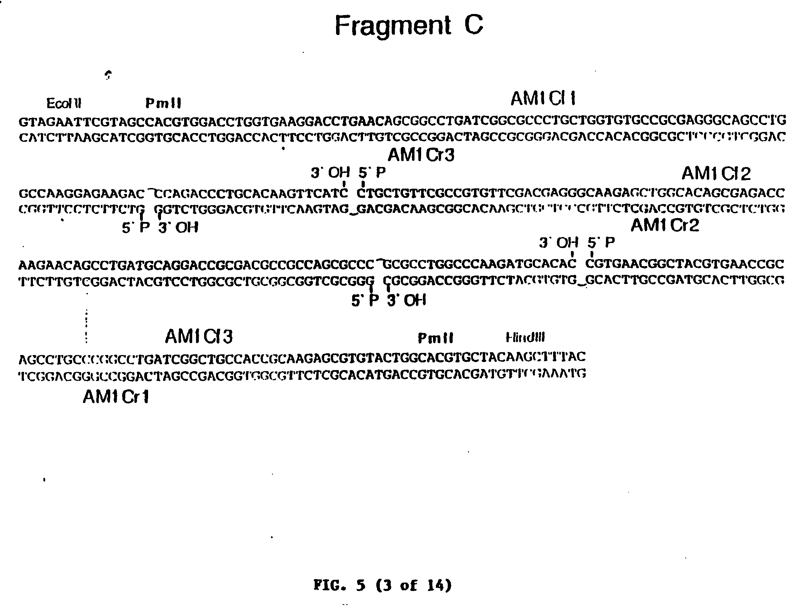

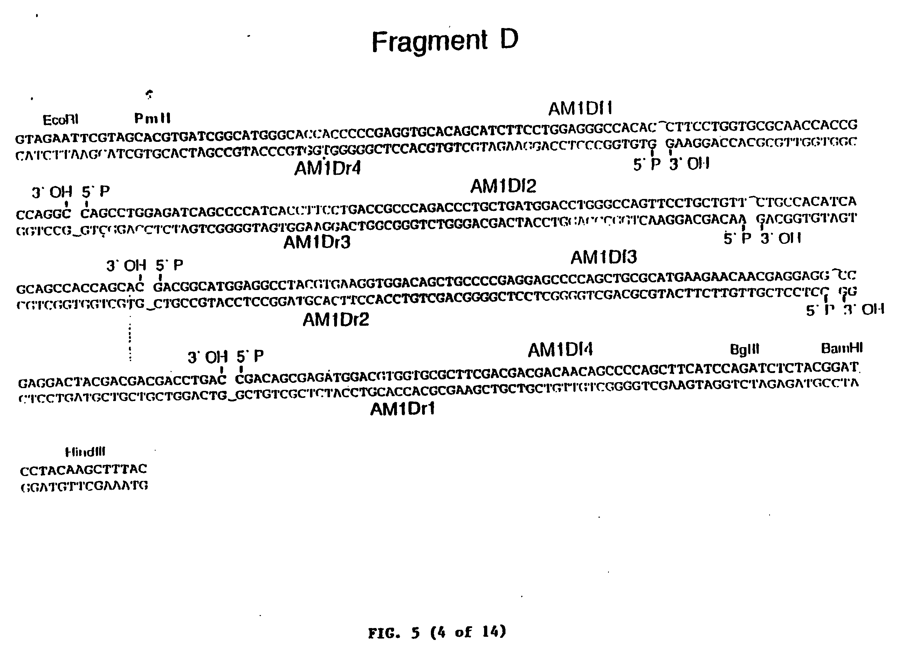

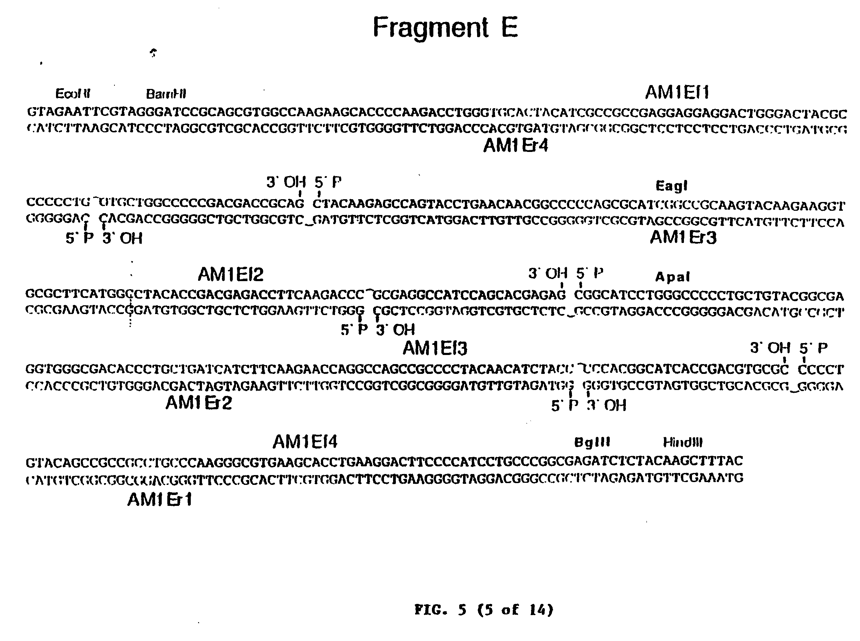

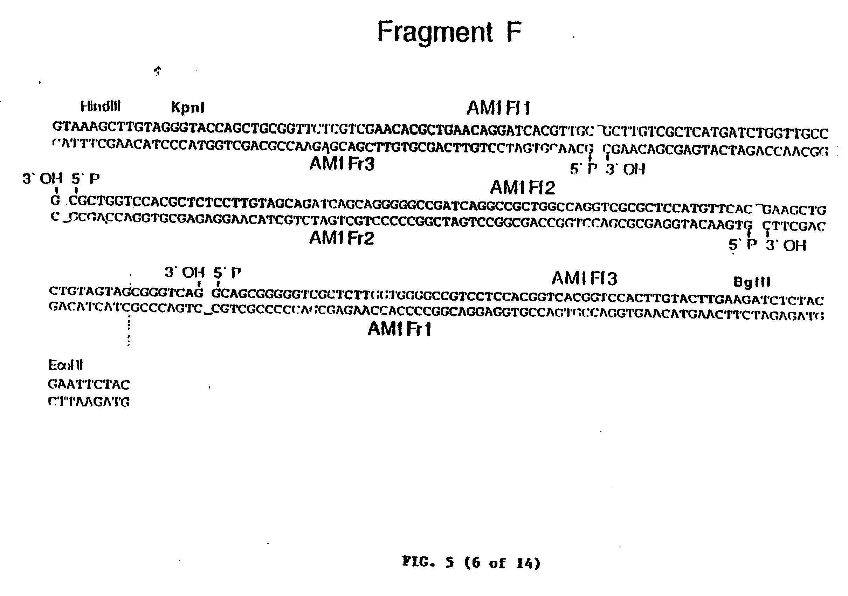

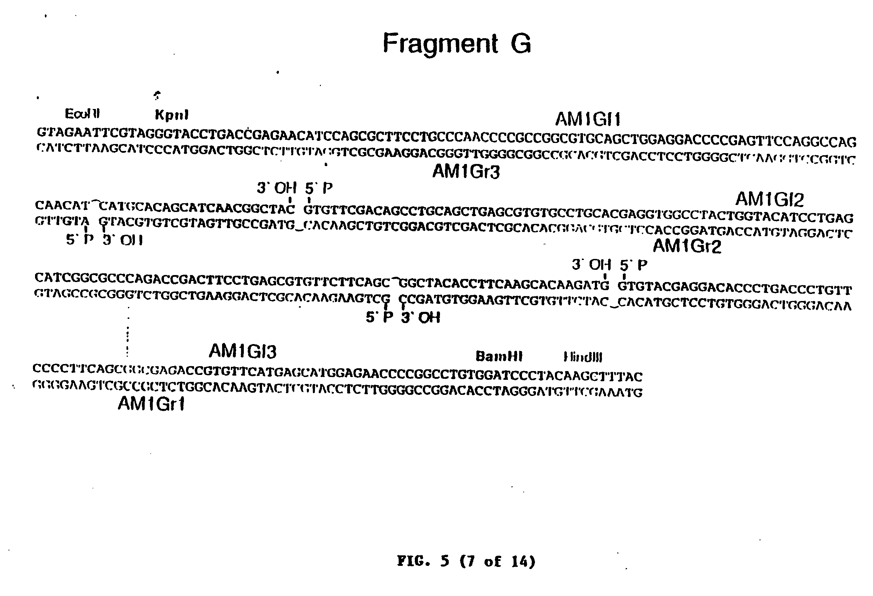

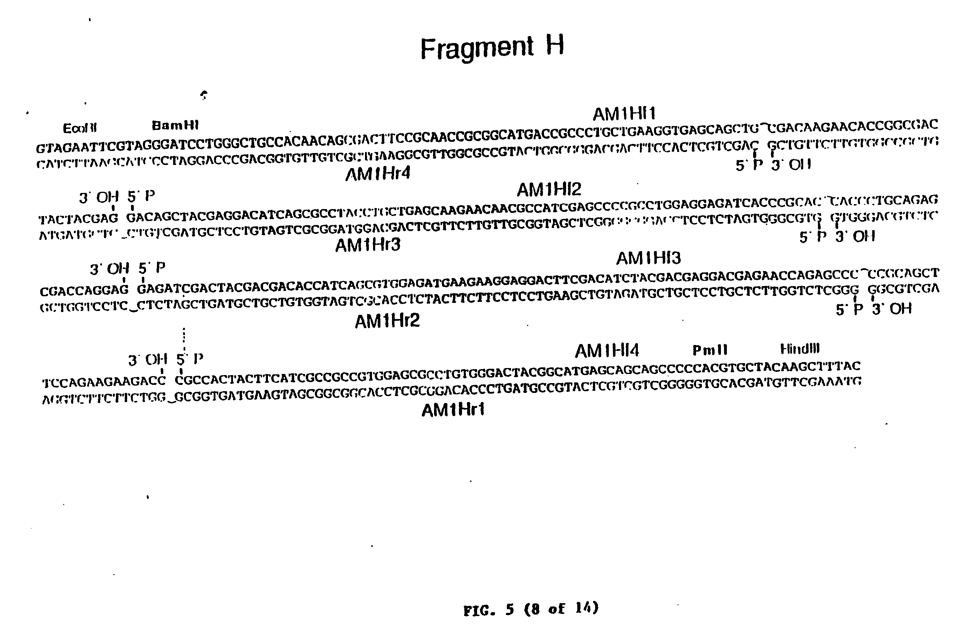

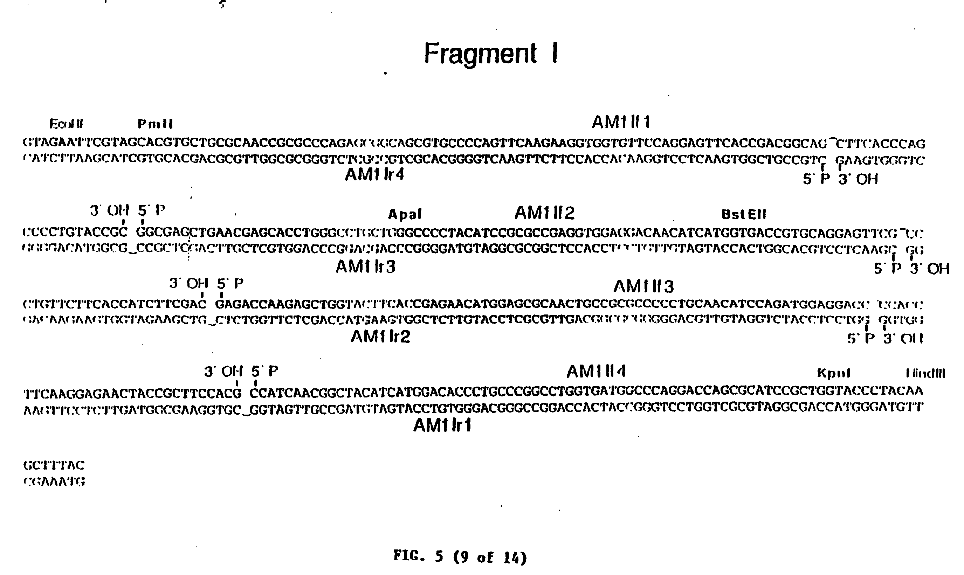

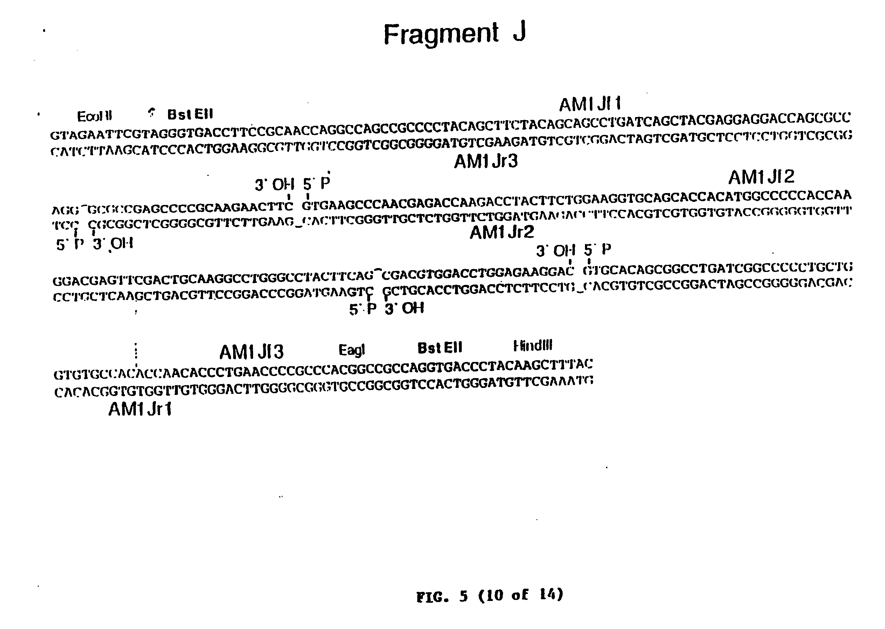

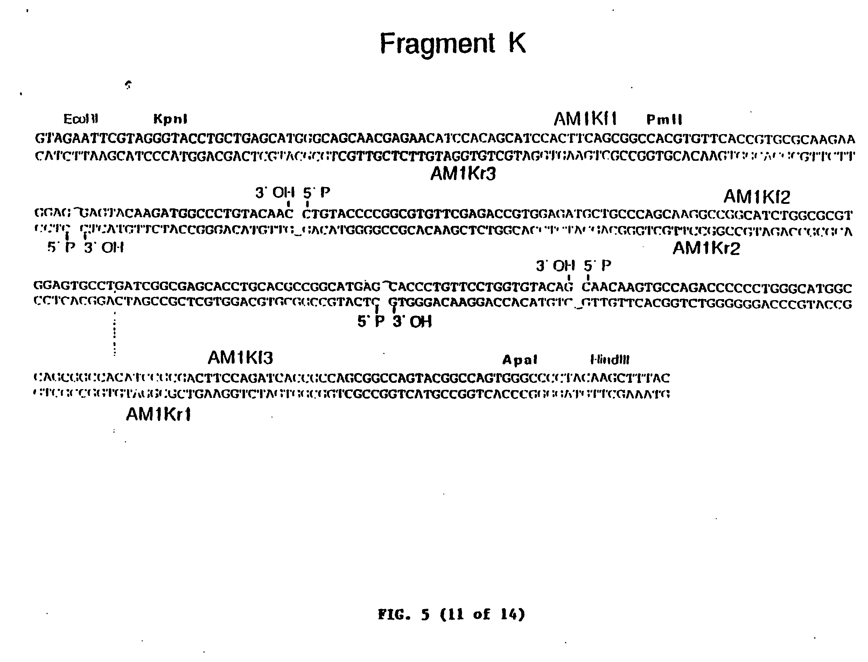

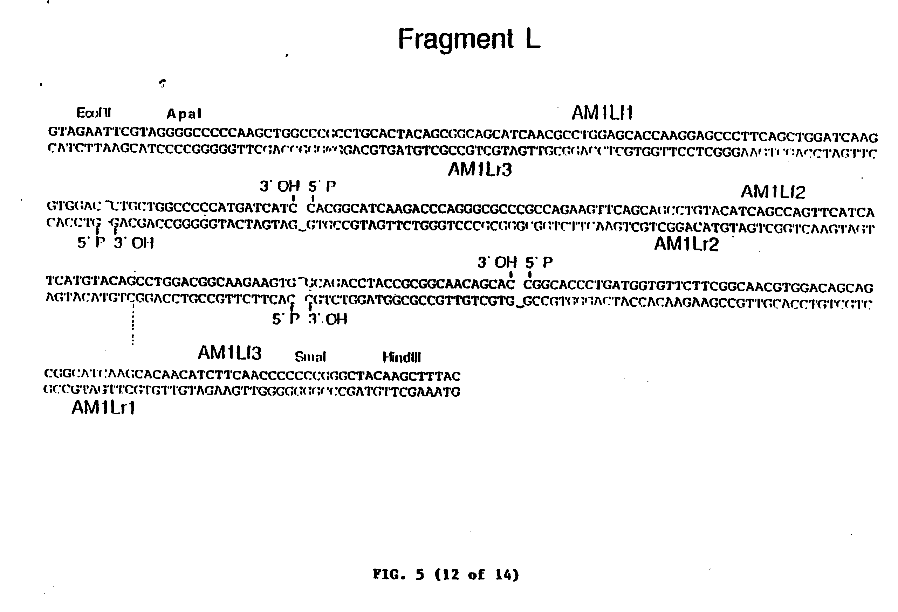

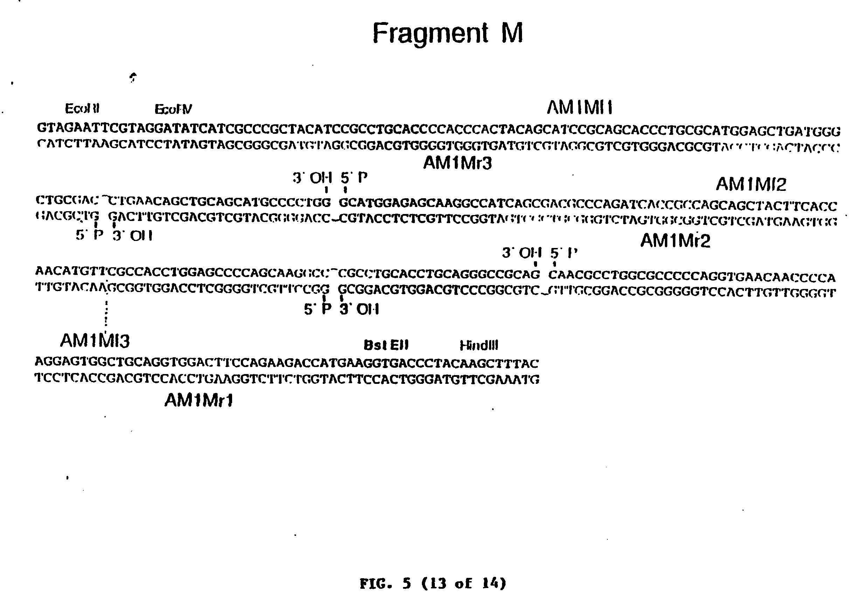

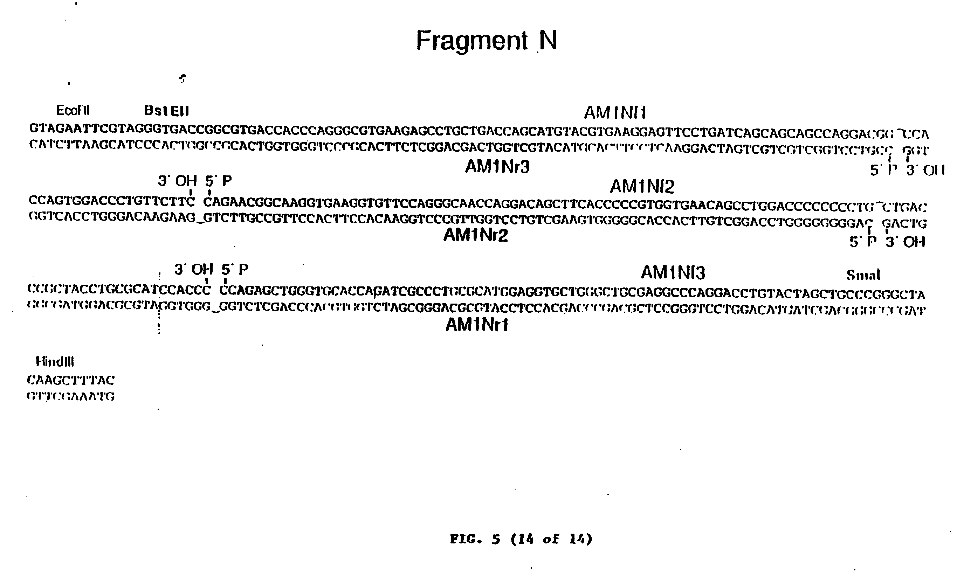

The fourteen gene fragments of the B-domain-deleted-FVIII optimized cDNA listed in Table 2 and shown in Figure 5 (Fragment A-Fragment N) were made as follows. 92 oligonucleotides were made by oligonucleotide synthesis on an ABI 391 synthesizer (Perkin Elmer). The 92 oligonucleotides are listed in Table 3. Figure 5 shows how these 92 oligonucleotides anneal to form the fourteen gene fragments of Table 2. For each strand of each gene fragment, the first oligonucleotide (i.e. the most 5') was manufactured with a 5'-hydroxyl terminus, and the subsequent oligonucleotides were manufactured as 5'-phosphorylated to allow the ligation of adjacent annealed oligonucleotides. For gene fragments A, B, C, F, G, J, K, L, M and N, six oligonucleotides were annealed, ligated, digested with EcoRI and HindIII and cloned into pUC18 digested with EcoRI and HindIII. For gene fragments D, E, H and I, eight oligonucleotides were annealed, ligated, digested with EcoRI and HindIII and cloned into pUC18 digested with EcoRI and HindIII. This procedure generated fourteen different plasmids-pAM1A through pAM1N.

**Table 2**

| **Fragment** | **5' end** | | **3' end** | | **Note** |
|---|---|---|---|---|---|
| A | NheI | 1 | ApaI | 279 | |
| B | ApaI | 279 | Pm1I | 544 | |
| C | Pm1I | 544 | Pm1I | 829 | |
| D | Pm1I | 829 | Bg1II(/BamHI) | 1172 | BamHI site 3' to seq |
| E | (Bg1II/)Bam HI | 1172 | Bg1II | 1583 | |
| F | Bg1II | 1583 | KpnI | 1817 | |
| G | KpnI | 1817 | BamHI | 2126 | |
| H | BamHI | 2126 | Pm1I | 2491 | |
| I | Pm1I | 2491 | KpnI | 3170 | ΔBstEII 2661-2955 |
| J | BstEII | 2661 | BstEII | 2955 | |
| K | KpnI | 3170 | ApaI | 3482 | |
| L | ApaI | 3482 | SmaI(/EcoRV) | 3772 | |
| M | (SmaI/)EcoR V | 3772 | BstEII | 4062 | |
| N | BstEII | 4062 | SmaI | 4348 | |

In Table 2 the restriction site positions are numbered by the first base of the palindrome; numbering begins at the NheI site.

**Table 3**

| **Oligo' Name** | **Oligo' Lengt h** | Oligonucleotide Sequence |
|---|---|---|
| AM1Af 1 | 118 | GTAGAATTCGTAGGCTAGCATGCAGATCGAGCTGAGCACCTGCTTCTTCC TGTGCCTGCTGCGCTTCTGCTTCAGCGCCACCCGCCGCTACTACCTGGGCGC CGTGGAGCTGAGCTGG (SEQ ID NO:7) |
| AM1Af 2 | 104 | GACTACATGCAGAGCGACCTGGGCGAGCTGCCCGTGGACGCCCGCTTCCC CCCCCGCGTGCCCAAGAGCTTCCCCTTCAACACCAGCGTGGTGTACAAGAA |
| | | GAC (SEQ ID NO: 8) |
| AM1Af 3 | 88 | CCTGTTCGTGGAGTTCACCGACCACCTGTTCAACATCGCCAAGCCCCGCC CCCCCTGGATGGGCCTGCTGGGCCCCTACAAGCTTTAC (SEQ ID NO: 9) |
| AM1Ar 1 | 119 | GTAAAGCTTGTAGGGGCCCAGCAGGCCCATCCAGGGGGGCGGGGCTTG GCGATGTTGAACAGGTGGTCGGTGAACTCCACGAACAGGGTCTTCTTGTAC ACCACGCTGGTGTTGAAGG (SEQ ID NO: 10) |
| AM1Ar 2 | 107 | GGAAGCTCTTGGGCACGCGGGGGGGAAGCGGCGTCCACGGCAGCTC GCCCAGGTCGCTCTGCATGTAGTCCCAGCTCAGCTCCACGGCGCCCAGGTA GTAGCGG (SEQ ID NO: 11) |
| AM1Ar 3 | 84 | CGGGTGGCGCTGAAGCAGAAGCGCAGCAGGCACAGGAAGAAGCAGGTG CTCAGCTCGATCTGCATGCTAGCCTACGAATTCTAC (SEQ ID NO: 12) |
| AM1Bf 1 | 115 | GTAGAATTCGTAGGGGCCCCACCATCCAGGCCGAGGTGTACGACACCGT GGTGATCACCCTGAAGAACATGGCCAGCCACCCCGTGAGCCTGCACGCCGT GGGCGTGAGCTACTG (SEQ ID NO: 13) |
| AM1Bf 2 | 103 | GAAGGCCAGCGAGGGCGCCGAGTACGACGACCAGACCAGCCAGCGCGA GAAGGAGGACGACAAGGTGTTCCCCGGCGGCAGCCACACCTACGTGTGGC AGGTG (SEQ ID NO: 14) |
| AM1Bf 3 | 79 | CTGAAGGAGAACGGCCCCATGGCCAGCGACCCCCTGTGCCTGACCTACA GCTACCTGAGCCACGTGCTACAAGCTTTAC (SEQ ID NO: 15) |
| AM1Br 1 | 107 | GTAAAGCTTGTAGCACGTGGCTCAGGTAGCTGTAGGTCAGGCACAGGGG GTCGCTGGCCATGGGGCCGTTCTCCTTCAGCACCTGCCACACGTAGGTGTG GCTGCCG(SEQ ID NO: 16) |
| AM1Br 2 | 101 | CCGGGGAACACCTTGTCGTCCTCCTTCTCGCGCTGGCTGGTCTGGTCGTCG TACTCGGCGCCCTCGCTGGCCTTCCAGTAGCTCACGCCCACGGCGTGCAG (SEQ ID NO: 17) |
| AM1Br 3 | 89 | GCTCACGGGGTGGCTGGCCATGTTCTTCAGGGTGATCACCACGGTGTCGT ACACCTCGGCCTGGATGGTGGGGCCCCTACGAATTCTAC (SEQ ID NO: 18) |
| AM1Cf 1 | 122 | GTAGAATTCGTAGCCACGTGGACCTGGTGAAGGACCTGAACAGCGGCCT GATCGGCGCCCTGCTGGTGTGCCGCGAGGGCAGCCTGGCCAAGGAGAAGA CCCAGACCCTGCACAAGTTCATC (SEQ ID NO: 19) |
| AM1Cf 2 | 110 | CTGCTGTTCGCCGTGTTCGACGAGGGCAAGAGCTGGCACAGCGAGACCA AGAACAGCCTGATGCAGGACCGCGACGCCGCCAGCGCCCGCGCCTGGCCC AAGATGCACAC (SEQ ID NO: 20) |
| AM1Cf 3 | 86 | CGTGAACGGCTACGTGAACCGCAGCCTGCCCGGCCTGATCGGCTGCCACC GCAAGAGCGTGTACTGGCACGTGCTACAAGCTTTAC (SEQ ID NO: 21) |
| AM1Cr 1 | 108 | GTAAAGCTTGTAGCACGTGCCAGTACACGCTCTTGCGGTGGCAGCCGATC AGGCCGGGCAGGCTGCGGTTCACGTAGCCGTTCACGGTGTGCATCTTGGGC CAGGCGC (SEQ ID NO: 22) |
| AM1Cr 2 | 110 | GGGCGCTGGCGGCGTCGCGGTCCTGCATCAGGCTGTTCTTGGTCTCGCTG TGCCAGCTCTTGCCCTCGTCGAACACGGCGAACAGCAGGATGAACTTGTGC AGGGTCTGG (SEQ ID NO: 23) |
| AM1Cr 3 | 100 | GTCTTCTCCTTGGCCAGGCTGCCCTCGCGGCACACCAGCAGGGCGCCGAT CAGGCCGCTGTTCAGGTCCTTCACCAGGTCCACGTGGCTACGAATTCTAC (SEQ ID NO: 24) |
| AM1Df | 99 | GTAGAATTCGTAGCACGTGATCGGCATGGGCACCACCCCCGAGGTGCAC |
| 1 | | AGCATCTTCCTGGAGGGCCACACCTTCCTGGTGCGCAACCACCGCCAGGC (SEQ ID NO: 25) |
| AM1Df 2 | 100 | CAGCCTGGAGATCAGCCCCATCACCTTCCTGACCGCCCAGACCCTGCTGA TGGACCTGGGCCAGTTCCTGCTGTTCTGCCACATCAGCAGCCACCAGCAC (SEQ ID NO: 26) |
| AM1Df 3 | 101 | GACGGCATGGAGGCCTACGTGAAGGTGGACAGCTGCCCCGAGGAGCCCC AGCTGCGCATGAAGAACAACGAGGAGGCCGAGGACTACGACGACGACCTG AC (SEQ ID NO: 27) |
| AM1Df 4 | 84 | CGACAGCGAGATGGACGTGGTGCGCTTCGACGACAACAGCCCCAGC TTCATCCAGATCTCTACGGATCCTACAAGCTTTAC (SEQ ID NO: 28) |
| AM1Dr 1 | 109 | GTAAAGCTTGTAGGATCCGTAGAGATCTGGATGAAGCTGGGGCTGTTGTC GTCGTCGAAGCGCACCACGTCCATCTCGCTGTCGGTCAGGTCGTCGTCGTA GTCCTCGG (SEQ ID NO: 29) |
| AM1Dr 2 | 101 | CCTCCTCGTTGTTCTTCATGCGCAGCTGGGGCTCCTCGGGGCAGCTGTCCA CCTTCACGTAGGCCTCCATGCCGTCGTGCTGGTGGCTGCTGATGTGGCAG (SEQ ID NO: 30) |
| AMIDr 3 | 102 | AACAGCAGGAACTGGCCCAGGTCCATCAGCAGGGTCTGGGCGGTCAGGA AGGTGATGGGGCTGATCTCCAGGCTGGCCTGGCGGTGGTTGCGCACCAGG AAG (SEQ ID NO: 31) |
| AM1Dr 4 | 72 | GTGTGGCCCTCCAGGAAGATGCTGTGCACCTCGGGGTGGTGCCCATGCC GATCACGTGCTACGAATTCTAC (SEQ ID NO: 32) |
| AM1Ef 1 | 122 | GTAGAATTCGTAGGATCCGCAGCGTGGCCAAGAAGCACCCCAAGACCT GGGTGCACTACATCGCCGCCGAGGAGGAGGACTGGGACTACGCCCCCCTG GTGCTGGCCCCCGACGACCGCAG (SEQ ID NO: 33) |
| AM1Ef 2 | 120 | CTACAAGAGCCAGTACCTGAACAACGGCCCCCAGCGCATCGGCCGCAAG TACAAGAAGGTGCGCTTCATGGCCTACACCGACGAGACCTTCAAGACCCGC GAGGCCATCCAGCACGAGAG (SEQ ID NO: 34) |
| AM1Ef 3 | 115 | CGGCATCCTGGCCCCCTGCTGTACGGCGAGGTGGCGACACCCTGCTGA TCATCTTCAAGAACCAGGCCAGCCGCCCCTACAACATCTACCCCCACGGCA TCACCGACGTGCGC (SEQ ID NO: 35) |
| AM1Ef 4 | 86 | CCCCTGTACAGCCGCCGCCTGCCCAAGGGCGTGAAGCACCTGAAGGACTT CCCCATCCTGCCCGGCGAGATCTCTACAAGCTTTAC (SEQ ID NO: 36) |
| AM1Er 1 | 109 | GTAAAGCTTGTAGAGATCTCGCCGGCAGGATGGGAAGTCCTTCAGGT GCTTCACGCCCTTGGGCAGGCGGCGGCTGTACAGGGGGCGCACGTCGGTG ATGCCGTGGG (SEQ ID NO: 37) |
| AM1Er 2 | 114 | GGTAGATGTTGTAGGGGCGGCTGGCCTGGTTCTTGAAGATGATCAGCAGG GTGTCGCCCACCTCGCCGTACAGCAGGGGGCCCAGGATGCCGCTCTCGTGC TGGATGGCCTCGC (SEQ ID NO: 38) |
| AM1Er 3 | 121 | GGGTCTTGAAGGTCTCGTCGGTGTAGGCCATGAAGCGCACCTTCTTGTAC TTGCGGCCGATGCGCTGGGGGCCGTTGTTCAGGTACTGGCTCTTGTAGCTG CGGTCGTCGGGGGCCAGCAC (SEQ ID NO: 39) |
| AM1Er 4 | 99 | CAGGGGGCGTAGTCCCAGTCCTCCTCCTCGGCGGCGATGTAGTGCACCC AGGTCTTGGGGTGCTTCTTGGCCACGCTGCGGATCCCTACGAATTCTAC (SEQ ID NO: 40) |
| AM1Ff1 | 102 | GTAGAATTCGTAGAGATCTTCAAGTACAAGTGGACCGTGACCGTGGAGG |
| | | ACGGCCCCACCAAGAGCGACCCCCGCTGCCTGACCCGCTACTACAGCAGCT TC (SEQ ID NO: 41) |
| AM1Ff 2 | 103 | GTGAACATGGAGCGCGACCTGGCCAGCGGCCTGATCGGCCCCCTGCTGAT CTGCTACAAGGAGAGCGTGGACCAGCGCGGCAACCAGATCATGAGCGACA AGC (SEQ ID NO: 42) |
| AM1Ff 3 | 61 | GCAACGTGATCCTGTTCAGCGTGTTCGACGAGAACCGCAGCTGGTACCCT ACAAGCTTTAC (SEQ ID NO: 43) |
| AM1Fr 1 | 87 | GTAAAGCTTGTAGGGTACCAGCTGCGGTTCTCGTCGAACACGCTGAACAG GATCACGTTGCGCTTGTCGCTCATGATCTGGTTGCCG (SEQ ID NO: 44) |
| AM1Fr 2 | 101 | CGCTGGTCCACGCTCTCCTTGTAGCAGATCAGCAGGGGGCCGATCAGGCC GCTGGCCAGGTCGCGCTCCATGTTCACGAAGCTGCTGTAGTAGCGGGTCAG (SEQ ID NO: 45) |
| AM1Fr 3 | 78 | GCAGCGGGGGTCGCTCTTGGTGGGCCGTCCTCCACGGTCACGGTCCACT TGTACTTGAAGATCTCTACGAATTCTAC (SEQ ID NO: 46) |
| AM1Gf 1 | 120 | GTAGAATTCGTAGGGTACCTGACCGAGAACATCCAGCGCTTCCTGCCCAA CCCCGCCGGCGTGCAGCTGGAGGACCCCGAGTTCCAGGCCAGCAACATCA TGCACAGCATCAACGGCTAC (SEQ ID NO: 47) |
| AM1Gf 2 | 126 | GTGTTCGACAGCCTGCAGCTGAGCGTGTGCCTGCACGAGGTGGCCTACTG GTACATCCTGAGCATCGGCGCCCAGACCGACTTCCTGAGCGTGTTCTTCAG CGGCTACACCTTCAAGCACAAGATG (SEQ ID NO: 48) |
| AM1Gf 3 | 95 | GTGTACGAGGACACCCTGACCCTGTTCCCCTTCAGCGGCGAGACCGTGTT CATGAGCATGGAGAACCCCGGCCTGTGGATCCCTACAAGCTTTAC (SEQ ID NO: 49) |
| AM1Gr 1 | 119 | GTAAAGCTTGTAGGGATCCACAGGCCGGGTTCTCCATGCTCATGAACAC GGTCTCGCCGCTGAAGGGAACAGGGTCAGGGTGTCCTCGTACACCATCTT GTGCTTGAAGGTGTAGCC (SEQ ID NO: 50) |
| AM1Gr 2 | 124 | GCTGAAGAACACGCTCAGGAAGTCGGTCTGGGCGCCGATGCTCAGGATG TACCAGTAGGCCACCTCGTGCAGGCACACGCTCAGCTGCAGGCTGTCGAAC ACGTAGCCGTTGATGCTGTGCATG (SEQ ID NO: 51) |
| AM1Gr 3 | 98 | ATGTTGCTGGCCTGGAACTCGGGGTCCTCCAGCTGCACGCCGGCGGGGTT GGGCAGGAAGCGCTGGATGTTCTCGGTCAGGTACCCTACGAATTCTAC (SEQ ID NO: 52) |
| AM1Hf 1 | 111 | GTAGAATTCGTAGGGATCCTGGGCTGCCACAACAGCGACTTCCGCAACCG CGGCATGACCGCCCTGCTGAAGGTGAGCAGCTGCGACAAGAACACCGGCG ACTACTACGAG (SEQ ID NO: 53) |
| AM1Hf 2 | 102 | GACAGCTACGAGGACATCAGCGCCTACCTGCTGAGCAAGAACAACGCCA TCGAGCCCCGCCTGGAGGAGATCACCCGCACCACCCTGCAGAGCGACCAG GAG (SEQ ID NO: 54) |
| AM1Hf 3 | 105 | GAGATCGACTACGACGACACCATCAGCGTGGAGATGAAGAAGGAGGACT TCGACATCTACGACGAGGACGAGAACCAGAGCCCCGCAGCTTCCAGAAG AAGACC (SEQ ID NO: 55) |
| AM1Hf 4 | 79 | CGCCACTACTTCATCGCCGCCGTGGAGCGCCTGTGGGACTACGGCATGAG CAGCAGCCCCCACGTGCTACAAGCTTTAC (SEQ ID NO: 56) |
| AM1Hr 1 | 101 | GTAAAGCTTGTAGCACGTGGGGCTGCTGCTCATGCCGTAGTCCCACAGG CGCTCCACGGCGGCGATGAAGTAGTGGCGGTCTTCTTCTGGAAGCTGCGG |
| | | (SEQ ID NO: 57) |
| AM1Hr 2 | 105 | GGGCTCTGGTTCTCGTCCTCGTCGTAGATGTCGAAGTCCTCCTTCTTCATC TCCACGCTGATGGTGTCGTCGTAGTCGATCTCCTCCTGGTCGCTCTGCAGGG TG (SEQ ID NO: 58) |
| AM1Hr 3 | 108 | GTGCGGGTGATCTCCTCCAGGCGGGGCTCGATGGCGTTGTTCTTGCTCAG CAGGTAGGCGCTGATGTCCTCGTAGCTGTCCTCGTAGTAGTCGCCGGTGTT CTTGTCG (SEQ ID NO: 59) |
| AM1Hr 4 | 83 | CAGCTGCTCACCTTCAGCAGGGCGGTCATGCCGCGGTTGCGGAAGTCGCTGTTGTGGCAG CCCAGGATCCCTACGAATTCTAC (SEQ ID NO: 60) |
| AM1If 1 | 115 | GTAGAATTCGTAGCACGTGCTGCGCAACCGCGCCCAGAGCGGCAGCGTG CCCCAGTTCAAGAAGGTGGTGTTCCAGGAGTTCACCGACGGCAGCTTCACC CAGCCCCTGTACCGC (SEQ ID NO: 61) |
| AM1If 2 | 111 | GGCGAGCTGAACGAGCACCTGGGCCTGCTGGGCCCCTACATCCGCGCCG AGGTGGAGGACAACATCATGGTGACCGTGCAGGAGTTCGCCCTGTTCTTCA CCATCTTCGAC (SEQ ID NO: 62) |
| AM1If 3 | 106 | GAGACCAAGAGCTGGTACTTCACCGAGAACATGGAGCGCAACTGCCGCG CCCCCTGCAACATCCAGATGGAGGACCCCACCTTCAAGGAGAACTACCGCT TCCACG (SEQ ID NO: 63) |
| AM1If 4 | 85 | CCATCAACGGCTACATCATGGACACCCTGCCCGGCCTGGTGATGGCCCAG GACCAGCGCATCCGCTGGTACCCTACAAGCTTTAC (SEQ ID NO: 64) |
| AM1Ir 1 | 115 | GTAAAGCTTGTAGGGTACCAGCGGATGCGCTGGTCCTGGGCCATCACCAG GCCGGGCAGGGTGTCCATGATGTAGCCGTTGATGGCGTGGAAGCGGTAGTT CTCCTTGAAGGTGG (SEQ ID NO: 65) |
| AM1Ir 2 | 99 | GGTCCTCCATCTGGATGTTGCAGGGGGCGCGGCAGTTGCGCTCCATGTTC TCGGTGAAGTACCAGCTCTTGGTCTCGTCGAAGATGGTGAAGAACAGGG (SEQ ID NO: 66) |
| AM1Ir 3 | 110 | CGAACTCCTGCACGGTCACCATGATGTTGTCCTCCACCTCGGCGCGGATG TAGGGGCCCAGCAGGCCCAGGTGCTCGTTCAGCTCGCCGCGGTACAGGGG CTGGGTGAAG (SEQ ID NO: 67) |
| AM1Ir 4 | 93 | CTGCCGTCGGTGAACTCCTGGAACACCACCTTCTTGAACTGGGCACGCT GCCGCTCTGGGCGCGGTTGCGCAGCACGTGCTACGAATTCTAC (SEQ ID NO: 68) |
| AM1Jf 1 | 116 | GTAGAATTCGTAGGGTGACCTTCCGCAACCAGGCCAGCCGCCCCTACAGCTTCTACAGCA GCCTGATCAGCTACGAGGAGGACCAGCGCCAGGGCGCCGAGCCCCGCAAGAACTTC (SEQ ID NO: 69) |
| AM1Jf 2 | 120 | GTGAAGCCCAACGAGACCAAGACCTACTTCTGGAAGGTGCAGCACCACA TGGCCCCCACCAAGGACGAGTTCGACTGCAAGGCCTGGGCCTACTTCAGCG ACGTGGACCTGGAGAAGGAC (SEQ ID NO: 70) |
| AM1Jf 3 | 91 | GTGCACAGCGGCCTGATCGGCCCCCTGCTGGTGTGCCACACCAACACCCT GAACCCCGCCCACGGCCGCCAGGTGACCCTACAAGCTTTAC (SEQ ID NO: 71) |
| AM1Jr 1 | 113 | GTAAAGCTTGTAGGGTCACCTGGCGGCCGTGGGCGGGGTTCAGGGTGTTG GTGTGGCACACCAGCAGGGGGCCGATCAGGCCGCTGTGCACGTCCTTCTCC AGGTCCACGTCG (SEQ ID NO: 72) |
| AM1Jr2 | 121 | CTGAAGTAGGCCCAGGCCTTGCAGTCGAACTCGTCCTTGGTGGGGGCCAT |
| | | GTGGTGCTGCACCTTCCAGAAGTAGGTCTTGGTCTCGTTGGGCTTCACGAA GTTCTTGCGGGGCTCGGCGC (SEQ ID NO: 73) |
| AM1Jr 3 | 93 | CCTGGCGCTGGTCCTCCTCGTAGCTGATCAGGCTGCTGTAGAAGCTGTAG GGGCGGCTGGCCTGGTTGCGGAAGGTCACCCTACGAATTCTAC (SEQ ID NO: 74) |
| AM1Kf 1 | 120 | GTAGAATTCGTAGGGTACCTGCTGAGCATGGGCAGCAACGAGAACATCC ACAGCATCCACTTCAGCGGCCACGTGTTCACCGTGCGCAAGAAGGAGGAG TACAAGATGGCCCTGTACAAC (SEQ ID NO: 75) |
| AM1Kf 2 | 122 | CTGTACCCCGGCGTGTTCGAGACCGTGGAGATGCTGCCCAGCAAGGCCGG CATCTGGCGCGTGGAGTGCCTGATCGGCGAGCACCTGCACGCCGGCATGA GCACCCTGTTCCTGGTGTACAG (SEQ ID NO: 76) |
| AM1Kf 3 | 102 | CAACAAGTGCCAGACCCCCCTGGGCATGGCCAGCGGCCACATCCGCGAC TTCCAGATCACCGCCAGCGGCCAGTACGGCCAGTGGGCCCCTACAAGCTTT AC (SEQ ID NO: 77) |
| AM1Kr 1 | 123 | GTAAAGCTTGTAGGGGCCCACTGGCCGTACTGGCCGCTGGCGGTGATCTG GAAGTCGCGGATGTGGCCGCTGGCCATGCCCAGGGGGGTCTGGCACTTGTT GCTGTACACCAGGAACAGGGTG (SEQ ID NO: 78) |
| AM1Kr 2 | 125 | CTCATGCCGGCGTGCAGGTGCTCGCCGATCAGGCACTCCACGCGCCAGAT GCCGGCCTTGCTGGGCAGCATCTCCACGGTCTCGAACACGCCGGGTACAG GTTGTACAGGGCCATCTTGTACTC (SEQ ID NO: 79) |
| AM1Kr 3 | 96 | CTCCTTCTTGCGCACGGTGAACACGTGGCCGCTGAAGTGGATGCTGTGGA TGTTCTCGTTGCTGCCCATGCTCAGCAGGTACCCTACGAATTCTAC (SEQ ID NO: 80) |
| AM1Lf 1 | 120 | GTAGAATTCGTAGGGGCCCCCAAGCTGGCCCGCCTGCACTACAGCGGCA GCATCAACGCCTGGAGCACCAAGGAGCCCTTCAGCTGGATCAAGGTGGAC CTGCTGGCCCCCATGATCATC (SEQ ID NO: 81) |
| AM1Lf 2 | 116 | CACGGCATCAAGACCCAGGGCGCCCGCCAGAAGTTCAGCAGCCTGTACA TCAGCCAGTTCATCATCATGTACAGCCTGGACGGCAAGAAGTGGCAGACCT ACCGCGGCAACAGCAC (SEQ ID NO: 82) |
| AM1Lf 3 | 86 | CGGCACCCTGATGGTGTTCTTCGGCAACGTGGACAGCAGCGGCATCAAGC ACAACATCTTCAACCCCCCGGGCTACAAGCTTTAC (SEQ ID NO: 83) |
| AM1Lr 1 | 110 | GTAAAGCTTGTAGCCCGGGGGGTTGAAGATGTTGTGCTTGATGCCGCTG CTGTCCACGTTGCCGAAGAACACCATCAGGGTGCCGGTGCTGTTGCCGCGG TAGGTCTGC (SEQ ID NO: 84) |
| AM1Lr 2 | 113 | CACTTCTTGCCGTCCAGGCTGTACATGATGATGAACTGGCTGATGTACAG GCTGCTGAACTTCTGGCGGCGCCCTGGGTCTTGATGCCGTGGATGATCAT GGGGGCCAGCAG (SEQ ID NO: 85) |
| AM1Lr 3 | 99 | GTCCACCTTGATCCAGCTGAAGGCTCCTTGGTGCTCCAGGCGTTGATGC TGCCGCTGTAGTGCAGGCGGGCCAGCTTGGGGGCCCCTACGAATTCTAC (SEQ ID NO: 86) |
| AM1M f1 | 122 | GTAGAATTCGTAGGATATCATCGCCCGCTACATCCGCCTGCACCCCACCC ACTACAGCATCCGCAGCACCCTGCGCATGGAGCTGATGGGCTGCGACCTGA ACAGCTGCAGCATGCCCCTGG (SEQ ID NO: 87) |
| AM1M f2 | 112 | GCATGGAGAGCAAGGCCATCAGCGACGCCCAGATCACCGCCAGCAGCTA CTTCACCAACATGTTCGCCACCTGGAGCCCCAGCAAGGCCCGCCTGCACCT |
| | | GCAGGGCCGCAG (SEQ ID NO: 88) |
| AM1M f3 | 89 | CAACGCCTGGCGCCCCAGGTGAACAACCCCAAGGAGTGGCTGCAGGTG GACTTCCAGAAGACCATGAAGGTGACCCTACAAGCTTTAC (SEQ ID NO: 89) |
| AM1M r1 | 112 | GTAAAGCTTGTAGGGTCACCTTCATGGTCTTCTGGAAGTCCACCTGCAGC CACTCCTTGGGGTTGTTCACCTGGGGGCGCCAGGCGTTGCTGCGGCCCTGC AGGTGCAGGCG (SEQ ID NO: 90) |
| AM1M r2 | 114 | GGCCTTGCTGGGGCTCCAGGTGGCGAACATGTTGGTGAAGTAGCTGCTGG CGGTGATCTGGGCGTCGCTGATGGCCTTGCTCTCCATGCCCAGGGGCATGC TGCAGCTGTTCAG (SEQ ID NO: 91) |
| AM1M r3 | 97 | GTCGCAGCCCATCAGCTCCATGCGCAGGGTGCTGCGGATGCTGTAGTGGG TGGGGTGCAGGCGGATGTAGCGGGCGATGATATCCTACGAATTCTAC (SEQ ID NO: 92) |
| AM1Nf 1 | 122 | GTAGAATTCGTAGGGTGACCGGCGTGACCACCCAGGGCGTGAAGAGCCT GCTGACCAGCATGTACGTGAAGGAGTTCCTGATCAGCAGCAGCCAGGACG GCCACCAGTGGACCCTGTTCTTC (SEQ ID NO: 93) |
| AM1Nf 2 | 104 | CAGAACGGCAAGGTGAAGGTGTTCCAGGGCAACCAGGACAGCTTCACCC CCGTGGTGAACAGCCTGGACCCCCCCTGCTGACCCGCTACCTGCGCATCC ACCC (SEQ ID NO: 94) |
| AM1Nf 3 | 92 | CCAGAGCTGGGTGCACCAGATCGCCCTGCGCATGGAGGTGCTGGGCTGC GAGGCCCAGGACCTGTACTAGCTGCCCGGGCTACAAGCTTTAC (SEQ ID NO: 95) |
| AM1Nr 1 | 118 | GTAAAGCTTGTAGCCCGGGCAGCTAGTACAGGTCCTGGGCCTCGCAGCCC AGCACCTCCATGCGCAGGGCGATCTGGTGCACCCAGCTCTGGGGGTGGATG CGCAGGTAGCGGGTCAG (SEQ ID NO: 96) |
| AM1Nr 2 | 100 | CAGGGGGGGTCCAGGCTGTTCACCACGGGGGTGAAGCTGTCCTGGTTGC CCTGGAACACCTTCACCTTGCCGTTCTGGAAGAACAGGGTCCACTGGTGG .(SEQ ID NO: 97) |
| AM1Nr 3 | 100 | CCGTCCTGGCTGCTGCTGATCAGGAACTCCTTCACGTACATGCTGGTCAG CAGGCTCTTCACGCCCTGGGTGGTCACGCCGGTCACCCTACGAATTCTAC (SEQ ID NO: 98) |

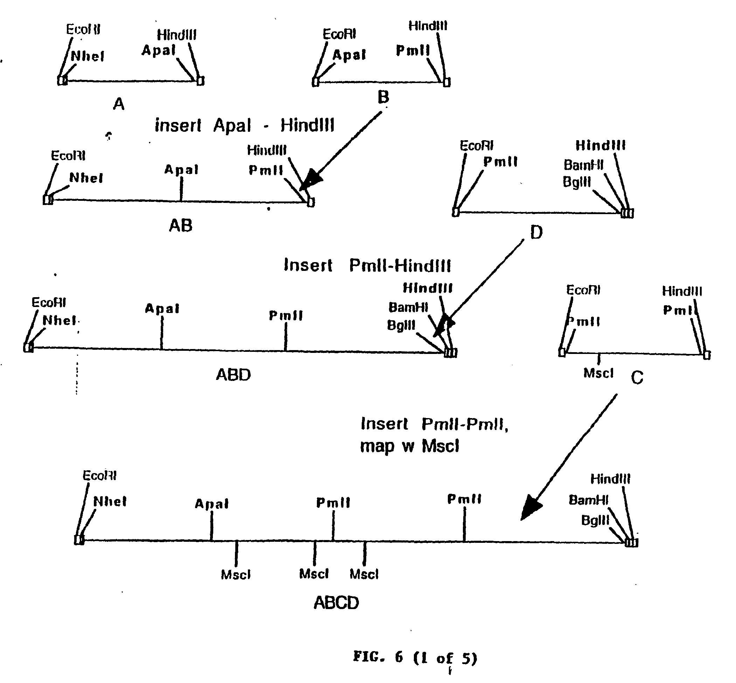

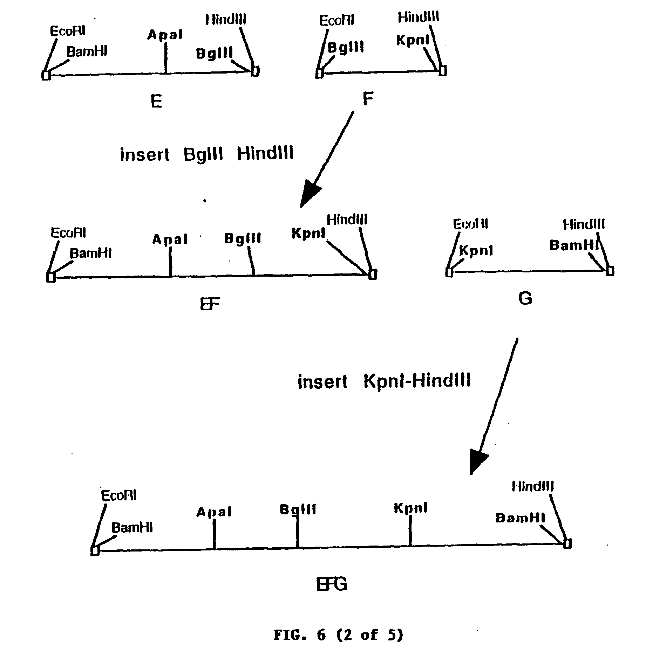

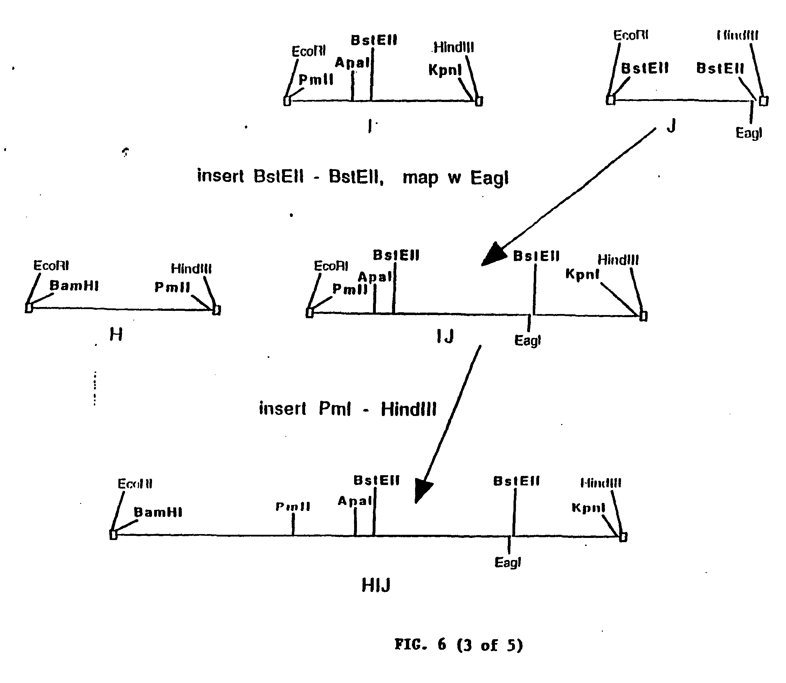

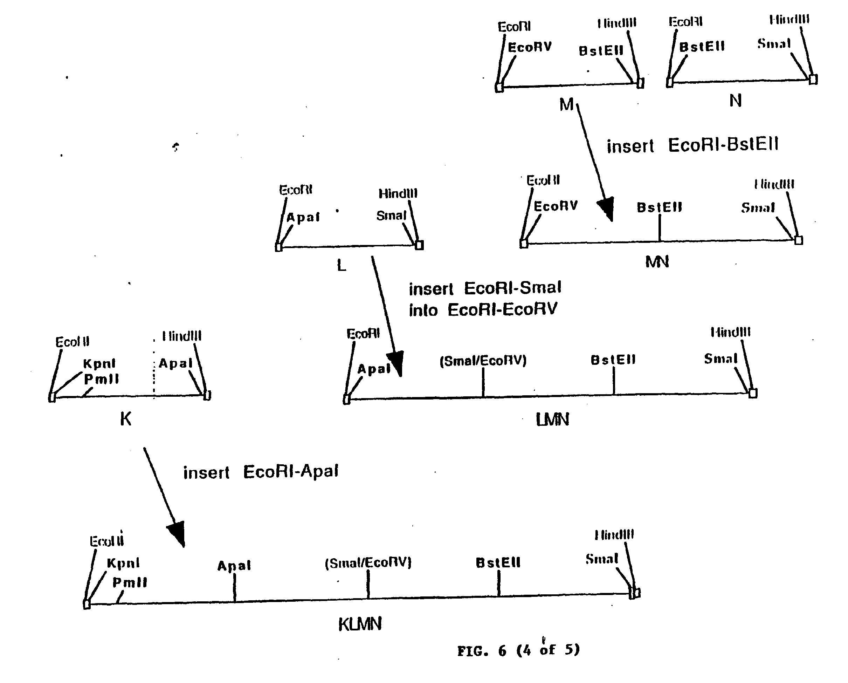

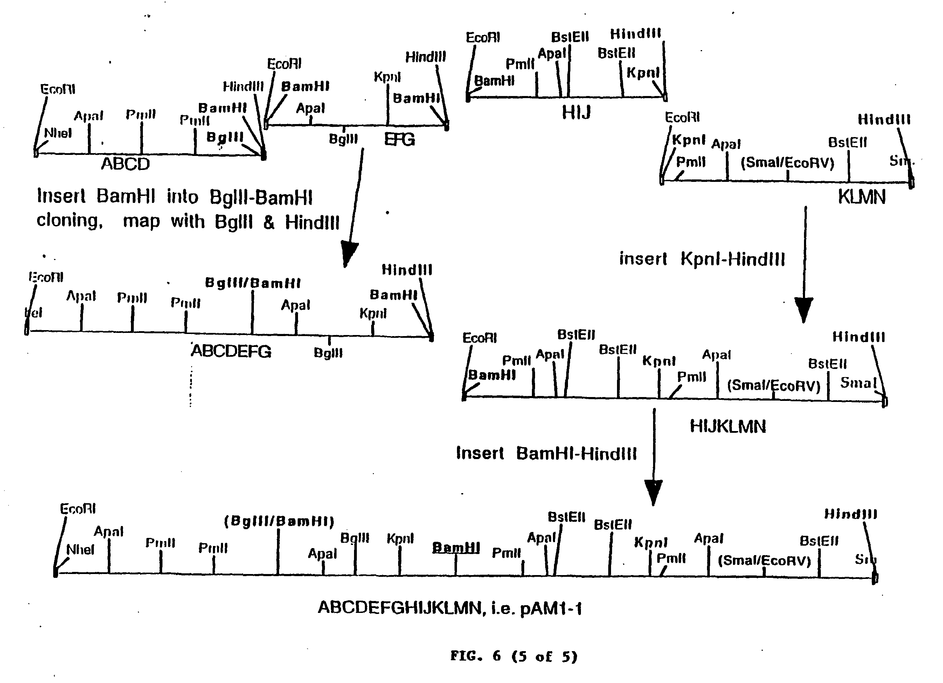

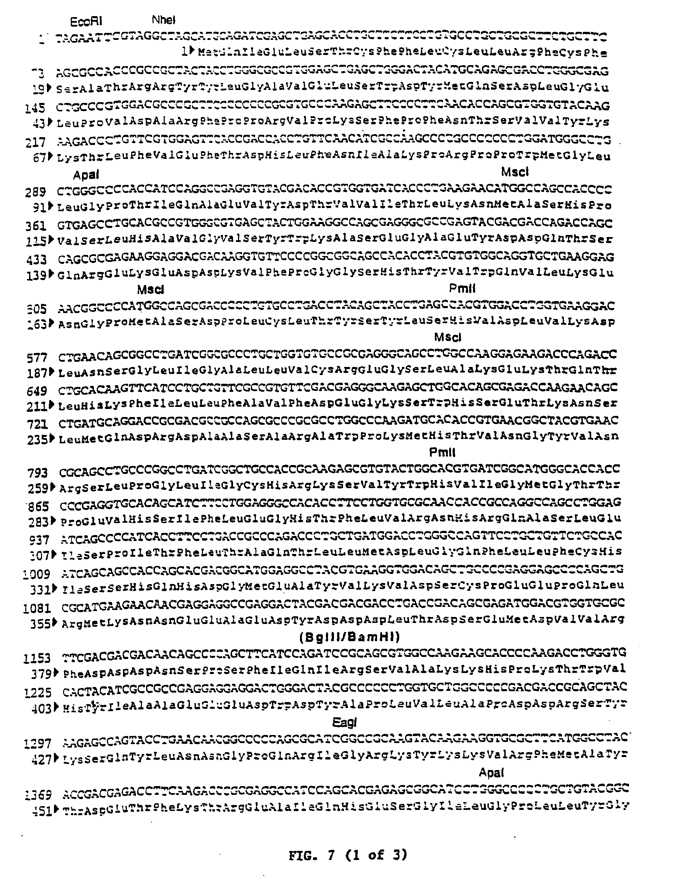

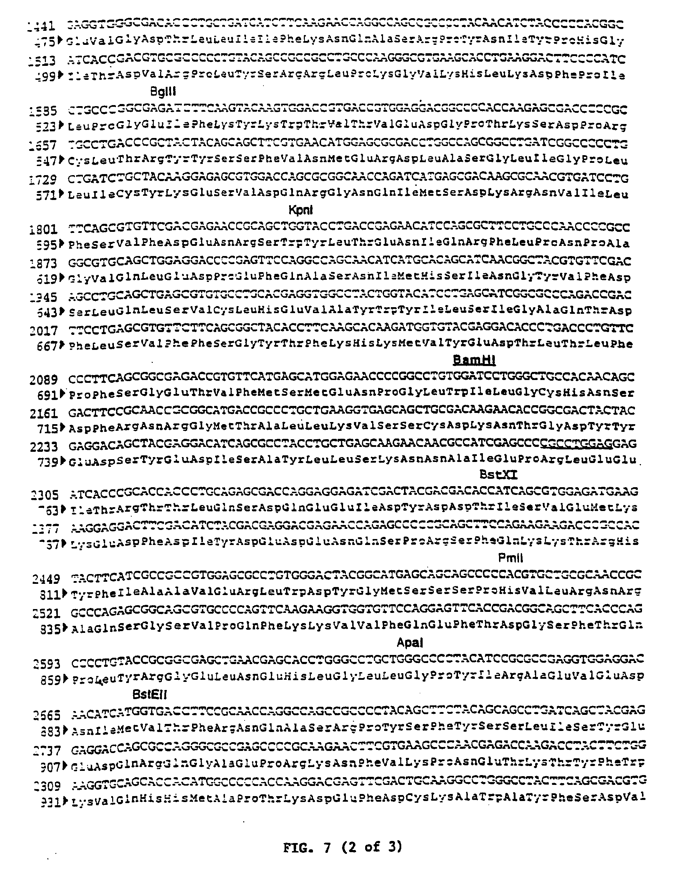

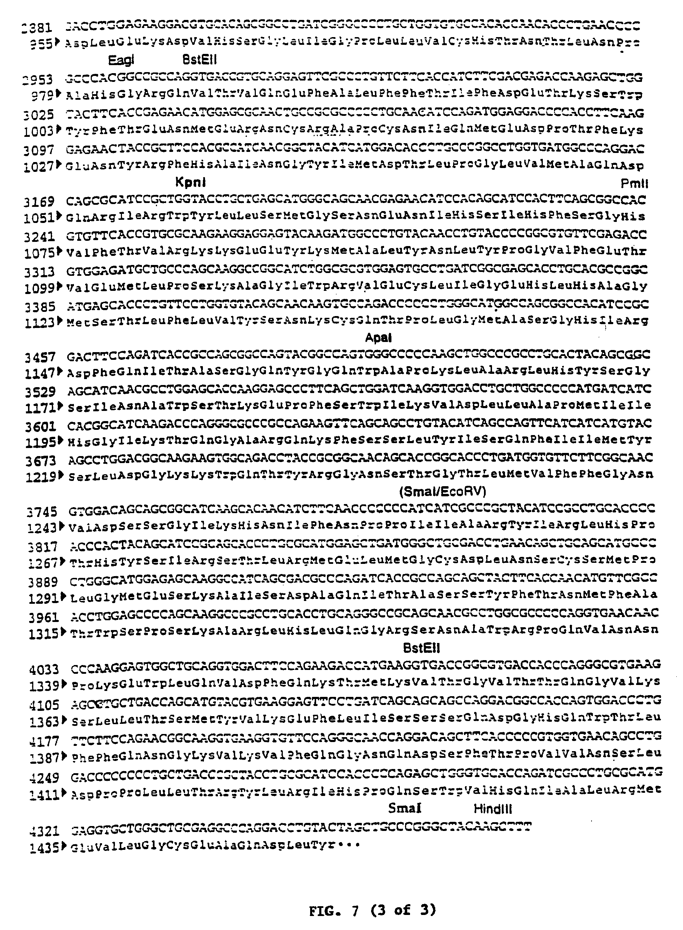

As noted in Table 2 and shown in Figure 5, fragment D was constructed with a BamHI restriction site placed between the BglII site and the HindIII site at the 3' end of the fragment. Fragment I was constructed to carry the DNA from PmlI (2491) to BstEII (2661) followed immediately by the DNA from BstEII (2955) to KpnI (3170), so that the insertion of the BstEII fragment from pAMJ into the BstEII site of pAMI in the correct orientation will generate the desired sequences from 2491 to 3170. Plasmid pAM1B was digested with ApaI and HindIII and the insert was purified by agarose gel electrophoresis and inserted into plasmid pAM1A digested with ApaI and HindIII, generating plasmid pAM1AB. Plasmid pAM1D was digested with PmlI and HindIII and the insert was purified by agarose gel electrophoresis and inserted into plasmid pAM1AB digested with PmlI and HindIII, generating plasmid pAM1ABD. Plasmid pAM1C was digested with PmlI and the insert was purified by agarose gel electrophoresis and inserted into plasmid pAM1ABD digested with PmlI, generating plasmid pAM1ABCD, insert orientation was confirmed by the appearance of a diagnostic 111bp fragment when digested with MscI. Plasmid pAM1F was digested with BglII and HindIII and the insert was purified by agarose gel electrophoresis and inserted into plasmid pAM1E digested with BglII and HindIII, generating plasmid pAM1EF. Plasmid pAM1G was digested with KpnI and HindIII and the insert was purified by agarose gel electrophoresis and inserted into plasmid pAM1EF digested with KpnI and HindIII, generating plasmid pAM1EFG. Plasmid pAM1J was digested with BstEII and the insert was purified by agarose gel electrophoresis and inserted into plasmid pAM1I digested with BstEII, generating plasmid pAM1IJ; orientation was confirmed by the appearance of a diagnostic 465bp fragment when digested with EcoRI and EagI. Plasmid pAM1IJ was digested with PmlI and HindIII and the insert was purified by agarose gel electrophoresis and inserted into plasmid pAM1H digested with PmlI and HindIII, generating plasmid pAM1HIJ. Plasmid pAM1M was digested with EcoRI and BstEII and the insert was purified by agarose gel electrophoresis and inserted into plasmid pAM1N digested with EcoRI and BstEII, generating plasmid pAM1MN. Plasmid pAM1L was digested with EcoRI and SmaI and the insert was purified by agarose gel electrophoresis and inserted into plasmid pAM1MN digested with EcoRI and EcoRV, generating plasmid pAM1LMN. Plasmid pAM1LMN was digested with ApaI and HindIII and the insert was purified by agarose gel electrophoresis and inserted into plasmid pAM1K digested with ApaI and HindIII, generating plasmid pAM1KLMN. Plasmid pAM1EFG was digested with BamHI and the insert was purified by agarose gel electrophoresis and inserted into plasmid pAM1ABCD digested with BamHI and BglII, generating plasmid pAM1ABCDEFG; orientation was confirmed by the appearance of a diagnostic 552bp fragment when digested with BglII and HindIII. Plasmid pAM1KLMN was digested with KpnI and HindIII and the insert was purified by agarose gel electrophoresis and inserted into plasmid pAM1HIJ digested with KpnI and HindIII, generating plasmid pAM1HIJKLMN. Plasmid pAM1HIJKLMN was digested with BamHI and HindIII and the insert was purified by agarose gel electrophoresis and inserted into plasmid pAM1ABCDEFG digested with BamHI and HindIII, generating plasmid pAM1-1. These cloning steps are depicted in Figure 6. Figure 7 shows the DNA sequence of the insert contained in pAM1-1 (SEQ ID NO:1). This insert can be cloned into any suitable expression vector as an NheI-SmaI fragment to generate an expression construct. pXF8.61 (Fig. 4), pXF8.38 (Fig. 11) and pXF8.224 (Fig. 13) are examples of such a construct.

### Construction of pXF8.186

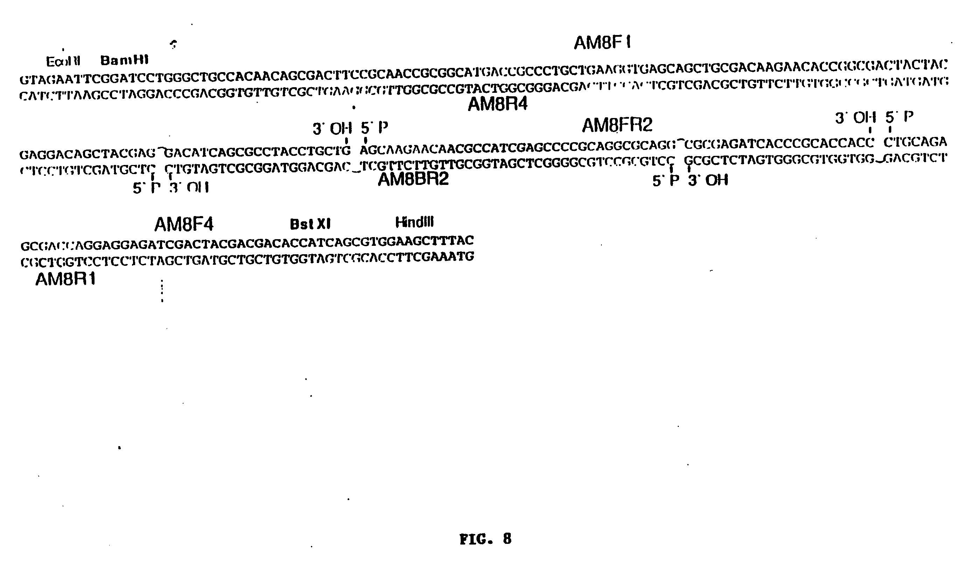

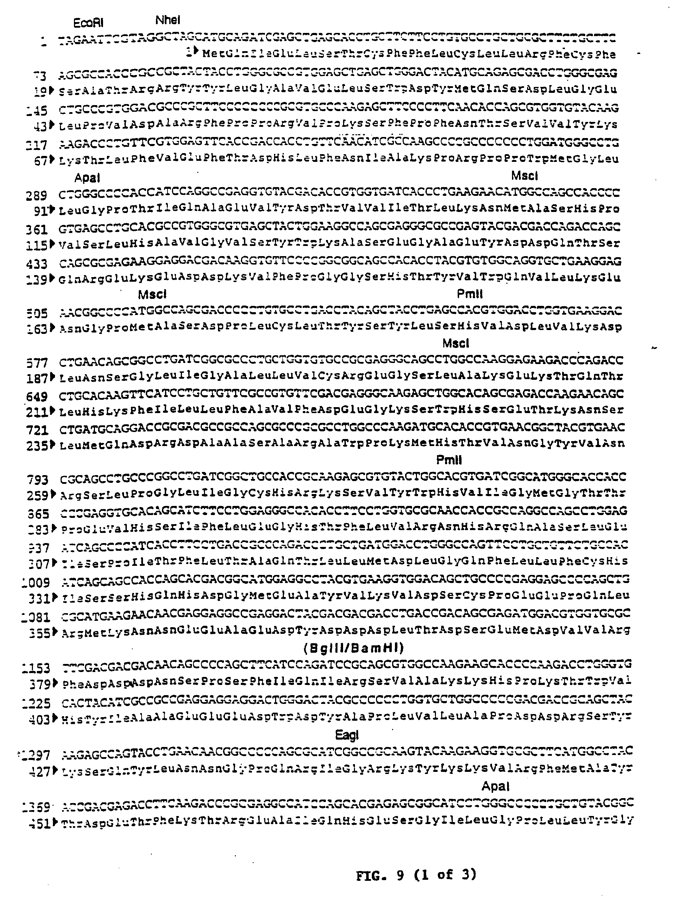

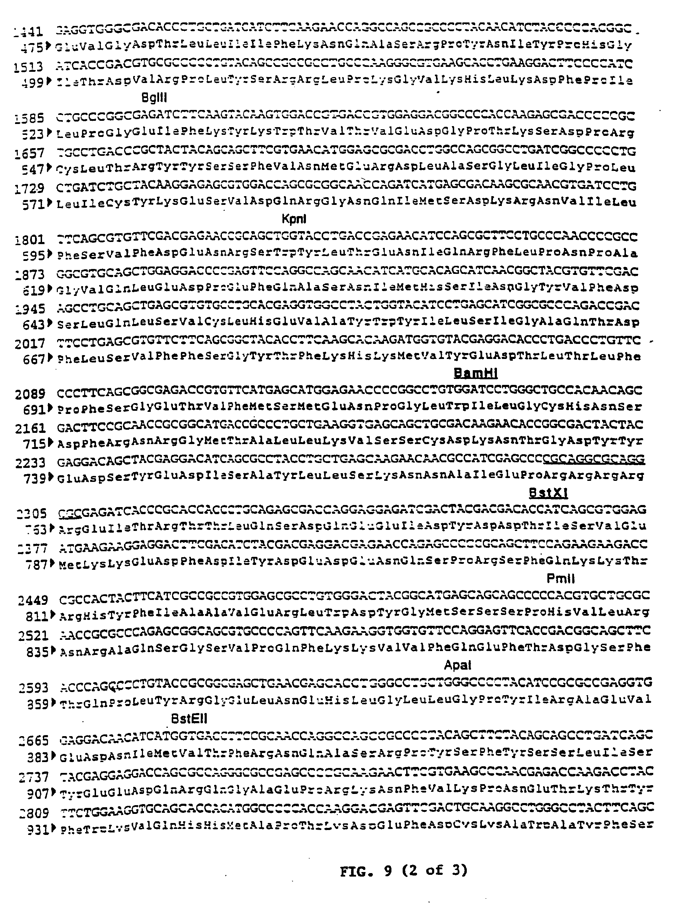

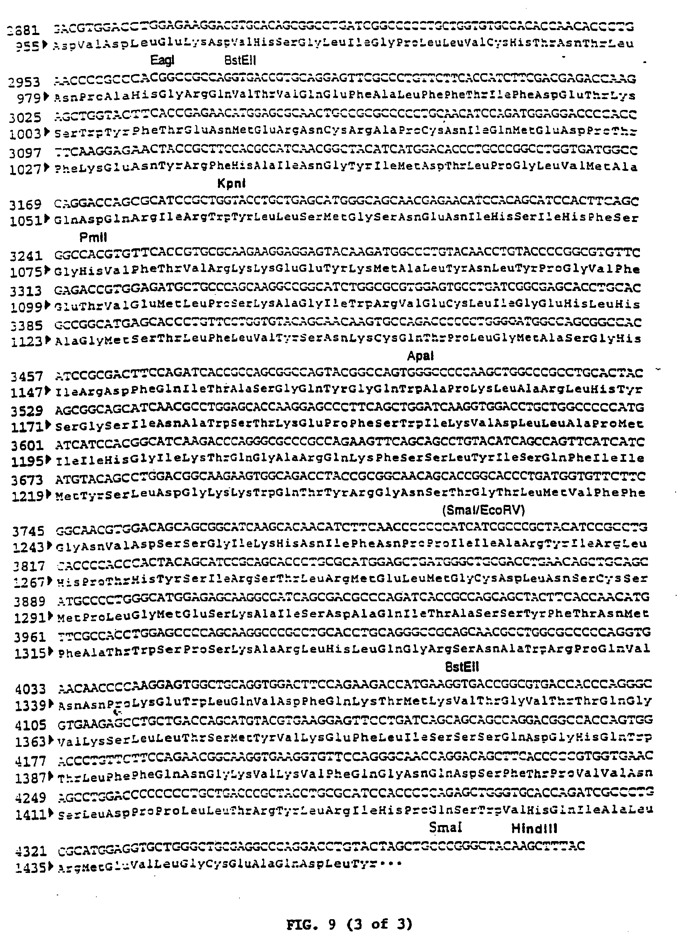

The "LE" version of the B-domain-deleted-FVIII optimized cDNA contained in pAM1-1 was modified by replacing the Leu-Glu dipeptide (2284-2289) at the junction of the heavy and light chains with four Arginine residues, making a total of five consecutive Arginine residues (SEQ ID NO:2). This was achieved as follows. The six oligonucleotides shown in Table 4 were annealed, ligated, digested with EcoRI and HindIII and cloned into pUC18 digested with EcoRI and HindIII, generating the plasmid pAM8B. Figure 8 shows how these oligonucleotides anneal to form the requisite DNA sequence. pAM8B was digested with BamHI and BstXI and the 230bp insert was purified by agarose gel electrophoresis and used to replace the BamHI(2126)-BstXI(2352) fragment of the "LE" version (See Figure 7). Figure 9 shows the sequence of the resulting cDNA (SEQ ID NO:2). This "5Arg" version of the B-domain-deleted-FVIII optimized cDNA can be cloned into any suitable expression vector as a NheI-SmaI fragment to generate an expression construct. pXF8.186 (Figure 3) is an example of such a construct.

**Table 4**

| OLIGO' NAME | OLIGO' LENGTH | OLIGONUCLEOTIDE SEQUENCE |
|---|---|---|
| AM8F1 | 140 | GTAGAATTCGGATCCTGGGCTGCCACAACAGCGACTT CCGCAACCGCGGCATGACCGCCCTGCTGAAGGTGAGC AGCTGCGACAAGAACACCGGCGACTACTACGAGGAC AGCTACGAGGACATCAGCGCCTACCTGCTG (SEQ ID NO:99) |
| AM8BF2 | 57 | AGCAAGAACAACGCCATCGAGCCCCGCAGGCGCAGG CGCGAGATCACCCGCACCACC (SEQ ID NO:100) |
| AM8F4 | 58 | CTGCAGAGCGACCAGGAGGAGATCGACTACGACGAC ACCATCAGCGTGGAAGCTTTAC (SEQ ID NO:101) |
| AM8R1 | 79 | GTAAAGCTTCCACGCTGATGGTGTCGTCGTAGTCGAT CTCCTCCTGGTCGCTCTGCAGGGTGGTGCGGGTGATCT CGCG (SEQ ID NO:102) |
| AM8BR2 | 57 | CCTGCGCCTGCGGGCTCGATGGCGTTGTTCTTGCTCA GCAGGTAGGCGCTGATGTC (SEQ ID NO:103) |
| AM8BR4 | 119 | CTCGTAGCTGTCCTCGTAGTAGTCGCCGGTGTTCTTGT CGCAGCTGCTCACCTTCAGCAGGGCGGTCATGCCGCG GTTGCGGAAGTCGCTGTTGTGGCAGCCCAGGATCCGA ATTCTAC (SEQ ID NO:104) |

### Construction of pXF8.36

The construct for expression of human Factor VIII, pXF8.36 (Fig. 10) is an 11.1 kilobase circular DNA plasmid which contains the following elements: A cytomegalovirus immediate early I gene (CMV) 5' flanking region comprised of a promoter sequence, a 5' untranslated sequence (5'UTS) and first intron sequence for initiation of transcription of the Factor VIII cDNA. The CMV region is next fused with a wild-type B domain-deleted Factor VIII cDNA sequence. The Factor VIII cDNA sequence is fused, at the 3' end, with a 0.3 kb fragment of the human growth hormone 3' untranslated sequence. A transcription termination signal and 3' untranslated sequence (3' UTS) of the human growth hormone gene is used to ensure processing of the message immediately following the stop codon. A selectable marker gene (the bacterial neomycin phosphotransferase (*neo*) gene) is inserted downstream of the Factor VIII cDNA to allow selection for stably transfected mammalian cells using the neomycin analog G418. Expression of the neo gene is under the control of the simian virus 40 (SV40) early promoter. The pUC 19-based amplicon carrying the pBR322-derived-β-lactamase (*amp*) and origin of replication (*ori*) allows for the uptake, selection and propagation of the plasmid in E coli K-12 strains. This region was derived from the plasmid pBSII SK+.

### Construction of pKF8.38

The construct for expression of human Factor VIII, pXF8.38 (Fig. 11) is an 11.1 kilobase circular DNA plasmid which contains the following elements: A cytomegalovirus immediate early I gene (CMV) 5' flanking region comprised of a promoter sequence, 5' untranslated sequence (5'UTS) and first intron sequence for initiation of transcription of the Factor VIII cDNA. The CMV region is next fused with a synthetic, optimally configured B domain-deleted Factor VIII cDNA sequence. The Factor VIII cDNA sequence is fused, at the 3' end, with a 0.3 kb fragment of the human growth hormone 3' untranslated sequence. A transcription termination signal and 3' untranslated sequence (3' UTS) of the human growth hormone gene is used to ensure processing of the message immediately following the stop codon. A selectable marker gene (the bacterial neomycin phosphotransferase (neo) gene) to allow selection for stably transfected mammalian cells using the neomycin analog G418 is inserted downstream of the Factor VIII cDNA. Expression of the neo gene is under the control of the simian virus 40 (SV40) early promoter. The pUC 19-based amplicon carrying the pBR322-derived β-lactamase (amp) and origin of replication (ori) allows for the uptake, selection and propagation of the plasmid in E coli K-12 strains. This region was derived from the plasmid pBSII SK+.

### pXF8.269 Construct

The construct for expression of human Factor VIII (Fig. 12), pXF8.269, is a 14.8 kilobase (kb) circular DNA plasmid which contains the following elements: A human collagen (I) α 2 promoter which contains 0.17 kb of 5' untranslated sequence (5'UTS), Aldolase A gene 5' untranslated sequence (5'UTS) and first intron sequence for initiation of transcription of the Factor VIII cDNA. The aldolase intron region is next fused with a synthetic, wild-type B domain-deleted Factor VIII cDNA sequence. A transcription termination signal and 3' untranslated sequence (3'UTS) of the human growth hormone gene to ensure processing of the message immediately following the stop codon. A selectable marker gene (the bacterial neomycin phosphotransferase (neo) gene) to allow selection for stably transfected mammalian cells using the neomycin analog G418 is inserted downstream of the Factor VIII cDNA.. The expression of the neo gene is under the control of the SV40 promoter. The pUC 19-based amplicon carrying the pBR322-derived β-lactamase (amp) and origin of replication (ori) allows for the uptake, selection and propagation of the plasmid in E coli K-12 strains. This region was derived from the plasmid pBSII SK+.

### pXF8.224 Construct

The construct for expression of human Factor VIII, pXF8.224 (Fig 13), is a 14.8 kilobase (kb) circular DNA plasmid which contains the following elements: A human collagen (I) α 2 promoter which contains 0.17 kb of 5' untranslated sequence (5'UTS), aldolase A gene 5' untranslated sequence (5'UTS) and first intron sequence for initiation of transcription of the Factor VIII cDNA. The aldolase intron region is next fused with a synthetic, optimally configured B domain-deleted Factor VIII cDNA sequence. A transcription termination signal and 3' untranslated sequence (3'UTS) of the human growth hormone gene is used to ensure processing of the message immediately following the stop codon. A selectable marker gene (the bacterial neomycin phosphotransferase (neo) gene) to allow selection for stably transfected mammalian cells using the neomycin analog G418 is inserted downstream of the Factor VIII cDNA. The expression of the neo gene is under the control of the SV40 promoter. The pUC 19-based amplicon carrying the pBR322-derived-β-lactamase (*amp*) and origin of replication (*ori*) allows for the uptake, selection and propagation of the plasmid in E coli K-12 strains. This region was derived from the plasmid pBSII SK+.

### Clotting Assay

A clotting assay based on an activated partial thromboplastin time (aPTT) (Proctor, et al., Am. J. Clin. Path., 36:212-219, (1961)) was performed to analyze the biological activity of the BDD hFVIII molecules expressed by constructs in which BDD-FVIII coding region was optimized.

### Biological activity as analyzed using the clotting Assay

The results of the aPTT-based clotting assay are presented in Table 5, below. Specific activity of the hFVIII preparations is presented as aPTT units per milligram hFVIII protein as determined by ELISA. Both of the human fibroblast-derived BDD hFVIII molecules (5R and LE) have high specific activity when measured the aPTT clotting assay. These specific activities have been determined to be up to 2- to 3-fold higher than those determined for CHO cell-derived full-length FVIII (as shown in Table 5). An average of multiple determinations of specific activities for various partially purified preparations of 5R and LE BDD hFVIII also shows consistently higher values for the BDD hFVIII molecules (11,622 Units/mg for 5R BDD hFVIII, and 14,561 Units/mg for LE BDD hFVIII as compared to 7097 Units/mg for full-length CHO cell-derived FVIII). An increased rate and/or extent of thrombin activation has been observed for various BDD hFVIII molecules, possibly due to an effect of the B-domain to protect the heavy and light chains from thrombin cleavage and activation (Eaton et al., Biochemistry, 25:8343-8347, (1986), Meulien et al., Protein Engineering, 2:301-306, (1988)).

**Table 5. Specific Activities of Various hFVIII Proteins**

| **hFVIII Product** | **Concentration by ELISA** (mg/mL) | **aPTT Activity** (aPTT U/mL) | **Specific Activity** (aPTT U/mg) |
|---|---|---|---|
| **5R BDD hFVIII** | 0.050 | 1306 | 26,120 |
| **LEBDD HFVIII** | 0.124 | 2908 | 23,452 |
| **Full-length (CHO-derived) FVIII** | 0.158 | 1454 | 9202 |

### Assay for Human Factor VIII in Transfected Cell Culture Supernatants

Samples of cell culture, supernatants having cells transfected with wild-type, or optimized human BDD-human Factor VIII were assayed for human Factor VIII (hFVIII) content by using an enzyme-linked immunosorbent assay (ELISA). This assay is based on the use of two non-crossreacting monoclonal antibodies (mAb) in conjunction with samples consisting of cell culture media collected from the supernatants of transfected human fibroblast cells. Methods of transfection and identification of positively transfected cells are described in the U.S. Patent No. 5,641,670.

**Table 6**

| **Plasmid** | **Promoter / 5' Untraslated sequence** | **Factor VIII cDNA Composition** | **Mean (FVIIImU / 10⁶ Cells / 24hr.)** | **Maximum(FVIII mU / 10⁶ Cells / 24 hr.)** | **Number of Strains** | **Fold Increase** |
|---|---|---|---|---|---|---|
| pXF836 | CMV IE1 | Wild Type | 567 | 2557 | 38 | - |
| pXF8.38 | CMV IE1 | Optimal Configuration | 5403 | 17106 | 24 | 9.5X |
| pXF8.269 | Collagen Iα2 / Aldolase Intron | Wild Type | 382 | 1227 | 18 | - |
| pXF8.224 | Collagen Iα2 / Aldolase Intron | Optimal Configuration | 2022 | 11930 | 218 | 5.3X |

| | | | | | | |
|---|---|---|---|---|---|---|
| ELISA units based on standard curves prepared from pooled normal plasma. | | | | | | |

### II. Factor IX Constructs and Uses thereof (Comparative example)

### Construction of Synthetic Gene Encoding Clotting Factor IX

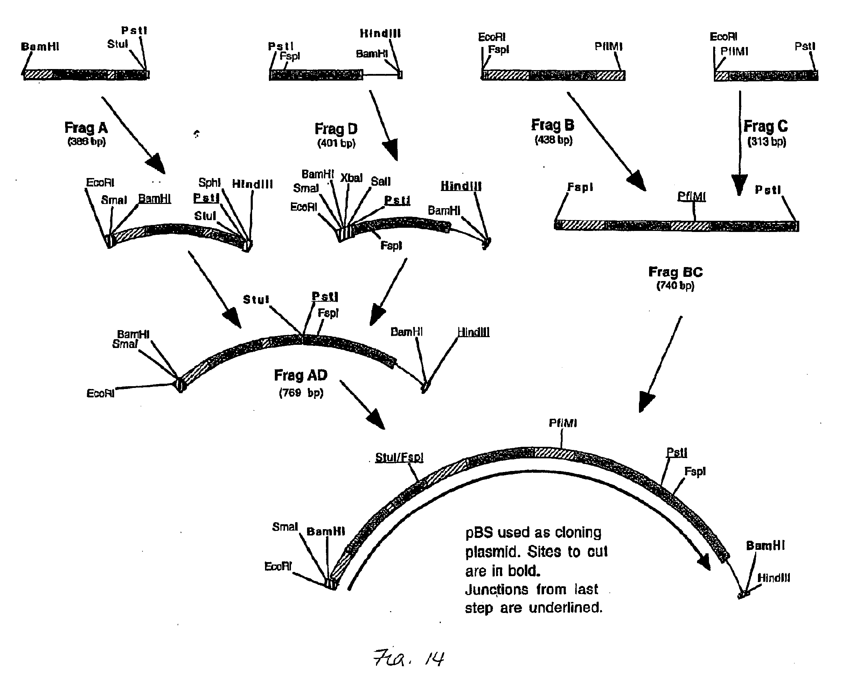

The four gene fragments listed in Table 7 and shown in Figure 14 were made by automated oligonucleotide synthesis and cloned into plasmid pBS to generate four plasmids, pFIXA through pFIXD.

**Table 7**

| Fragment | 5' end | | 3'end | |
|---|---|---|---|---|
| A | BamHI | 1 | StuI(/FspI) | 379 |
| B | (StuI/)FspI | 379 | PflMI | 810 |
| C | PflMI | 810 | PstI | 1115 |
| D | PstI | 1115 | BamHI | 1500 |

As shown in Figure 14, plasmids pFIXA through pFIXD were used to construct pFIXABCD, which carries the complete synthetic gene. Fragment A was synthesized with a PstI site 3' to the StuI site, and was cloned as a BamHI - PstI fragment. Plasmid pFIXD was digested with PstI and HindIII, and the insert was purified by agarose gel electrophoresis and inserted into plasmid pFIXA digested with PstI and HindIII, generating plasmid pFIXAD. Plasmid pFIXB was digested with EcoRI and PtlMI and the insert was purified by agarose gel electroporesis and inserted into plasmid pFIXC digested with EcoRI and PflMI, generating plasmid pFIXBC. Plasmid pFIXBC was digested with FspI and PstI and the insert was purified by agarose gel electrophoresis and inserted into plasmid PFIXAD digested with StuI and PstI, generating plasmid PFIXABCD.

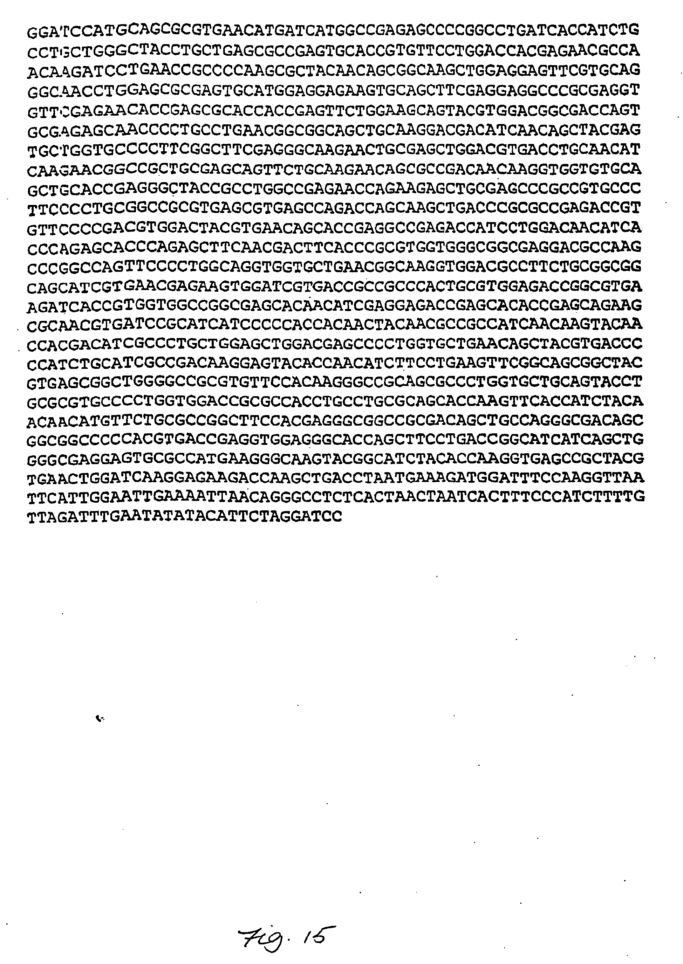

Figure 15 shows the DNA sequence of the BamHI insert contained in pFIXABCD. This insert can be cloned into any suitable expression vector as a BamHI fragment to generate an expression construct. This example illustrates how a fusion site can be used in the construction even when there exists an identical sequence in close proximity (Fragments A, B and D all contain the hexamer "AGGGCA", the product of blunt end ligation of StuI-FspI digested DNA). This is possible because the resulting fusion sites are not cut by the restriction enzymes used to create them. This example also illustrates how the gene fragments can by synthesized with additional restriction sites outside of the actual gene sequence, and these sites can be used to facilitate intermediate cloning steps.

### Expression of Human Factor IX from Optimized and Non-optimized cDNA

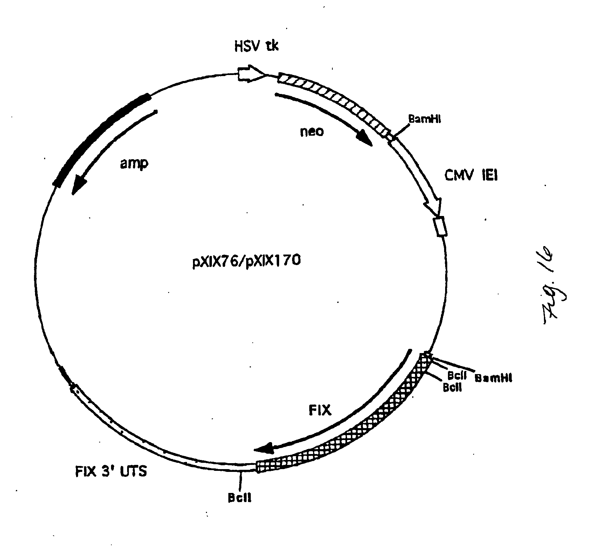

The construct for the expression of human Factor IX (Figure 16), pXIX76, is a 8.4 kilobase (kb) circular DNA plasmid which contains the following elements: a cytomegalovirus (CMV) immediate early I gene 5' flanking region comprising a promoter sequence, 5' untranslated sequence (5'UTS) and a first intron sequence (equivalent to nucleotides 174328 - 172767 of Genbank Accession X17403). The CMV region is next fused with a wild-type Factor IX cDNA sequence, with a BamHI site at the junction. The Factor IX cDNA sequence is next fused to a 1.5 kb fragment from the 3' region of the Factor IX gene that includes the transcription termination signal (equivalent to nucleotides 34335 - 35857 of Genbank Accession K02402). A selectable marker gene (the bacterial neomycin phosphotransferase gene (neo)) to allow selection for stably transfected mammalian cells using the neomycin analog G418 is inserted upstream of the CMV sequences. Expression of the neo gene is under the control of the herpes simplex virus thymidine kinase promoter. The neo expression cassette is equivalent to nucleotides 452-1596 of Genbank Accession U43612. The pUC19 - based amplicon carrying the pBR322-derived beta-lactamase gene and origin of replication allows for the selection and propagation of the plasmid in *E*. *coli.*

Plasmid pXIX170 containing a Factor IX coding region with an optimized configuration can be derived from pXIX76 by digestion with BamHI and BclI and insertion of the BamHI fragment shown in Figure 15, thus producing an equivalent construct that directs the expression of human Factor IX from an optimized cDNA.

Samples of cell culture supernatants from normal human foreskin fibroblast clones transfected with either wild-type or optimized expression constructs were assayed for expression of Factor IX. As seen in Table 8, a 2.7-fold increase in mean expression of Factor IX could be demonstrated when optimized cDNA was substituted for the wild-type sequence.

**Table 8: Expression data for strains expressing Factor IX**

| Plasmid | Promoter/5' untranslated sequence | cDNA composition | Mean | Maximum | Number of Cell Strains |
|---|---|---|---|---|---|
| | | | Nanograms/10⁶ cells/24hr | | |
| pXIX76 | CMV | Wild Type | 418 | 8384 | 144 |
| pXIX170 | CMV | Optimal Configuration | 1127 | 3316 | 33 |

### III. Alpha-Galactosidase Constructs and Uses thereof

### Construction of a Synthetic Gene Encoding α-Galactosidase

The four gene fragments listed in Table 9 were made by automated oligonucleotide synthesis and cloned into the vector pUC18 as EcoRI - Hind III fragments (with the N-terminus of each gene fragment adjacent to the EcoRI site) to generate four plasmids, pAM2A through pAM2D.

**Table 9**

| Fragment | 5'end | | | |
|---|---|---|---|---|
| A | BamHI | 1 | PstI | 364 |
| B | PstI | 364 | Bg1II(BamHI) | 697 |
| C | (Bg1II/)BamHI | 697 | SmaI(/StuI) | 1012 |
| D | (SmaI/)StuI | 1012 | XhoI | 1347 |

Plasmids pAM2A through pAM2D were used to construct pAM2ABCD, which carries the complete synthetic gene. Plasmid pAM2B was digested with PstI and HindIII and the insert was purified by agarose gel electrophoresis and inserted into plasmid pAM2A digested with PstI and HindIII, generating plasmid pAM2AB. Plasmid pAM2D was digested with StuI and HindIII and the insert was purified by agarose gel electrophoresis and inserted into plasmid pAM2C digested with Smal and HindIII, generating plasmid pAM2CD. Plasmid pAM2CD was digested with BamHI and HindII and the insert was purified by agarose gel electrophoresis and inserted into plasmid pAM2AB digested with Bg1II and HindIII, generating plasmid pAM2ABCD.

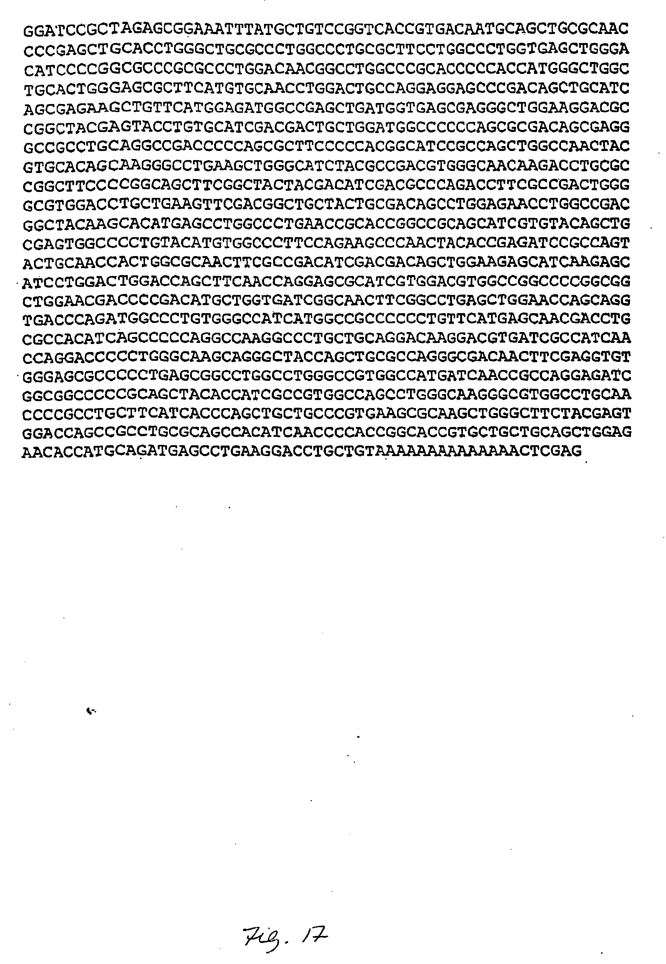

Figure 17 shows the DNA sequence of the BamHI-XhoI fragment contained in pAM2ABCD. This insert can be cloned into any suitable expression vector as a BamHI - XhoI fragment to generate an expression construct. This example illustrates the use of fusion sites that arise from the ligation of two complementary overhangs (BglII/BamHI) and from the ligation of blunt ends (SmaI/StuI).

### Expression of Human α-Galactosidase from Optimized and Non-optimized cDNAs

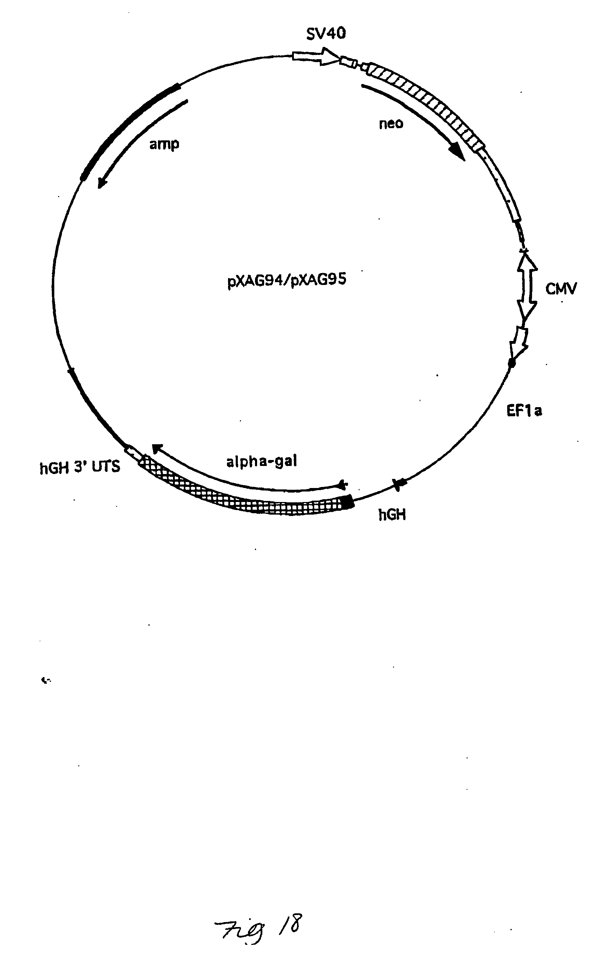

The construct for the expression of human α-galactosidase, plasmid pXAG94 (Figure 18) is a 8.5kb circular DNA plasmid which contains the following elements. A selectable marker gene (the bacterial neomycin phosphotransferase gene (neo)) is inserted upstream of the α-galactosidase expression cassette to allow selection for stably transfected mammalian cells using the neomycin analog G418. Expression of the neo gene is under the control of the SV40 early promoter. Specifically, the 342 bp PvuII - HindIII fragment equivalent to nucleotides 273 - 1/5243 - 5172 of Genbank Accession J02400 is fused via a Xhol linker to a fragment equivalent to nucleotides 502 - 561 of Genbank Accession J02400, which is next fused to the neo coding region, equivalent to nucleotides 350 - 1322 of Genbank Accession U13862. Poly-adenylation signals for this expression cassette are supplied by sequences 3393 - 3634 of SYNPRSVNEO. This selectable marker is fused to a short plasmid sequence, equivalent to nucleotides 2067 (PvuII) - 2122 of SYNPBR322.

Expression of the α-galactosidase cDNA is directed from a CMV enhancer (equivalent to nucleotides 174253 - 173848 of Genbank Accession X17403). This DNA is fused via the linker sequence TCGACAAGCCGAATTCCAGCACACTGGCGGCCGTTACTAGTGGATCCGAG (SEQ ID NO:107) to human elongation factor 1α sequences extending from-207 to +982 nucleotides relative to the cap site. These sequences provide the EF1 alpha promoter, CAP site and a 943 nucleotide intron present in the 5' untranslated sequences of this gene. The DNA is next fused to the linker sequence GAATTCTCTAGATCGAATTCCTGCAGCCCGGGGGATCCACC (SEQ ID NO:108) followed immediately by 335 nucleotides of the human growth hormone gene, starting with the ATG initiator codon, equivalent to nucleotides 5225 - 5559 of Genbank Accession J03071. This DNA codes for the signal peptide of the hGH gene, including the first intron.

This DNA is next fused to the portion of the wild-type α-galactosidase cDNA that codes for amino acids 31 to 429. The coding region is next fused via the linker AAAAAAAAAAAACTCGAGCTCTAG (SEQ ID NO:109) to the 3' untranslated region of the hGH gene, corresponding to nucleotides 6699 - 7321 of Genbank Accession J03071. Finally, this DNA is fused to a pUC - based amplicon carrying the pBR322-derived beta-lactamase gene and origin of replication which allows for the selection and propagation of the plasmid in *E. coli;* the sequences are equivalent to nucleotides 229 - 1/2680 - 281 of SYNPUC12V.

Plasmid pXAG95 is equivalent to pXAG94, with the α-galactosidase cDNA sequence replaced with the corresponding optimized configuration sequence (coding for amino acids 31 to 429) from Figure 17.

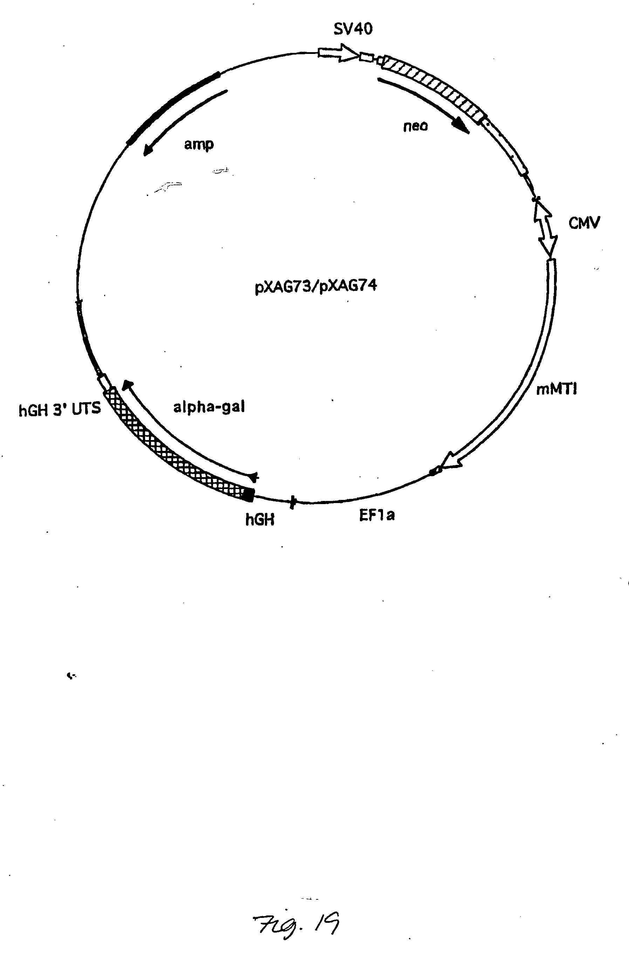

Plasmid pXAG73 (Figure 19) is a 10kb plasmid similar to pXAG94, but with the following differences. The linker sequence GCCGAATTCCAGCACACTGGCGGCCGTTACTAGTGGATCCGAG (SEQ ID NO: 110) and the adjacent EF1 alpha DNA as far as +30 beyond the cap site have been replaced with the mouse metallothionein promoter and cap site (nucleotides -1752 to +54 relative to the mMTI cap site). Also the attachment of the EFIα UTS to the hGH coding sequence differs: EF1α sequences extend as far as +973 from the EF1α cap site, followed by the linker CTAGGATCCACC (SEQ ID NO:111), in place of the GAATTCTCTAGATCGAATTCCTGCAGCCCGGGGGATCCACC (SEQ ID NO:108) linker described above.

Plasmid pXAG74 is equivalent to pXAG73, with the wild-type α-galactosidase cDNA sequence replaced with the corresponding optimized configuration sequence (coding for amino acids 31 to 429) from Figure 17.

The construction of such plasmids, including the creation of hGH -α-galactosidase fusions, is described in the U.S. Patent 6,083,725.

Samples of cell culture supernatants from normal human foreskin fibroblast clones transfected with either wild-type or optimized expression constructs were assayed for expression of α-galactosidase.

**Table 10: Expression data for strains expressing alpha-galactosidase**

| Plasmid | Promoter/5' untranslated sequence | cDNA composition | Mean | Maximum | Number of Cell Strains |
|---|---|---|---|---|---|
| | | | Units/10⁶ cells/24hr | | |
| pXAG-73 | CMV/mMT/EF1a | Wild Type | 323 | 752 | 12 |
| pXAG-74 | CMV/mMT/EF1a | Optimal Configuration | 1845 | 8586 | 27 |
| pXAG-94 | CMV/EF1a | Wild Type | 417 | 1758 | 39 |
| pXAG-95 | CMV/EF1a | Optimal Configuration | 842 | 3751 | 75 |

As shown in Table 10, 5.7- and 2.0-fold increases in mean α-galactosidase expression were seen when optimized cDNA was expressed from the EFIa (PXAG-95) and mMT1 (PXAG-74) promoters, respectively, when compared to wild type coding sequences. Furthermore, significant increases in maximum expression were also seen when the optimized cDNA was expressed from either promoter.

## Claims

1. A synthetic nucleic acid which encodes alpha-galactosidase and which differs in sequence from a wild type alpha-galactosidase nucleic acid in that at least one non-common codon or less-common codon has been replaced by a common codon and wherein the synthetic nucleic acid sequence has one or more of the following properties:
(a) it has a continuous stretch of at least 90 codons all of which are common codons;
(b) it has a continuous stretch of common codons which comprise at least 60% of the codons of the synthetic nucleic acid sequence;
(c) at least 94% or more of the codons in the synthetic nucleic acid sequence encoding the protein are common codons and the synthetic nucleic acid sequence encodes a protein of at least about 90 amino acids in length;
(d) it is at least 80 base pairs in length;
wherein by a common codon is meant Ala (gcc); Arg (cgc); Asn (aac); Asp (gac); Cys (tgc); Gln (cag); Gly (ggc); His (cac); Ile (atc); Leu (ctg); Lys (aag); Pro (ccc); Phe (ttc); Ser (agc); Thr (acc); Tyr (tac); Glu (gag) and Val (gtg);
wherein less-common codons are Gly (ggg); Ile (att); Leu (ctc); Ser (tcc); Val (gtc) and Arg (agg); and
wherein all codons other than common codons and less-common codons are non-common codons.

2. The synthetic nucleic acid of claim 1 where the alpha-galactosidase nucleic acid is inserted into a non-transformed cell.

3. The synthetic nucleic acid of claim 1 wherein the number of non-common or less-common codons remaining is less than 15.

4. The synthetic nucleic acid of claim 1 wherein all non-common or less-common codons are replaced with common codons.

5. A vector comprising the synthetic nucleic acid of claim 1, 3 or 4.

6. A cell comprising the vector of claim 5.

7. A method of producing alpha-galactosidase comprising culturing the cell of claim 6 under conditions in which the nucleic acid is expressed.

8. A method for preparing a synthetic nucleic acid encoding alpha-galactosidase which is at least 90 codons in length, comprising:
(a) identifying a non-common codon and a less-common codon in a non-optimized gene sequence which encodes an alpha-galactosidase protein; and
(b) replacing at least 94% of the non-common and less-common codons with a common codon encoding the same amino acid as the replaced codon;
wherein by a common codon is meant Ala (gcc); Arg (cgc); Asn (aac); Asp (gac); Cys (tgc); Gln (cag); Gly (ggc); His (cac); Ile (atc); Leu (ctg); Lys (aag); Pro (ccc); Phe (ttc); Ser (agc); Thr (acc); Tyr (tac); Glu (gag) and Val (gtg);
wherein less-common codons are Gly (ggg); Ile (att); Leu (ctc); Ser (tcc); Val (gtc) and Arg (agg); and
wherein all codons other than common codons and less-common codons are non-common codons.

9. The method of claim 8, wherein at least 96% of the non-common and less-common codons are replaced with a common codon encoding the same amino acid as the replaced codon.

10. Use of a synthetic nucleic acid of claim 1 that directs synthesis of an optimized message for alpha-galactosidase in the preparation of a medicament for the treatment of a disease **characterized by** alpha-galactosidase deficiency in a subject, wherein the synthetic nucleic acid is introduced into the subject and the subject expresses the alpha-galactosidase.

11. A synthetic nucleic acid of claim 1 that directs synthesis of an optimized message for alpha-galactosidase for use in treating a disease **characterized by** alpha-galactosidase deficiency in a subject, wherein the synthetic nucleic acid is introduced into the subject and the subject expresses the alpha-galactosidase.

12. The use of claim 10 or the synthetic nucleic acid of claim 11, wherein the synthetic nucleic acid is introduced into a cell.

13. The use of claim 10 or the synthetic nucleic acid of claim 11, wherein the cell can be an autologous, allogeneic, or xenogeneic cell.

14. The use of claim 10 or the synthetic nucleic acid of claim 11, wherein at least 98%, or all of the codons in the synthetic nucleic acid sequence are common codons.

15. The use of claim 10 or the synthetic nucleic acid of claim 11, wherein the subject has Fabry disease.

16. The synthetic nucleic acid of claim 1, wherein the synthetic nucleic acid comprises SEQ ID NO:106.

## Patentansprüche

1. Synthetische Nukleinsäure, die für Alpha-Galactosidase codiert und deren Sequenz sich von einer Wildtyp-Alpha-Galactosidase-Nukleinsäure dadurch unterscheidet, dass mindestens ein nicht bevorzugtes Codon oder weniger bevorzugtes Codon durch ein bevorzugtes Codon ersetzt ist, und wobei die synthetische Nukleinsäuresequenz eine oder mehrere der folgenden Eigenschaften aufweist:
(a) sie weist ein durchgehendes Stück von mindestens 90 Codons auf, die alle bevorzugte Codons sind;
(b) sie weist ein durchgehendes Stück von bevorzugten Codons auf, die mindestens 60 % der Codons der synthetischen Nukleinsäuresequenz umfassen;
(c) mindestens 94 % oder mehr der Codons in der synthetischen Nukleinsäuresequenz, die für das Protein codieren, sind bevorzugte Codons und die synthetische Nukleinsäuresequenz codiert für ein Protein, das mindestens etwa 90 Aminosäuren lang ist.
(d) sie ist mindestens 80 Basenpaare lang;
wobei ein bevorzugtes Codon Ala (gcc); Arg (cgc); Asn (aac); Asp (gac); Cys (tgc); Gln (cag); Gly (ggc); His (cac); Ile (atc); Leu (ctg); Lys (aag); Pro (ccc); Phe (ttc); Ser (agc); Thr (acc); Tyr (tac); Glu (gag) und Val (gtg) bedeutet;
wobei weniger bevorzugte Codons Gly (ggg); Ile (att); Leu (ctc); Ser (tcc); Val (gtc) und Arg (agg) sind; und
wobei alle Codons, die nicht bevorzugte oder weniger bevorzugte Codons sind, nicht bevorzugte Codons sind.

2. Synthetische Nukleinsäuresequenz nach Anspruch 1, wobei die Alpha-Galactosidase-Nukleinsäure in eine nicht-transformierte Zelle insertiert wird.

3. Synthetische Nukleinsäuresequenz nach Anspruch 1, wobei die Anzahl der nicht bevorzugten oder weniger bevorzugten Codons, die verbleiben, weniger als 15 ist.

4. Synthetische Nukleinsäuresequenz nach Anspruch 1, wobei alle nicht bevorzugten oder weniger bevorzugten Codons durch bevorzugte Codons ersetzt werden.

5. Vektor, der die synthetische Nukleinsäure von Anspruch 1, 3 oder 4 umfasst.

6. Zelle, die den Vektor von Anspruch 5 umfasst.

7. Verfahren zur Herstellung von Alpha-Galactosidase, das Kultivieren der Zelle von Anspruch 6 unter Bedingungen, unter denen die Nukleinsäure exprimiert wird, umfasst.

8. Verfahren zur Herstellung einer synthetischen Nukleinsäure, die für Alpha-Galactosidase codiert und die mindestens 90 Codons lang ist, umfassend:
(a) Identifizieren eines nicht bevorzugten Codons und eines weniger bevorzugten Codons in einer nicht optimierten Gensequenz, die für ein Alpha-Galactosidase-Protein codiert;
(b) Ersetzen mindestens 94 % der nicht bevorzugten und weniger bevorzugten Codons mit einem bevorzugten Codon, das für die gleiche Aminosäure codiert wie das ersetzte Codon;
wobei ein bevorzugtes Codon Ala (gcc); Arg (cgc); Asn (aac); Asp (gac); Cys (tgc); Gln (cag); Gly (ggc); His (cac); Ile (atc); Leu (ctg); Lys (aag); Pro (ccc); Phe (ttc); Ser (agc); Thr (acc); Tyr (tac); Glu (gag) und Val (gtg) bedeutet;
wobei weniger bevorzugte Codons Gly (ggg); Ile (att); Leu (ctc); Ser (tcc); Val (gtc) und Arg (agg) sind; und
wobei alle Codons, die nicht bevorzugte oder weniger bevorzugte Codons sind, nicht bevorzugte Codons sind.

9. Verfahren von Anspruch 8, wobei mindestens 96 % der nicht bevorzugten und weniger bevorzugten Codons mit einem bevorzugten Codon, das für die gleiche Aminosäure codiert, wie das ersetzte Codon, ersetzt werden.

10. Verwendung einer Nukleinsäure von Anspruch 1, die die Synthese einer optimierten Botschaft für Alpha-Galactosidase kontrolliert, in der Herstellung eines Medikaments zur Behandlung einer Krankheit, die durch Alpha-Galactosidase-Mangel gekennzeichnet ist, in einem Probanden, wobei die synthetische Nukleinsäure in den Probanden eingeführt wird und der Proband die Alpha-Galactosidase exprimiert.

11. Synthetische Nukleinsäure von Anspruch 1, die die Synthese einer optimierten Botschaft für Alpha-Galactosidase kontrolliert, zur Verwendung bei der Behandlung einer Krankheit, die durch Alpha-Galactosidase-Mangel gekennzeichnet ist, in einem Probanden, wobei die synthetische Nukleinsäure in den Probanden eingeführt wird und der Proband die Alpha-Galactosidase exprimiert.

12. Verwendung von Anspruch 10 oder die synthetische Nukleinsäure von Anspruch 11, wobei die synthetische Nukleinsäure in die Zelle eingeführt wird.

13. Verwendung von Anspruch 10 oder die synthetische Nukleinsäure von Anspruch 11, wobei die Zelle eine autologe, allogene oder xenogene Zelle sein kann.

14. Verwendung von Anspruch 10 oder die synthetische Nukleinsäure von Anspruch 11, wobei mindestens 98 % oder alle Codons in der synthetischen Nukleinsäuresequenz bevorzugte Codons sind.

15. Verwendung von Anspruch 10 oder die synthetische Nukleinsäure von Anspruch 11, wobei der Proband an der Fabry-Krankheit leidet.

16. Synthetische Nukleinsäure von Anspruch 1, wobei die synthetische Nukleinsäure SEQ ID NO:106 umfasst.

## Revendications

1. Acide nucléique de synthèse codant une alpha-galactosidase, et dont la séquence diffère d'un acide nucléique de type sauvage, dans la mesure où au moins un codon non commun ou un codon moins commun y a été remplacé par un codon commun, et où la séquence de l'acide nucléique de synthèse présente au moins une des propriétés suivantes :
(a) elle présente une longueur continue d'au moins 90 codons, dont tous sont des codons communs;
(b) elle présente une longueur continue de codons communs, qui comprennent au moins 60 % des codons de la séquence d'acides nucléiques de synthèse ;
(c) au moins 94 % des codons de la séquence de l'acide nucléique de synthèse codant la protéine sont des codons communs, et la séquence de l'acide nucléique de synthèse code pour une protéine d'une longueur d'au moins environ 90 acides aminés ;
(d) elle a une longueur d'au moins 80 paires de base ;
dans lequel, par codon commun, on entend Ala (gcc), Arg (cgc), Asn (aac), Asp (gac), Cys (tgc), Gln (cag), Gly (ggc), His (cac), Ile (atc), Leu (ctg), Lys (aag), Pro (ccc), Phe (ttc), Ser (agc), Thr (acc), Tyr (tac), Glu (gag) et Val (gtg) ;
dans lequel les codons moins communs sont Gly (ggg), Ile (att), Leu (ctc), Ser (tcc), Val (gtc) et Arg (agg) et
dans lequel l'ensemble des codons autres que les codons communs et que les codons moins communs sont des codons non communs.

2. Acide nucléique de synthèse selon la revendication 1, dans lequel l'acide nucléique alpha-galactosidase est inséré dans une cellule non transformée.

3. Acide nucléique de synthèse selon la revendication 1, dans lequel le nombre de codons non communs ou moins communs résiduels est inférieur à 15.

4. Acide nucléique de synthèse selon la revendication 1, dans lequel l'ensemble des codons non communs ou moins communs sont remplacés par des codons communs.

5. Vecteur comprenant l'acide nucléique de synthèse de la revendication 1, 3 ou 4.

6. Cellule comprenant le vecteur de la revendication 5.

7. Procédé de fabrication d'une alpha-galactosidase, comprenant la culture de la cellule de la revendication 6 dans des conditions dans lesquelles l'acide nucléique est exprimé.

8. Procédé de préparation d'un acide nucléique de synthèse codant pour l'alpha-galactosidase, dont la longueur atteint 90 codons, et comprenant :
(a) l'identification d'un codon non commun et d'un codon moins commun dans une séquence non optimisée de gènes, qui code pour une protéine d'alpha-galactosidase ; et
(b) le remplacement d'au moins 94 % des codons non communs et moins communs par un codon commun, codant pour le même acide aminé que le codon remplacé ;
dans lequel un « codon commun » correspond à Ala (gcc) ; Arg (cgc) ; Asn (aac) ; Asp (gac) ; Cys (tgc) ; Gln (cag) ; Gly (ggc) ; His (cac) ; Ile (atc); Leu (ctg) ; Lys (aag); Pro (ccc) ; Phe (ttc) ; Ser (agc); Thr (acc) *;* Tyr (tac) ; Glu (gag) et Val (gtg) ;
dans lequel les codons moins communs sont Gly (ggg) ; Ile (att) ; Leu (ctc) ; Ser (tcc) ; Val (gtc) et Arg (agg) ; et
dans lequel l'ensemble des codons autres que les codons communs et les codons moins communs sont des codons non communs.

9. Procédé selon la revendication 8, dans lequel au moins 96 % des codons non communs et moins communs sont remplacés par un codon commun codant pour le même acide aminé que le codon remplacé.

10. Emploi d'un acide nucléique de synthèse selon la revendication 1, qui permet la synthèse d'un message optimisé pour l'alpha-galactosidase lors de la préparation d'un médicament destiné au traitement d'une maladie **caractérisée par** une déficience de l'alpha-galactosidase chez un sujet, dans lequel l'acide nucléique de synthèse est introduit chez le sujet et où le sujet exprime l'alpha-galactosidase.

11. Acide nucléique de synthèse selon la revendication 1, qui permet la synthèse d'un message optimisé pour l'alpha-galactosidase à utiliser dans le traitement d'une maladie **caractérisée par** une déficience en galactosidase chez un sujet, l'acide nucléique de synthèse étant introduit chez le sujet et le sujet exprimant l'alpha-galactosidase.

12. Emploi selon la revendication 10 ou acide nucléique de synthèse selon la revendication 11, dans lequel l'acide nucléique de synthèse est introduit dans une cellule.

13. Emploi selon la revendication 10 ou acide nucléique de synthèse selon la revendication 11, dans lequel la cellule peut être une cellule autologue, allogénique ou xénogénique.

14. Emploi selon la revendication 10 ou acide nucléique de synthèse selon la revendication 11, dans lequel au moins 98 % ou l'ensemble des codons de la séquence de l'acide nucléique de synthèse sont des codons communs.

15. Emploi selon la revendication 10 ou acide nucléique de synthèse selon la revendication 11, dans lequel le sujet souffre de la maladie de Fabry.

16. Acide nucléique de synthèse selon la revendication 1, dans lequel l'acide nucléique de synthèse comprend SEQ ID NO :106.
